# EUROPEAN PATENT SPECIFICATION

(11) **EP 3 553 050 B1**
(45) Date of publication and mention of the grant of the patent: **02.03.2022**
(21) Application number: 19167152.8
(22) Date of filing: 03.04.2019
(51) Int. Cl.: C07D 209/80, C07D 405/04, C07D 405/14, H01L 51/50

(54) **COMPOSITION AND ORGANIC OPTOELECTRONIC DEVICE AND DISPLAY DEVICE**
ZUSAMMENSETZUNG UND ORGANISCHE OPTOELEKTRONISCHE VORRICHTUNG UND ANZEIGEVORRICHTUNG
COMPOSITION ET DISPOSITIF OPTOÉLECTRONIQUE ORGANIQUE ET DISPOSITIF D'AFFICHAGE

(30) Priority: 10.04.2018 KR 20180041650
(43) Date of publication of application: 16.10.2019
(73) Proprietor: Samsung SDI Co., Ltd., Gyeonggi-do 17084 (KR); Samsung Electronics Co., Ltd., Suwon-si, Gyeonggi-do 16677 (KR)
(72) Inventor: KANG, Dong Min, 16678 Suwon-si, Gyeonggi-do (KR); KIM, Jun Seok, 16678 Suwon-si, Gyeonggi-do (KR); LUI, Jinhyun, 16678 Suwon-si, Gyeonggi-do (KR); LEE, Byoungkwan, 16678 Suwon-si, Gyeonggi-do (KR); LEE, Sangshin, 16678 Suwon-si, Gyeonggi-do (KR); WON, Jongwoo, 443-803 Suwon-si, Gyeonggi-do (KR); LEE, Namheon, 443-803 Suwon-si, Gyeonggi-do (KR); JUNG, Sung-Hyun, 16678 Suwon-si Gyeonggi-do (KR); JUNG, Ho Kuk, 16678 Suwon-si, Gyeonggi-do (KR)
(74) Representative: Michalski Hüttermann & Partner Patentanwälte mbB

(56) References cited:
- WO-A1-2018/021663
- WO-A1-2019/151682
- KR-A- 20180 036 529

## Description

### CROSS-REFERENCE TO RELATED APPLICATION

Korean Patent Application No. 10-2018-0041650 filed on April 10, 2018, in the Korean Intellectual Property Office on, and entitled: "Composition and Organic Optoelectronic Device and Display Device".

### BACKGROUND

### 1. Field

Embodiments relate to a composition, an organic optoelectronic device, and a display device.

### 2. Description of the Related Art

An organic optoelectronic device may be used to convert electrical energy into photo energy or vice versa.

An organic optoelectronic device may be classified as follows in accordance with its driving principles. One is a photoelectric device where excitons are generated by photo energy, separated into electrons and holes, and are transferred to different electrodes to generate electrical energy. Another is a light emitting device where a voltage or a current is supplied to an electrode to generate photo energy from electrical energy.

Examples of the organic optoelectronic diode may be an organic photoelectric device, an organic light emitting diode, an organic solar cell, and an organic photo conductor drum. Compositions for organic optoelectronic devices are inter alia known from KR 2018 0036529 A and WO 2018/021663 A1.

### SUMMARY

Embodiments are directed to a composition includes a first compound, the first compound being represented by a combination of Chemical Formula 1 and Chemical Formula 2 bonded together, and a second compound, the second compound being represented by Chemical Formula 3,

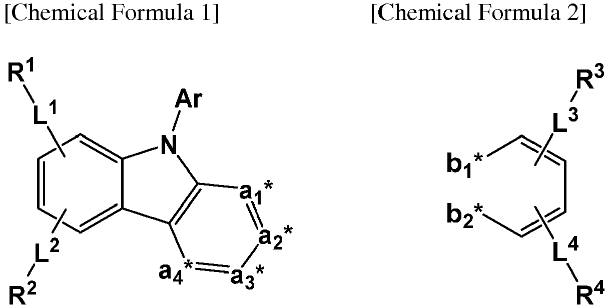

In Chemical Formula 1 and Chemical Formula 2,

Ar may be a substituted or unsubstituted C6 to C30 aryl group, a substituted or unsubstituted C2 to C30 heterocyclic group, or a combination thereof,
adjacent two of a₁^{∗} to a₄^{∗} are linked with b₁^{∗} and b₂^{∗}, respectively, and remaining two of a₁^{∗} to a₄^{∗} not linking with b₁^{∗} and b₂^{∗} may each independently be C-L^{a}-R^{a},

L^{a} and L¹ to L⁴ may each independently be a single bond, a substituted or unsubstituted C6 to C20 arylene group, a substituted or unsubstituted divalent C2 to C20 heterocyclic group, or a combination thereof,

R^{a} and R¹ to R⁴ may each independently be hydrogen, deuterium, a cyano group, a substituted or unsubstituted amine group, a substituted or unsubstituted C1 to C30 alkyl group, a substituted or unsubstituted C6 to C30 aryl group, a substituted or unsubstituted C2 to C30 heterocyclic group, or a combination thereof. At least one of R^{a} and R¹ to R⁴ may be a group represented by Chemical Formula A:

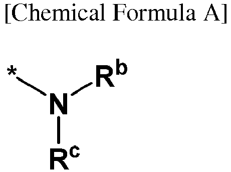

In Chemical Formula A,

R^{b} and R^{c} may each independently be a substituted or unsubstituted C6 to C30 aryl group, a substituted or unsubstituted C2 to C30 heterocyclic group, or a combination thereof, and

^{∗} is a linking point with L^{a} and L¹ to L⁴;

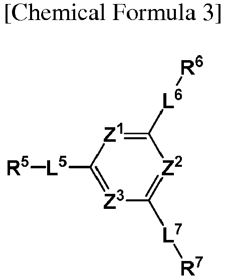

In Chemical Formula 3,

Z¹ to Z³ may each independently be N or CR^{d}, wherein R^{d} is hydrogen, deuterium, a substituted or unsubstituted C1 to C30 alkyl group, a substituted or unsubstituted C6 to C30 aryl group, a substituted or unsubstituted C3 to C30 heterocyclic group, a substituted or unsubstituted silyl group, a substituted or unsubstituted amine group, a halogen, a cyano group, or a combination thereof. At least two of Z¹ to Z³ may be N,

L⁵ to L⁷ may each independently be a single bond, a substituted or unsubstituted C6 to C20 arylene group, a substituted or unsubstituted divalent C2 to C20 heterocyclic group, or a combination thereof,

R⁵ to R⁷ may each independently be a substituted or unsubstituted C6 to C30 aryl group, a substituted or unsubstituted C2 to C30 heterocyclic group, or a combination thereof. At least one of R⁵ to R⁷ may be a group represented by Chemical Formula B:

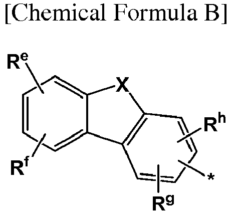

In Chemical Formula B,

X may be O or S,

R^{e} to R^{h} may each independently be hydrogen, deuterium, a substituted or unsubstituted C1 to C30 alkyl group, a substituted or unsubstituted C6 to C30 aryl group, a substituted or unsubstituted C2 to C30 heterocyclic group, a substituted or unsubstituted silyl group, a substituted or unsubstituted amine group, halogen, a cyano group, or a combination thereof,

R^{e} and R^{f} may each be separate or may be bonded with each other to form a ring,

R^{g} and R^{h} may each be separate or may be bonded with each other to form a ring, and

^{∗} is a linking point with one of L⁵ to L⁷.

Embodiments are also directed to an organic optoelectronic device, including an anode and a cathode facing each other, and at least one organic layer disposed between the anode and the cathode. The organic layer may include a composition according to an embodiment.

Embodiments are also directed to a display device, includes an organic optoelectronic device according to an embodiment.

### BRIEF DESCRIPTION OF THE DRAWINGS

Features will become apparent to those of skill in the art by describing in detail example embodiments with reference to the attached drawings in which:

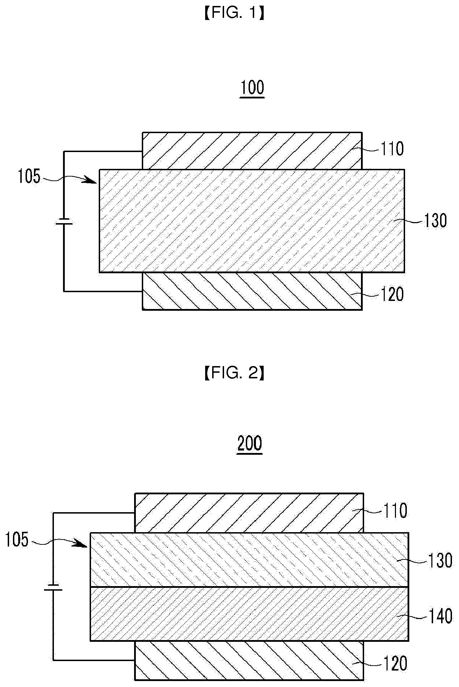

FIGS. 1 and 2 illustrate cross-sectional views of organic light emitting diodes according to embodiments.

### DETAILED DESCRIPTION

Example embodiments will now be described more fully hereinafter with reference to the accompanying drawings; however, they may be embodied in different forms and should not be construed as limited to the embodiments set forth herein. Rather, these embodiments are provided so that this disclosure will be thorough and complete, and will fully convey example implementations to those skilled in the art. In the drawing figures, the dimensions of layers and regions may be exaggerated for clarity of illustration. Like reference numerals refer to like elements throughout.

As used herein, when a definition is not otherwise provided, "substituted" refers to replacement of at least one hydrogen of a substituent or a compound by deuterium, a halogen, a hydroxyl group, an amino group, a substituted or unsubstituted C1 to C30 amine group, a nitro group, a substituted or unsubstituted C1 to C40 silyl group, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, a C1 to C20 alkoxy group, a C1 to C10 trifluoroalkyl group, a cyano group, or a combination thereof.

In one example of the present disclosure, the "substituted" refers to replacement of at least one hydrogen of a substituent or a compound by deuterium, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, or a C2 to C30 heteroaryl group. In one example of the present disclosure, the "substituted" refers to replacement of at least one hydrogen of a substituent or a compound by deuterium, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, or a C2 to C30 heteroaryl group. In addition, in specific examples of the present disclosure, the "substituted" refers to replacement of at least one hydrogen of a substituent or a compound by deuterium, a C1 to C5 alkyl group, a C6 to C18 aryl group, a pyridinyl group, a quinolinyl group, an isoquinolinyl group, a dibenzofuranyl group, a dibenzothiophenyl group, or a carbazolyl group. In addition, in specific examples of the present disclosure, the "substituted" refers to replacement of at least one hydrogen of a substituent or a compound by deuterium, a C1 to C5 alkyl group, a C6 to C18 aryl group, a dibenzofuranyl group, or a dibenzothiophenyl group. In addition, in specific examples of the present disclosure, the "substituted" refers to replacement of at least one hydrogen of a substituent or a compound by deuterium, a methyl group, an ethyl group, a propyl group, a butyl group, a phenyl group, a biphenyl group, a terphenyl group, a naphthyl group, a triphenylenyl group, a dibenzofuranyl group, or a dibenzothiophenyl group.

As used herein, when a definition is not otherwise provided, "hetero" refers to one including one to three heteroatoms selected from N, O, S, P, and Si, and remaining carbons in one functional group.

As used herein, "aryl group" refers to a group including at least one hydrocarbon aromatic moiety, and all the elements of the hydrocarbon aromatic moiety have p-orbitals which form conjugation, for example a phenyl group, a naphthyl group, and the like, two or more hydrocarbon aromatic moieties may be linked by a sigma bond and may be, for example a biphenyl group, a terphenyl group, a quarterphenyl group, and the like, and two or more hydrocarbon aromatic moieties are fused directly or indirectly to provide a non-aromatic fused ring, for example a fluorenyl group.

The aryl group may include a monocyclic, polycyclic or fused ring polycyclic (i.e., rings sharing adjacent pairs of carbon atoms) functional group.

As used herein, "heterocyclic group" is a generic concept of a heteroaryl group, and may include at least one hetero atom selected from N, O, S, P, and Si instead of carbon (C) in a cyclic compound such as aryl group, a cycloalkyl group, a fused ring thereof, or a combination thereof. When the heterocyclic group is a fused ring, the entire ring or each ring of the heterocyclic group may include one or more heteroatoms.

For example, "heteroaryl group" may refer to an aryl group including at least one hetero atom selected from N, O, S, P, and Si instead of carbon (C). Two or more heteroaryl groups are linked by a sigma bond directly, or when the C2 to C60 heteroaryl group includes two or more rings, the two or more rings may be fused. When the heteroaryl group is a fused ring, each ring may include 1 to 3 hetero atoms.

More specifically, the substituted or unsubstituted C6 to C30 aryl group may be a substituted or unsubstituted phenyl group, a substituted or unsubstituted naphthyl group, a substituted or unsubstituted anthracenyl group, a substituted or unsubstituted phenanthrenyl group, a substituted or unsubstituted naphthacenyl group, a substituted or unsubstituted pyrenyl group, a substituted or unsubstituted biphenyl group, a substituted or unsubstituted p-terphenyl group, a substituted or unsubstituted m-terphenyl group, a substituted or unsubstituted o-terphenyl group, a substituted or unsubstituted chrysenyl group, a substituted or unsubstituted triphenylenyl group, a substituted or unsubstituted perylenyl group, a substituted or unsubstituted fluorenyl group, a substituted or unsubstituted indenyl group , or a combination thereof.

More specifically, the substituted or unsubstituted C2 to C30 heterocyclic group may be a substituted or unsubstituted furanyl group, a substituted or unsubstituted thiophenyl group, a substituted or unsubstituted pyrrolyl group, a substituted or unsubstituted pyrazolyl group, a substituted or unsubstituted imidazolyl group, a substituted or unsubstituted triazolyl group, a substituted or unsubstituted oxazolyl group, a substituted or unsubstituted thiazolyl group, a substituted or unsubstituted oxadiazolyl group, a substituted or unsubstituted thiadiazolyl group, a substituted or unsubstituted pyridyl group, a substituted or unsubstituted pyrimidinyl group, a substituted or unsubstituted pyrazinyl group, a substituted or unsubstituted triazinyl group, a substituted or unsubstituted benzofuranyl group, a substituted or unsubstituted benzothiophenyl group, a substituted or unsubstituted benzimidazolyl group, a substituted or unsubstituted indolyl group, a substituted or unsubstituted quinolinyl group, a substituted or unsubstituted isoquinolinyl group, a substituted or unsubstituted quinazolinyl group, a substituted or unsubstituted quinoxalinyl group, a substituted or unsubstituted naphthyridinyl group, a substituted or unsubstituted benzoxazinyl group, a substituted or unsubstituted benzthiazinyl group, a substituted or unsubstituted acridinyl group, a substituted or unsubstituted phenazinyl group, a substituted or unsubstituted phenothiazinyl group, a substituted or unsubstituted phenoxazinyl group, a substituted or unsubstituted dibenzofuranyl group, or a substituted or unsubstituted dibenzothiophenyl group, or a combination thereof.

In the present specification, hole characteristics refer to an ability to donate an electron to form a hole when an electric field is applied, and that a hole formed in the anode may be easily injected into a light emitting layer, and a hole formed in a light emitting layer may be easily transported into an anode and transported in the light emitting layer due to conductive characteristics according to a highest occupied molecular orbital (HOMO) level.

In addition, electron characteristics refer to an ability to accept an electron when an electric field is applied, and that an electron formed in a cathode may be easily injected into a light emitting layer, and an electron formed in a light emitting layer may be easily transported into a cathode and transported in the light emitting layer due to conductive characteristics according to a lowest unoccupied molecular orbital (LUMO) level.

Hereinafter, a composition for an organic optoelectronic device according to an example embodiment is described.

A composition for an organic optoelectronic device according to an example embodiment includes a first compound and a second compound. According to an embodiment of the present invention, the first compound may have hole characteristics and the second compound may have electron characteristics.

According to an example embodiment, the first compound is represented by a combination of Chemical Formula 1 and Chemical Formula 2 bonded together:

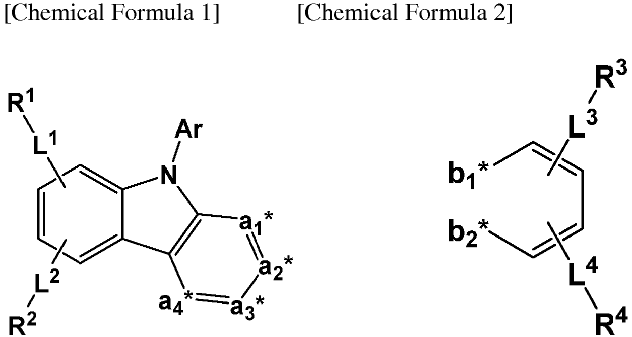

In Chemical Formula 1 and Chemical Formula 2,

Ar may be a substituted or unsubstituted C6 to C30 aryl group, a substituted or unsubstituted C2 to C30 heterocyclic group, or a combination thereof,

adjacent two of a₁^{∗} to a₄^{∗} may be linked with b₁^{∗} and b₂^{∗}, respectively, and remaining two of a₁^{∗} to a₄^{∗} not linking with b₁^{∗} and b₂^{∗} may each independently be C-L^{a}-R^{a},

L^{a} and L¹ to L⁴ may each independently be a single bond, a substituted or unsubstituted C6 to C20 arylene group, a substituted or unsubstituted divalent C2 to C20 heterocyclic group, or a combination thereof,

R^{a} and R¹ to R⁴ may each independently be hydrogen, deuterium, a cyano group, a substituted or unsubstituted amine group, a substituted or unsubstituted C1 to C30 alkyl group, a substituted or unsubstituted C6 to C30 aryl group, a substituted or unsubstituted C2 to C30 heterocyclic group, or a combination thereof, and

at least one of R^{a} and R¹ to R⁴ may be a group represented by Chemical Formula A:

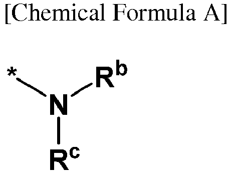

In Chemical Formula A,

R^{b} and R^{c} may each independently be a substituted or unsubstituted C6 to C30 aryl group, a substituted or unsubstituted C2 to C30 heterocyclic group, or a combination thereof, and

^{∗} is a linking point with L^{a} and L¹ to L⁴.

Without being bound by theory, it is believed that, the first compound has a structure where benzocarbazole is substituted with amine, a HOMO electron cloud is expanded from amine into benzocarbazole and thus hole injection and transport characteristics may be improved due to high HOMO energy. In addition, since the benzocarbazole has relatively high HOMO energy compared with bicarbazole and indolocarbazole, a device having a low driving voltage may be realized due to the structure where benzocarbazole is substituted with amine. In addition, bicarbazole and the indolocarbazole may not be desirable as a red host due to high T1 energy, but the structure where benzocarbazole is substituted with amine has a desirable T1 energy as a red host. Accordingly, a device including the first compound may realize high efficiency/long life-span characteristics. Further, it may be included with the second compound to exhibit good interface characteristics and hole and electron transport capability and thus may lower a driving voltage of a device.

According to an embodiment of the present invention, R^{b} and R^{c} may independently be a substituted or unsubstituted phenyl group, a substituted or unsubstituted biphenyl group, a substituted or unsubstituted anthracenyl group, a substituted or unsubstituted naphthyl group, a substituted or unsubstituted phenanthrenyl group, a substituted or unsubstituted triphenylenyl group, a substituted or unsubstituted fluorenyl group, a substituted or unsubstituted carbazolyl group, a substituted or unsubstituted dibenzofuranyl group, or a substituted or unsubstituted dibenzothiophenyl group.

According to an embodiment of the present invention, R^{b} and R^{c} may independently be a substituted or unsubstituted, phenyl group, a substituted or unsubstituted p-biphenyl group, or a substituted or unsubstituted fluorenyl group, wherein the substituent may be a phenyl group, a naphthyl group, an anthracenyl group, a phenanthrenyl group, a triphenylenyl group, a carbazolyl group, a dibenzofuranyl group, or a dibenzothiophenyl group.

According to an embodiment of the present invention, Ar may independently be a substituted or unsubstituted C6 to C20 aryl group or a substituted or unsubstituted C2 to C20 heterocyclic group.

According to an embodiment of the present invention, Ar may be a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted phenanthrenyl group, a substituted or unsubstituted triphenylenyl group, a substituted or unsubstituted fluorenyl group, a substituted or unsubstituted dibenzofuranyl group, a substituted or unsubstituted dibenzothiophenyl group, a substituted or unsubstituted carbazolyl group, or a combination thereof.

According to an embodiment of the present invention, Ar may be a substituted or unsubstituted phenyl group, a substituted or unsubstituted biphenyl group, a substituted or unsubstituted naphthyl group, a substituted or unsubstituted dibenzofuranyl group, a substituted or unsubstituted dibenzothiophenyl group, or a substituted or unsubstituted carbazolyl group.

According to an embodiment of the present invention, L^{a} and L¹ to L⁴ may independently be a single bond or a substituted or unsubstituted C6 to C20 arylene group.

According to an embodiment of the present invention, L^{a} and L¹ to L⁴ may independently be a single bond, a substituted or unsubstituted phenylene group, a substituted or unsubstituted biphenylene group, a substituted or unsubstituted terphenylene group, or a substituted or unsubstituted naphthylene group.

According to an embodiment of the present invention, L^{a} and L¹ to L⁴ may independently be a single bond, a substituted or unsubstituted m-phenylene group, a substituted or unsubstituted p-phenylene group, a substituted or unsubstituted o-phenylene group, a substituted or unsubstituted m-biphenylene group, a substituted or unsubstituted p-biphenylene group, a substituted or unsubstituted o-biphenylene group, a substituted or unsubstituted m-terphenylene group, a substituted or unsubstituted p-terphenylene group, or a substituted or unsubstituted o-terphenylene group. Herein, the "substituted" may for example refer to replacement of at least one hydrogen by deuterium, a C1 to C20 alkyl group, a C6 to C20 aryl group, a halogen, a cyano group, or a combination thereof.

According to an embodiment of the present invention, R^{a} and R¹ to R⁴ may independently be hydrogen, deuterium, a cyano group, a substituted or unsubstituted C1 to C10 alkyl group, a substituted or unsubstituted C6 to C20 aryl group, a substituted or unsubstituted C2 to C20 heterocyclic group, or the group represented by Chemical Formula A.

According to an embodiment of the present invention, R^{a} and R¹ to R⁴ may independently be hydrogen or the group represented by Chemical Formula A.

According to an embodiment of the present invention, the first compound may for example be represented by one of Chemical Formula 1A to Chemical Formula 1C according to a fusion position of Chemical Formula 1 and Chemical Formula 2.

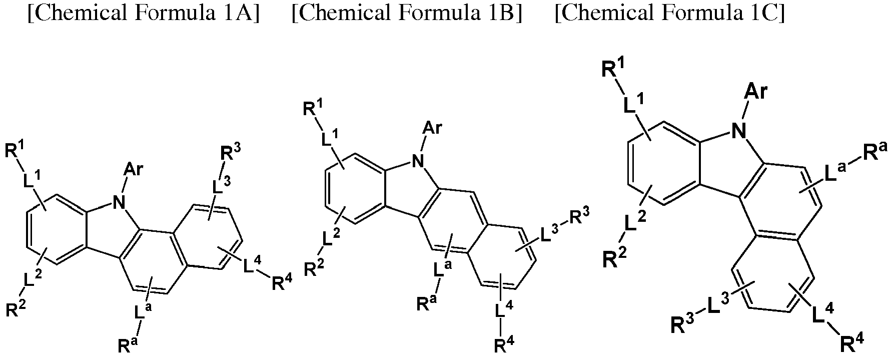

In Chemical Formula 1A to Chemical Formula 1C, Ar, L^{a} and L¹ to L⁴, and R^{a} and R¹ to R⁴ are the same as described above.

According to an embodiment of the present invention, Chemical Formula 1A may be represented by one of Chemical Formula 1A-1 to Chemical Formula 1A-3 according to a substitution position of the group represented by Chemical Formula A.

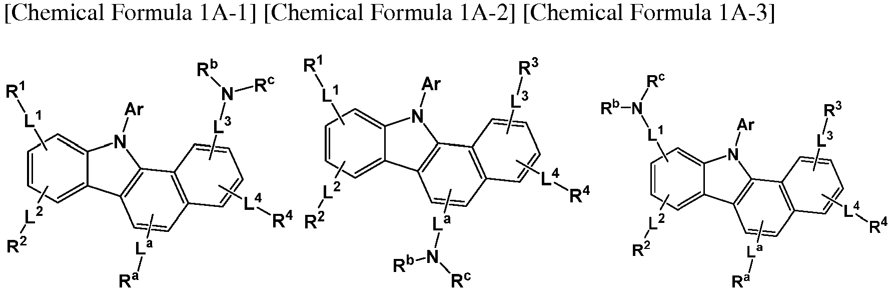

In Chemical Formula 1A-1 to Chemical Formula 1A-3, Ar, L^{a}, L¹ to L⁴, R^{a}, R¹ to R⁴, R^{b}, and R^{c} are the same as described above.

According to an embodiment of the present invention, Chemical Formula 1A-1 may be represented by one of Chemical Formula 1A-1-a to Chemical Formula 1A-1-d according to a specific substitution position of the group represented by Chemical Formula A.

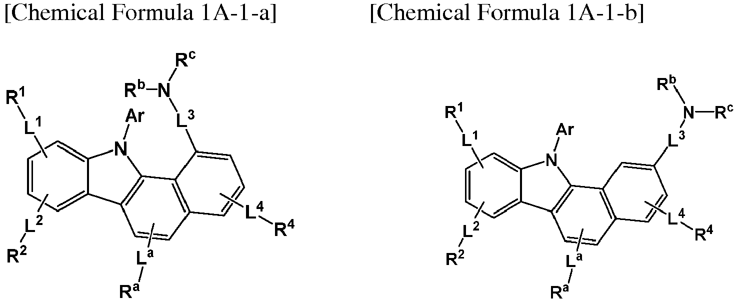

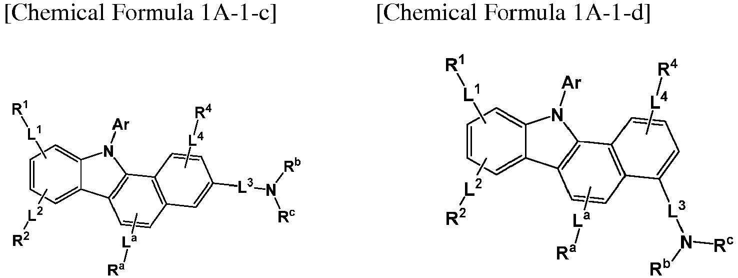

In Chemical Formula 1A-1-a to Chemical Formula 1A-1-d, Ar, L^{a}, L¹ to L⁴, R^{a}, R¹ to R⁴, R^{b}, and R^{c} are the same as described above.

According to an embodiment of the present invention, Chemical Formula 1A-1 may be represented by Chemical Formula 1A-1-b or Chemical Formula 1A-1-c.

According to an embodiment of the present invention, Chemical Formula 1A-2 may be represented by Chemical Formula 1A-2-a or Chemical Formula 1A-2-b according to a specific substitution position of the group represented by Chemical Formula A.

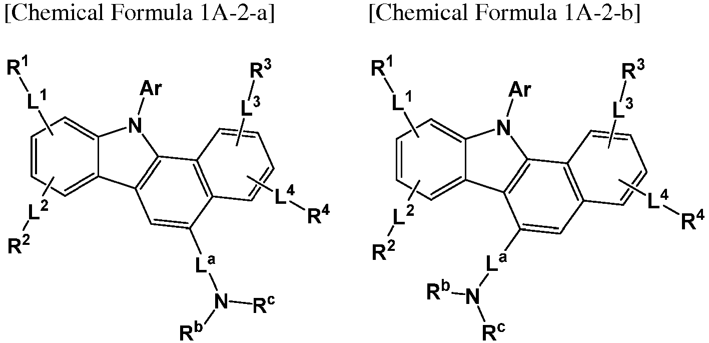

In Chemical Formula 1A-2-a and Chemical Formula 1A-2-b, Ar, L^{a} and L¹ to L⁴, and R¹ to R⁴ and R^{b} and R^{c} are the same as described above.

According to an embodiment of the present invention, Chemical Formula 1A-2 may be represented by Chemical Formula 1A-2-a.

According to an embodiment of the present invention, Chemical Formula 1A-3 may be represented by one of Chemical Formula 1A-3-a to Chemical Formula 1A-3-d according to a specific substitution position of the group represented by Chemical Formula A.

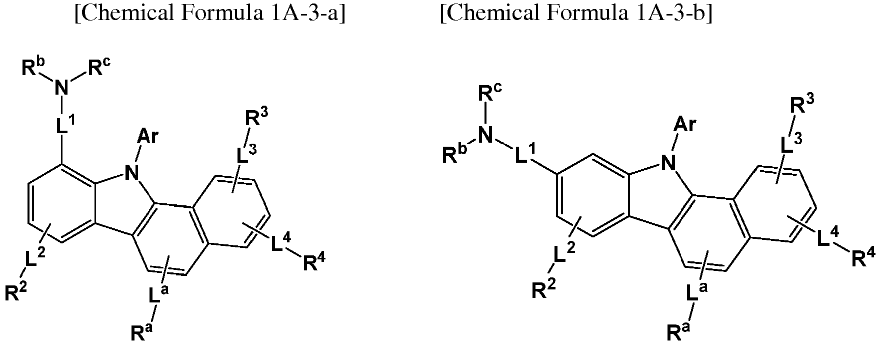

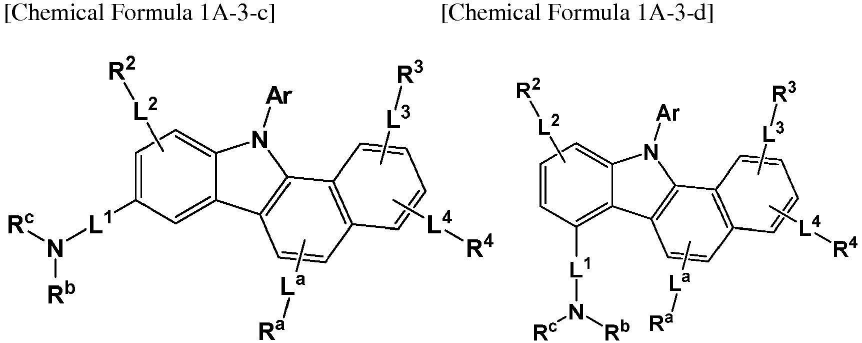

In Chemical Formula 1A-3-a to Chemical Formula 1A-3-d, Ar, L^{a}, L¹ to L⁴, R^{a}, R¹ to R⁴, R^{b}, and R^{c} are the same as described above.

According to an embodiment of the present invention, Chemical Formula 1A-3 may be represented by Chemical Formula 1A-3-b or Chemical Formula 1A-3-c.

According to an embodiment of the present invention, Chemical Formula 1B may be represented by one of Chemical Formula 1B-1 to Chemical Formula 1B-3 according to a substitution position of the group represented by Chemical Formula A.

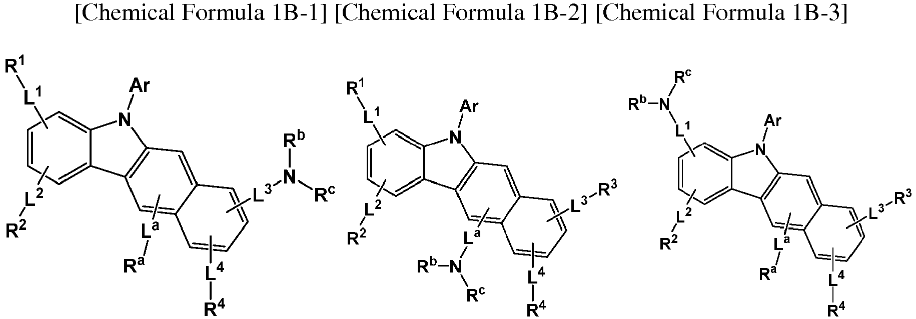

In Chemical Formula 1B-1 to Chemical Formula 1B-3, Ar, L^{a}, L¹ to L⁴, R^{a}, R¹ to R⁴, R^{b}, and R^{c} are the same as described above.

According to an embodiment of the present invention, Chemical Formula 1B-1 may be represented by one of Chemical Formula 1B-1-a to Chemical Formula 1B-1-d according to a specific substitution position of the group represented by Chemical Formula A.

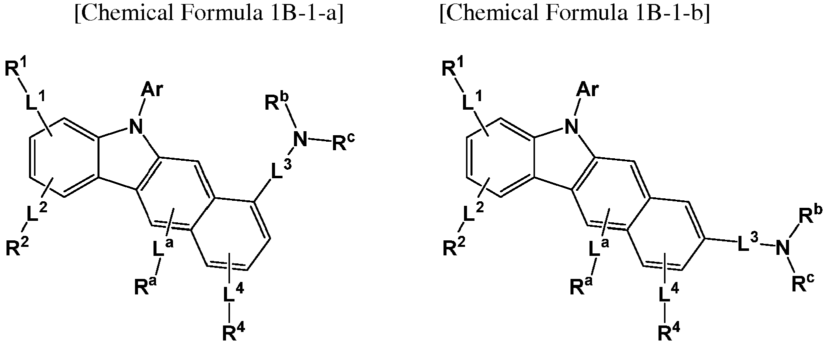

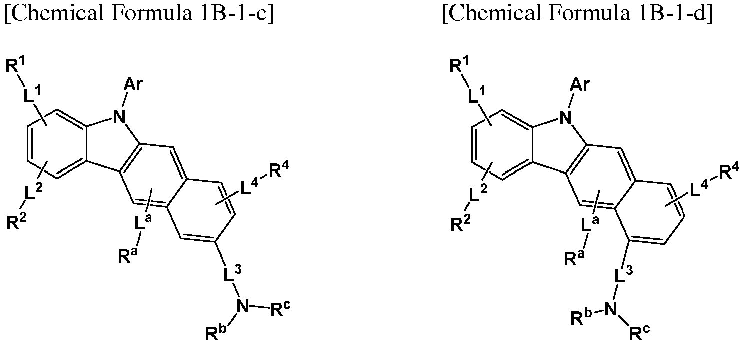

In Chemical Formula 1B-1-a to Chemical Formula 1B-1-d, Ar, L^{a}, L¹ to L⁴, R^{a}, R¹ to R⁴, R^{b}, and R^{c} are the same as described above.

According to an embodiment of the present invention, Chemical Formula 1B-2 may be represented by Chemical Formula 1B-2-a or Chemical Formula 1B-2-b according to a specific substitution position of the group represented by Chemical Formula A.

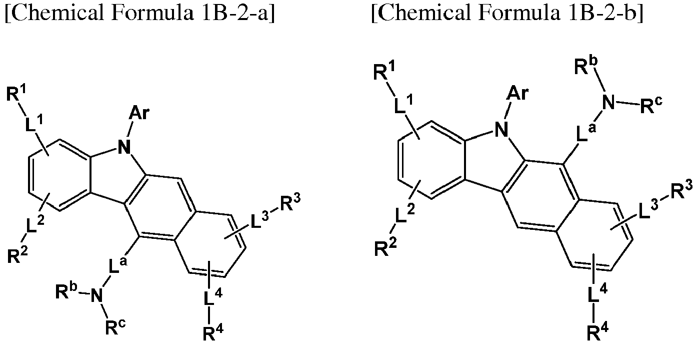

In Chemical Formula 1B-2-a and Chemical Formula 1B-2-b, Ar, L^{a}, L¹ to L⁴, R¹ to R⁴, R^{b}, and R^{c} are the same as described above.

According to an embodiment of the present invention, Chemical Formula 1B-3 may be represented by one of Chemical Formula 1B-3-a to Chemical Formula 1B-3-d according to a specific substitution position of the group represented by Chemical Formula A.

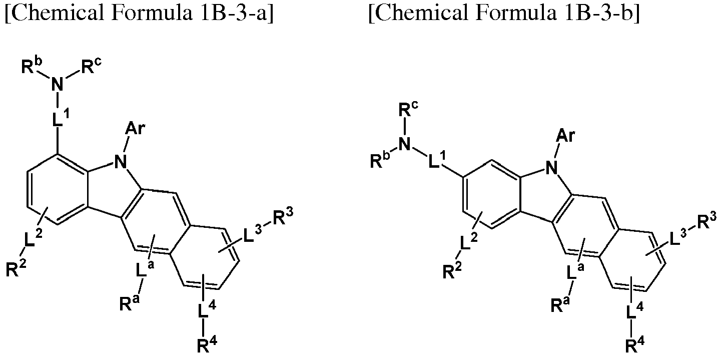

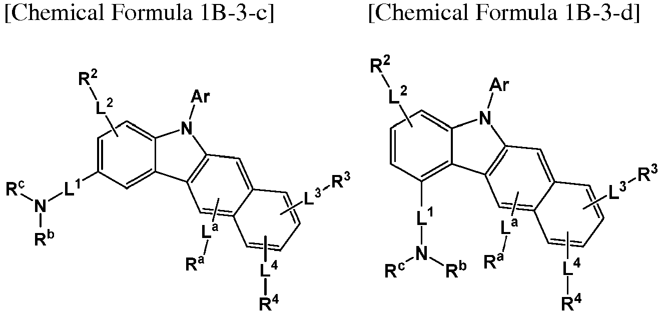

In Chemical Formula 1B-3-a to Chemical Formula 1B-3-d, Ar, L^{a}, L¹ to L⁴, R^{a}, R¹ to R⁴, R^{b}, and R^{c} are the same as described above.

According to an embodiment of the present invention, Chemical Formula 1B-3 may be represented by Chemical Formula 1B-3-b.

According to an embodiment of the present invention, Chemical Formula 1C may be represented by one of Formula 1C-1 to Chemical Formula 1C-3 according to a substitution position of the group represented by Chemical Formula A.

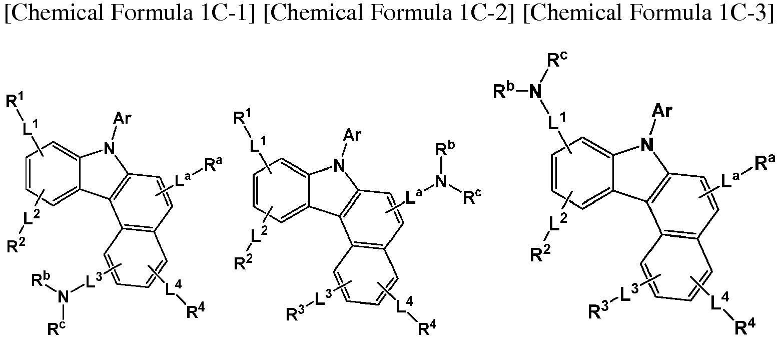

In Chemical Formula 1C-1 to Chemical Formula 1C-3, Ar, L^{a}, L¹ to L⁴, R^{a}, R¹ to R⁴, R^{b}, and R^{c} are the same as described above.

According to an embodiment of the present invention, Chemical Formula 1C-1 may be represented by one of Chemical Formula 1C-1-a to Chemical Formula 1C-1-d according to a specific substitution position of the group represented by Chemical Formula A.

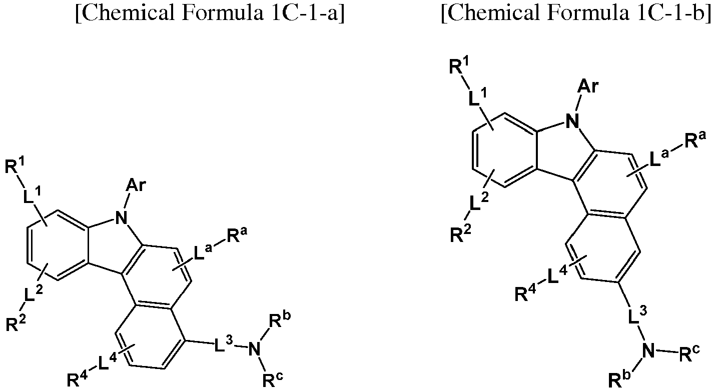

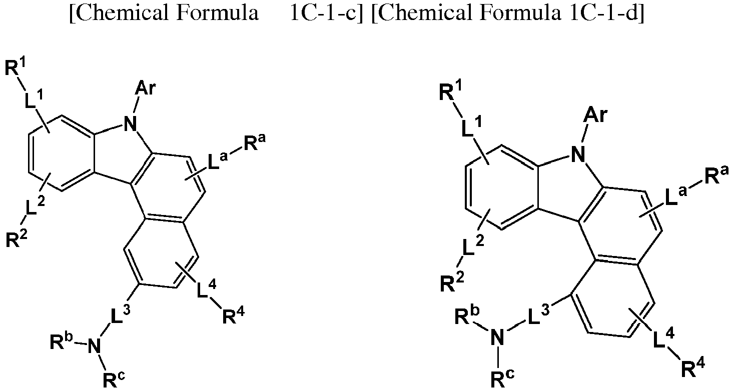

In Chemical Formula 1C-1-a to Chemical Formula 1C-1-d, Ar, L^{a}, L¹ to L⁴, R^{a}, R¹ to R⁴, R^{b}, and R^{c} are the same as described above.

According to an embodiment of the present invention, Chemical Formula 1C-1 may be represented by Chemical Formula 1C-1-b.

According to an embodiment of the present invention, above Chemical Formula 1C-2 may be represented by Chemical Formula 1C-2-a or Chemical Formula 1C-2-b according to a specific substitution position of the group represented by Chemical Formula A.

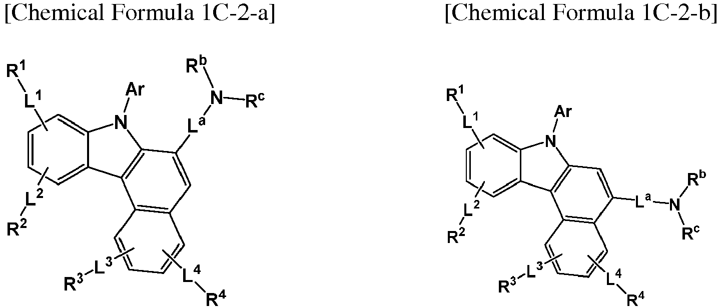

In Chemical Formula 1C-2-a and Chemical Formula 1C-2-b, Ar, L^{a}, L¹ to L⁴, R¹ to R⁴, R^{b}, and R^{c} are the same as described above.

According to an embodiment of the present invention, Chemical Formula 1C-3 may be represented by one of Chemical Formula 1C-3-a to Chemical Formula 1C-3-d according to a specific substitution position of the group represented by Chemical Formula A.

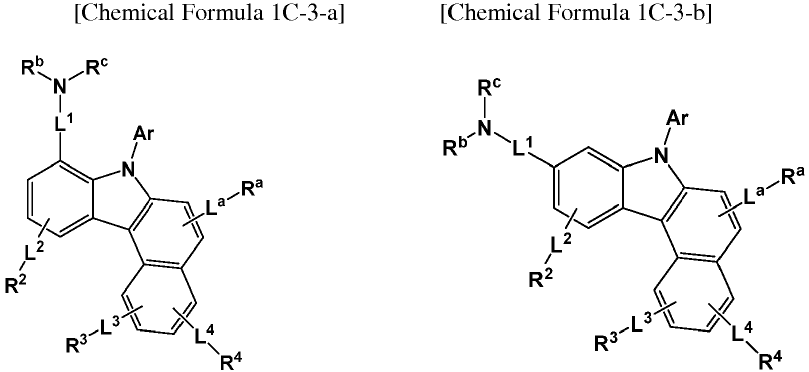

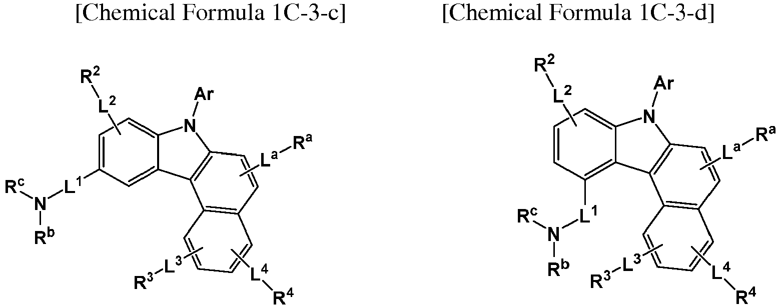

In Chemical Formula 1C-3-a to Chemical Formula 1C-3-d, Ar, L^{a}, L¹ to L⁴, R^{a}, R¹ to R⁴, R^{b}, and R^{c} are the same as described above.

According to an embodiment of the present invention, Chemical Formula 1C-3 may be represented by Chemical Formula 1C-3-b.

According to an embodiment of the present invention, the first compound may be represented by Chemical Formula 1A, for example Chemical Formula 1A-1, for example Chemical Formula 1A-1-b.

The first compound may be, for example, one of the compounds of Group 1:

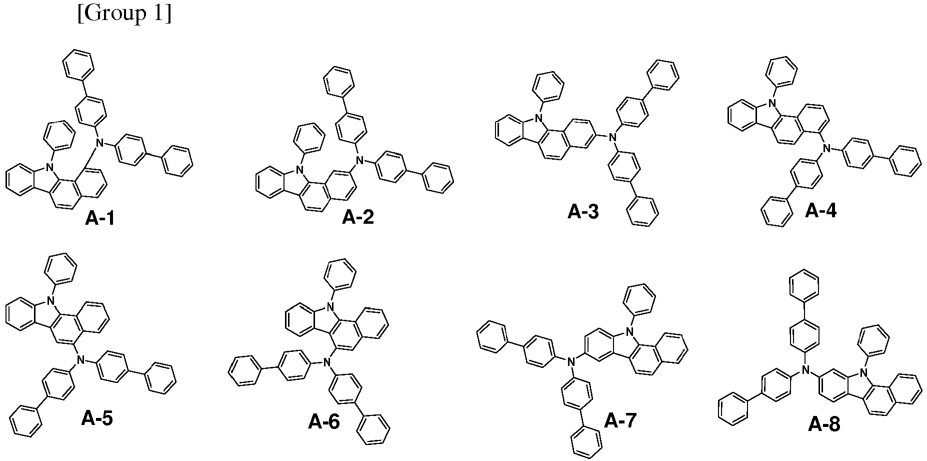

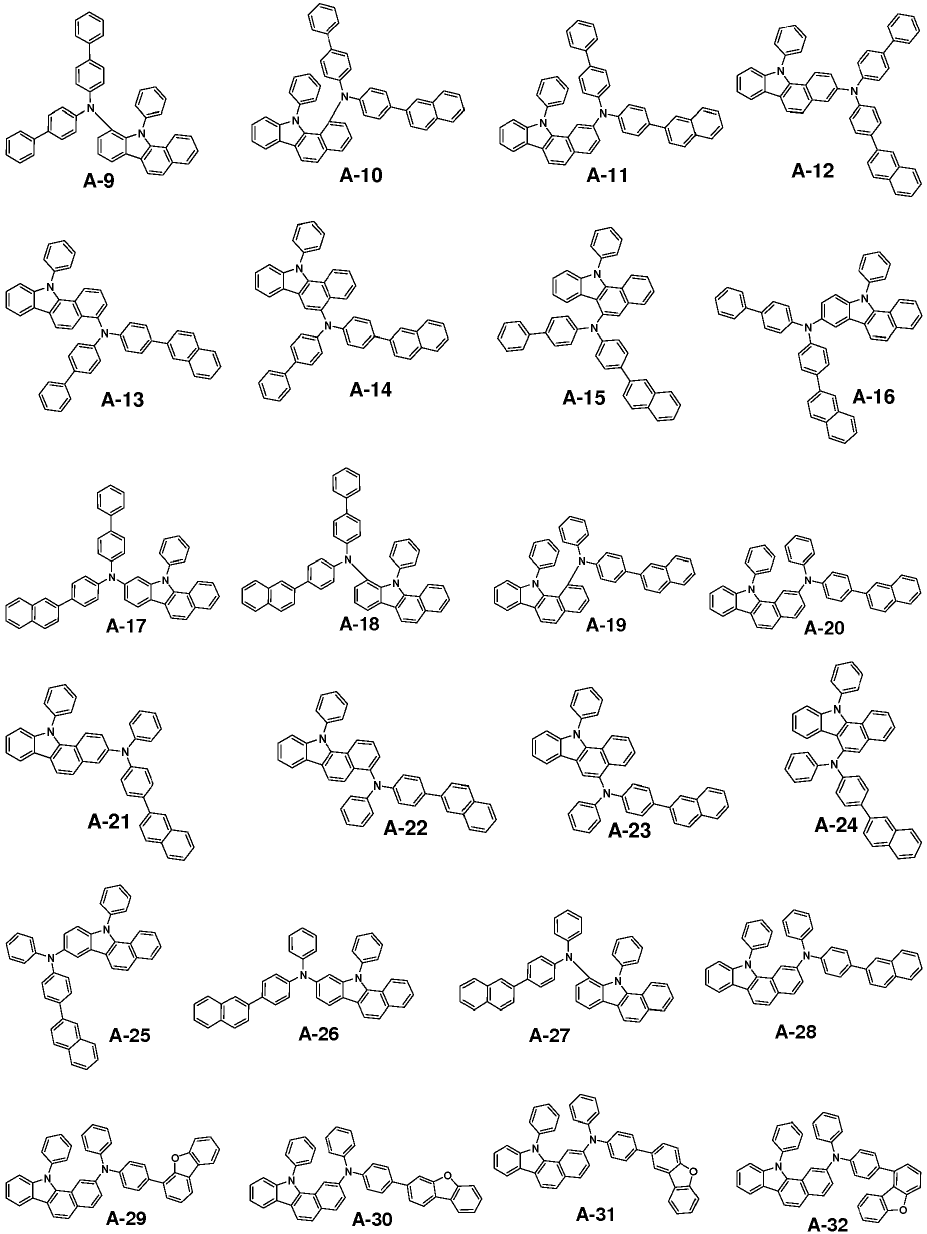

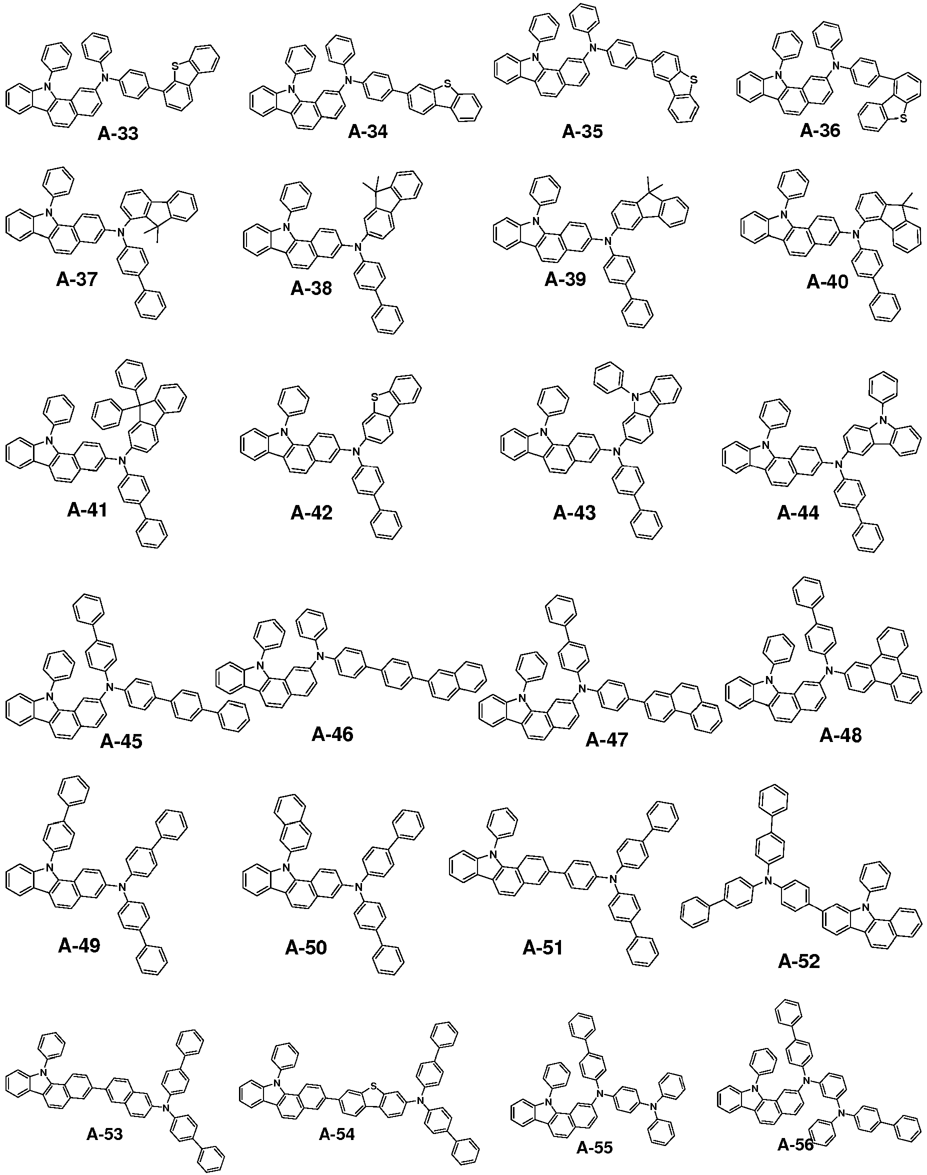

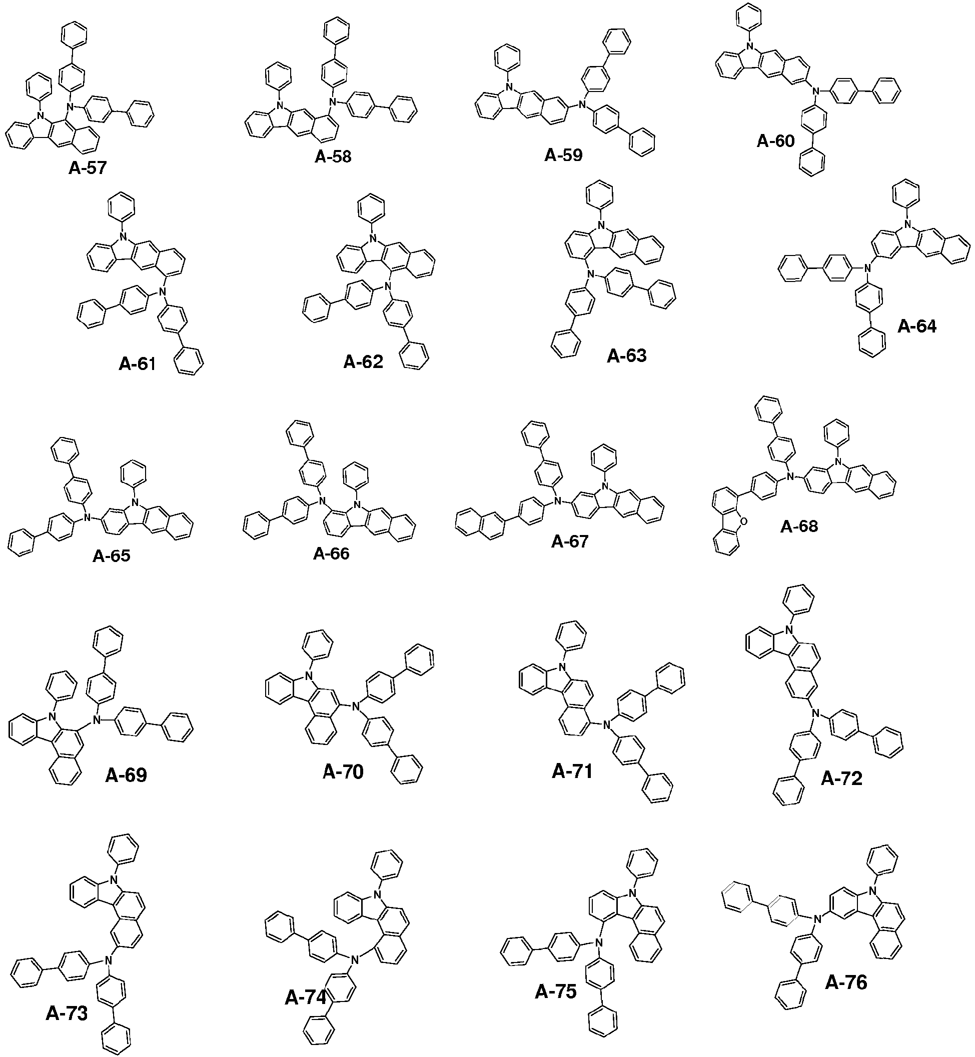

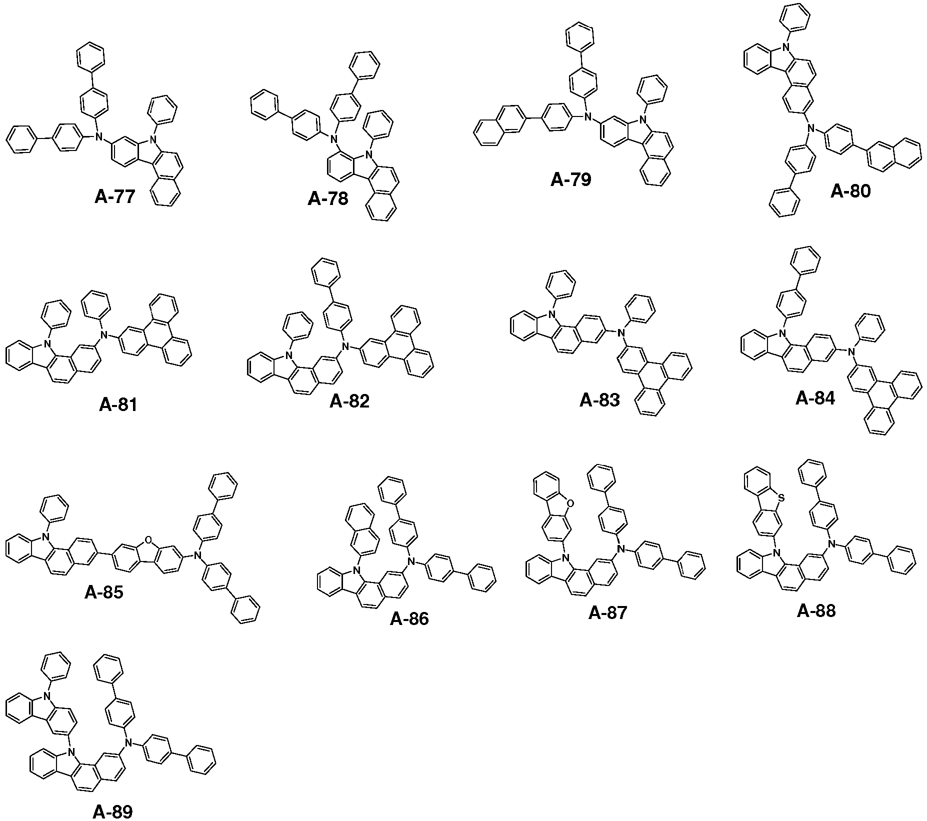

According to an embodiment of the present invention, the second compound is represented by Chemical Formula 3.

The second compound may be a compound having electron characteristics and may exhibit bipolar characteristics with the first compound.

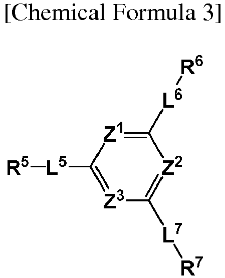

In Chemical Formula 3,

Z¹ to Z³ may each independently be N or CR^{d}, wherein R^{d} is hydrogen, deuterium, a substituted or unsubstituted C1 to C30 alkyl group, a substituted or unsubstituted C6 to C30 aryl group, a substituted or unsubstituted C3 to C30 heterocyclic group, a substituted or unsubstituted silyl group, a substituted or unsubstituted amine group, a halogen, a cyano group, or a combination thereof. In an implementation, at least two of Z¹ to Z³ may be N.

L⁵ to L⁷ may each independently be a single bond, a substituted or unsubstituted C6 to C20 arylene group, a substituted or unsubstituted divalent C2 to C20 heterocyclic group, or a combination thereof,

R⁵ to R⁷ may each independently be a substituted or unsubstituted C6 to C30 aryl group, a substituted or unsubstituted C2 to C30 heterocyclic group, or a combination thereof. In an implementation, at least one of R⁵ to R⁷ is a group represented by Chemical Formula B:

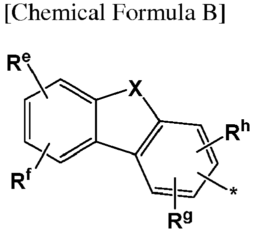

In Chemical Formula B,

X may be O or S,

R^{e} to R^{h} may each independently be hydrogen, deuterium, a substituted or unsubstituted C1 to C30 alkyl group, a substituted or unsubstituted C6 to C30 aryl group, a substituted or unsubstituted C2 to C30 heterocyclic group, a substituted or unsubstituted silyl group, a substituted or unsubstituted amine group, a halogen, a cyano group, or a combination thereof,

R^{e} and R^{f} may each independently be present or may be bonded with each other to form a ring,

R^{g} and R^{h} may each independently be present or may be bonded with each other to form a ring, and

^{∗} is a linking point with one of L⁵ to L⁷.

Without being bound by theory, the second compound may accept an electron when an electric field is applied, that is, it is a compound having electron characteristics, and has a structure where at least one of a fused ring represented by Chemical Formula B is bound to a nitrogen-containing ring, which is a pyrimidine or triazine ring, and thus may exhibit good interface characteristics and hole and electron transport capability along with the first compound and may lower a driving voltage of an organic optoelectronic device.

According to an embodiment of the present invention, two of Z¹ to Z³ may be nitrogen (N) and the remaining one may be CR^{d}.

According to an embodiment of the present invention, Z¹ and Z² may be nitrogen and Z³ may be CR^{d}.

According to an embodiment of the present invention, Z² and Z³ may be nitrogen and Z¹ may be CR^{d}.

According to an embodiment of the present invention, Z¹ and Z³ may be nitrogen and Z² may be CR^{d}.

According to an embodiment of the present invention, Z¹ to Z³ may independently be nitrogen (N).

According to an embodiment of the present invention, L⁵ to L⁷ may independently be a single bond or a substituted or unsubstituted C6 to C20 arylene group.

According to an embodiment of the present invention, L⁵ to L⁷ may independently be a single bond, a substituted or unsubstituted phenylene group, a substituted or unsubstituted biphenylene group, a substituted or unsubstituted terphenylene group, or a substituted or unsubstituted naphthylene group.

According to an embodiment of the present invention, L⁵ to L⁷ may independently be a single bond, a substituted or unsubstituted m-phenylene group, a substituted or unsubstituted p-phenylene group, a substituted or unsubstituted o-phenylene group, a substituted or unsubstituted m-biphenylene group, a substituted or unsubstituted p-biphenylene group, a substituted or unsubstituted o-biphenylene group, a substituted or unsubstituted m-terphenylene group, a substituted or unsubstituted p-terphenylene group, or a substituted or unsubstituted o-terphenylene group. Herein, the "substituted" may for example refer to replacement of at least one hydrogen by deuterium, a C1 to C20 alkyl group, a C6 to C20 aryl group, a halogen, a cyano group, or a combination thereof.

According to an embodiment of the present invention, R⁵ to R⁷ may each independently be a substituted or unsubstituted phenyl group, a substituted or unsubstituted biphenyl group, a substituted or unsubstituted terphenyl group, a substituted or unsubstituted quaterphenyl group, a substituted or unsubstituted naphthyl group, a substituted or unsubstituted anthracenyl group, a substituted or unsubstituted phenanthrenyl group, a substituted or unsubstituted triphenylenyl group, a substituted or unsubstituted fluorenyl group, a substituted or unsubstituted pyridinyl group, a substituted or unsubstituted pyrimidinyl group, a substituted or unsubstituted triazinyl group, or the group represented by Chemical Formula B.

According to an embodiment of the present invention, the group represented by Chemical Formula B may be represented by one of Chemical Formula B-1 to Chemical Formula B-4 according to a bonding position.

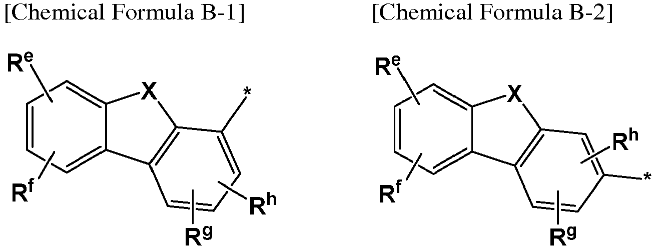

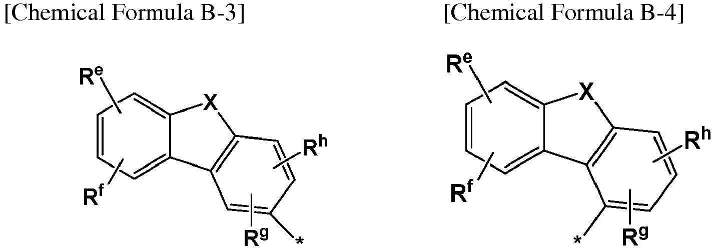

In Chemical Formula B-1 to Chemical Formula B-4, X and R^{e} to R^{h} are the same as described above.

According to an embodiment of the present invention, the group represented by Chemical Formula B may be represented by Chemical Formula B-1 or Chemical Formula B-2.

The second compound may be for example represented by one of Chemical Formula 2A to Chemical Formula 2C according to the number of the group represented by Chemical Formula B.

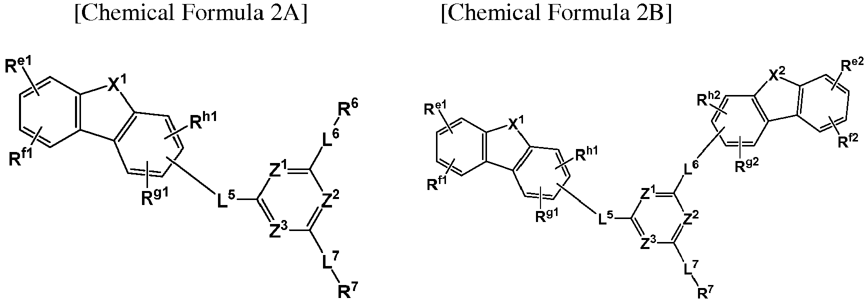

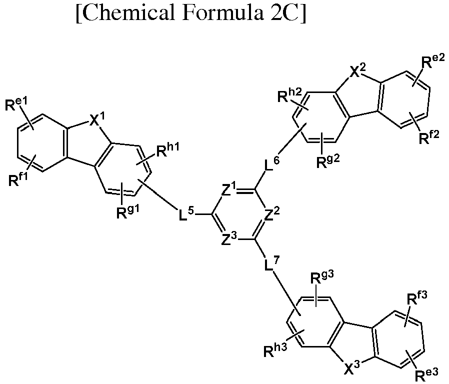

In Chemical Formulae 2A to 2C, Z¹ to Z³, L⁵ to L⁷, R⁶ and R⁷ are the same as described above,

X¹ to X³ may each independently be O or S, and

R^{e1} to R^{e3}, R^{f1} to R^{f3}, R^{g1} to R^{g3}, and R^{h1} to R^{h3} may each independently be hydrogen, deuterium, a substituted or unsubstituted C1 to C30 alkyl group, a substituted or unsubstituted C6 to C30 aryl group, a substituted or unsubstituted C3 to C30 heterocyclic group, a substituted or unsubstituted silyl group, a substituted or unsubstituted amine group, a halogen, a cyano group, or a combination thereof.

According to an embodiment of the present invention, in Chemical Formula 2B, X¹ and X² may be the same or different.

According to an embodiment of the present invention, in Chemical Formula 2B, X¹ and X² may be the same and X¹ and X² may independently be O.

According to an embodiment of the present invention, in Chemical Formula 2B, X¹ and X² may be the same and X¹ and X² may independently be S.

According to an embodiment of the present invention, in Chemical Formula 2B, X¹ and X² may be different and X¹ may be S and X² may be O or X¹ may be O and X² may be S.

According to an embodiment of the present invention, in Chemical Formula 2C, X¹ to X³ may be the same or different.

According to an embodiment of the present invention, in Chemical Formula 2C, X¹ to X³ may be the same and X¹ to X³ may independently be O.

According to an embodiment of the present invention, in Chemical Formula 2C, X¹ to X³ may be the same and X¹ to X³ may independently be S.

According to an embodiment of the present invention, in Chemical Formula 2C, one of X¹ to X³ may be different and two of X¹ to X³ may be S and one of X¹ to X³ may be O or two of X¹ to X³ may be O and one of X¹ to X³ may be S.

According to an embodiment of the present invention, the second compound may be represented by Chemical Formula 2B.

According to an embodiment of the present invention, in Chemical Formula 2B, L⁵ and L⁶ may independently be a single bond.

According to an embodiment of the present invention, Chemical Formula 2B may be represented by Chemical Formula 2B-1.

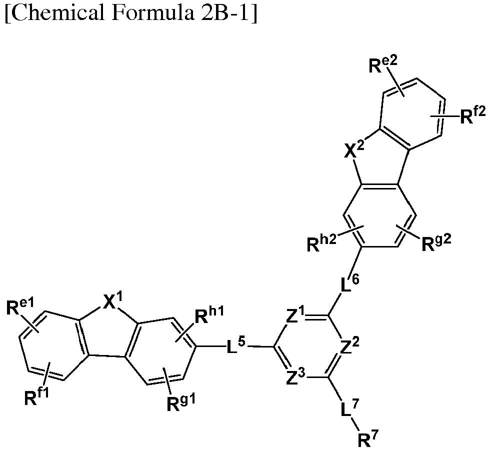

In Chemical Formula 2B-1,

Z¹ to Z³, R⁷, L⁵ to L⁷, R^{e1} to R^{e3}, R^{f1} to R^{f3}, R^{g1} to R^{g3}, and R^{h1} to R^{h3} are the same as described above.

Without being bound by theory, it is believed that the second compound represented by Chemical Formula 2B-1 has an effectively expanded LUMO energy band and larger planarity of a molecular structure, thereby the second compound may become a structure capable of accepting electrons when an electric field is applied, and accordingly an organic optoelectronic device including the second compound may exhibit a lowered driving voltage. Such a LUMO expansion and ring fusion may increase stability of electrons of the pyrimidine or triazine rings, and life-span of a device including the second compound may be further effectively improved.

According to an embodiment of the present invention, in Chemical Formula 2B-1, X¹ and X² may independently be O.

The second compound may be, for example, one of compounds of Group 2.

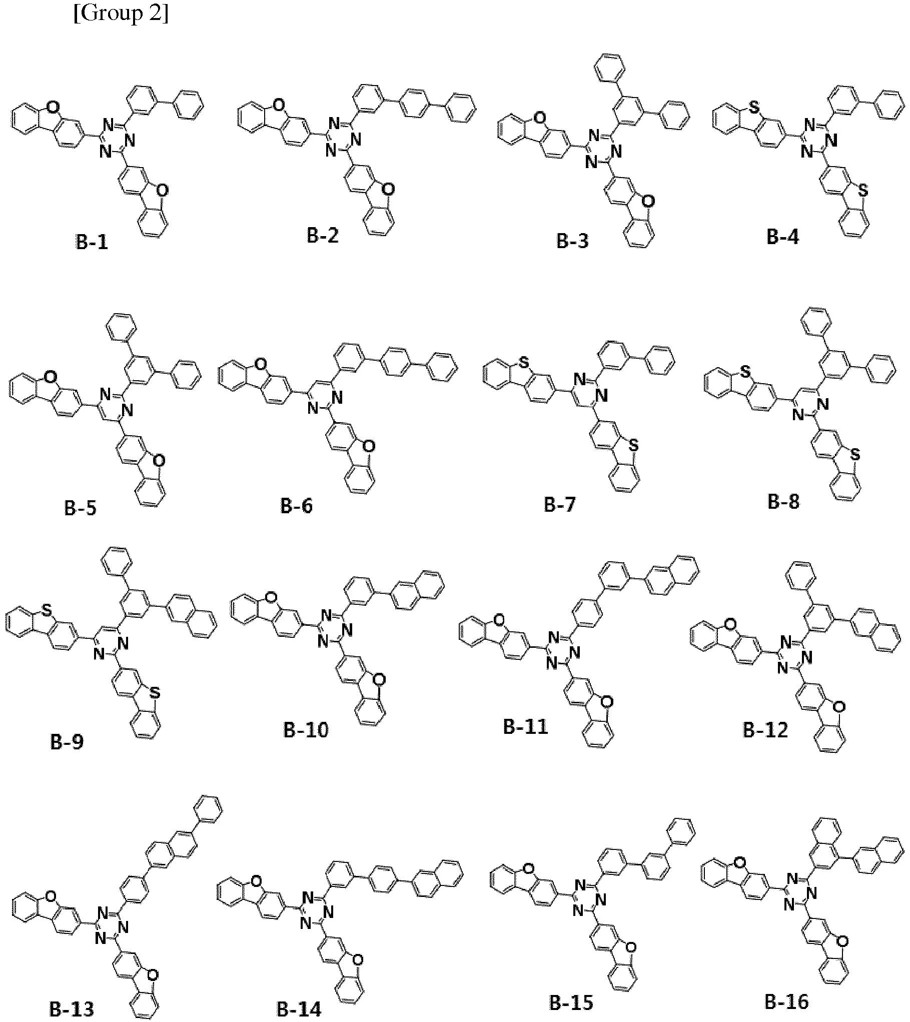

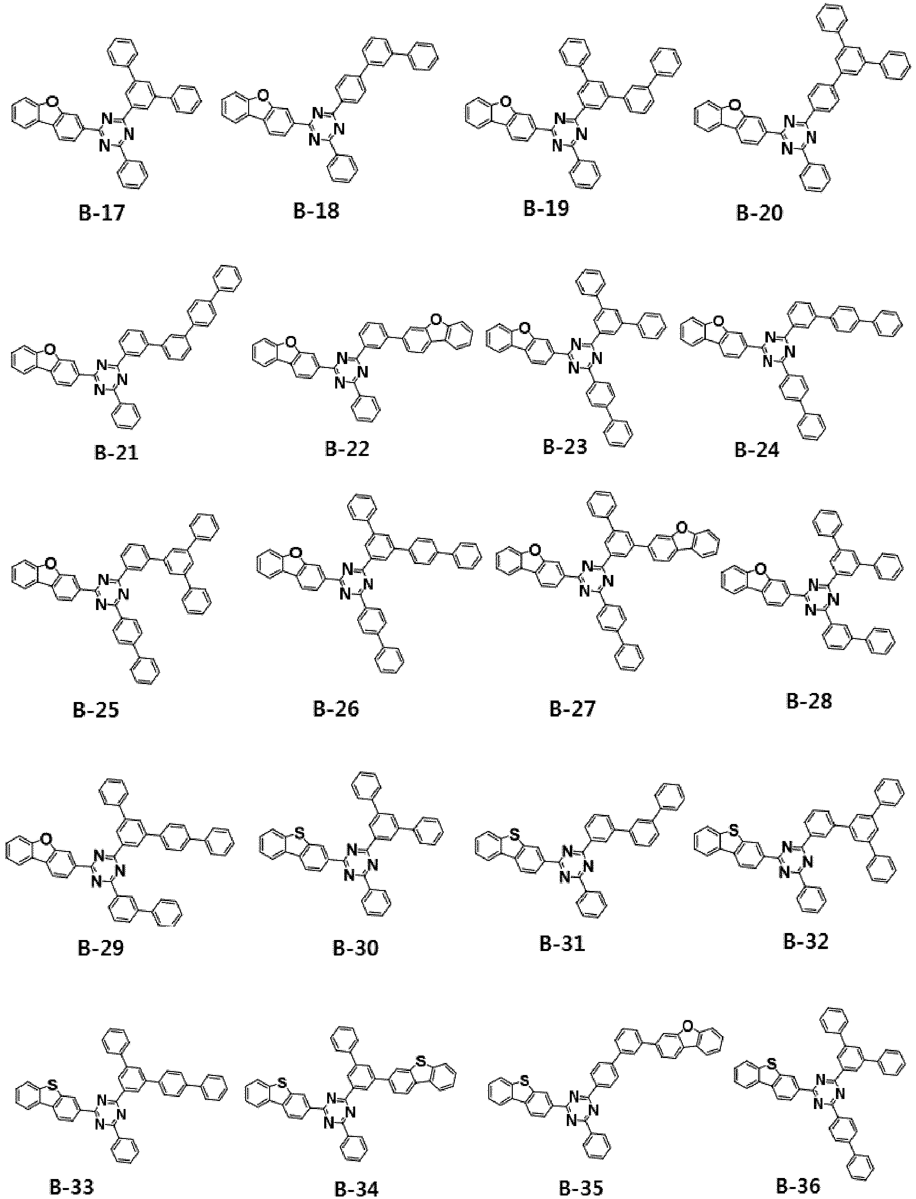

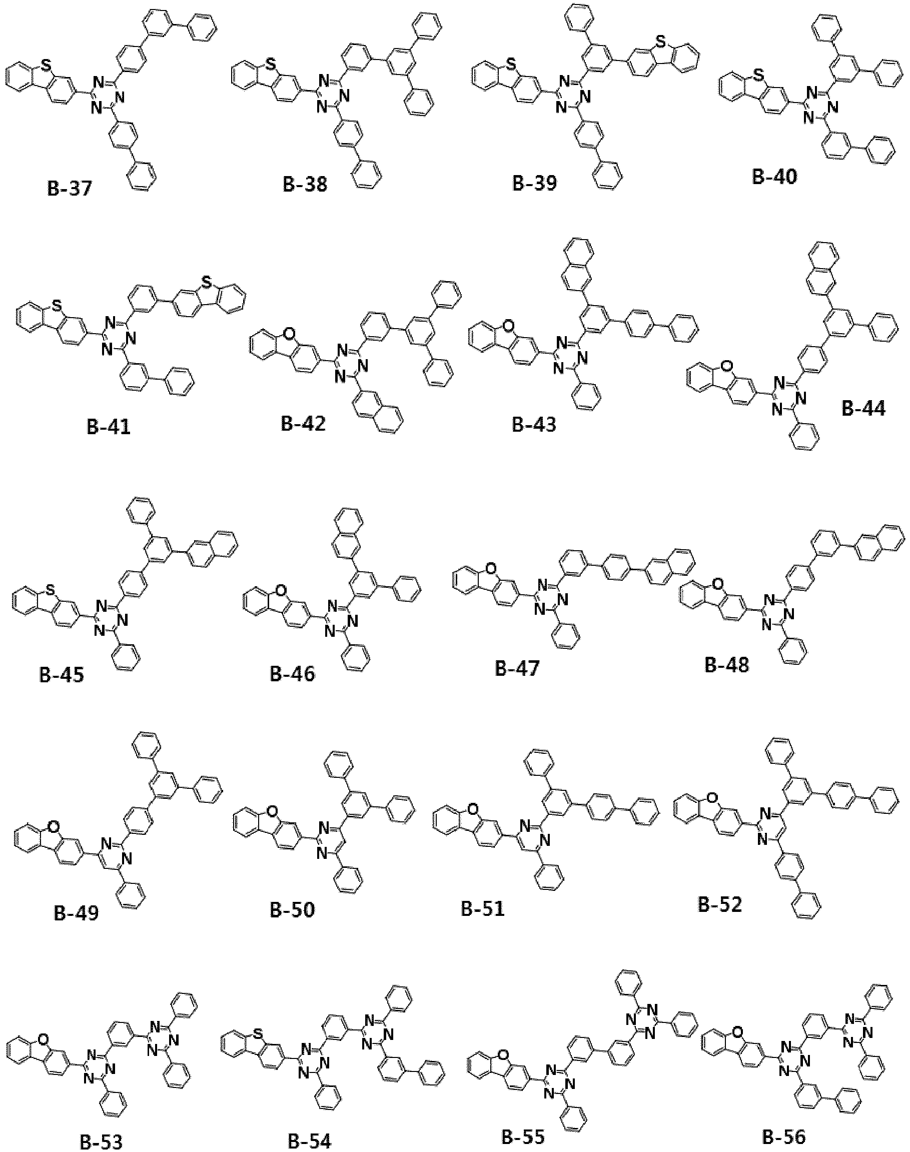

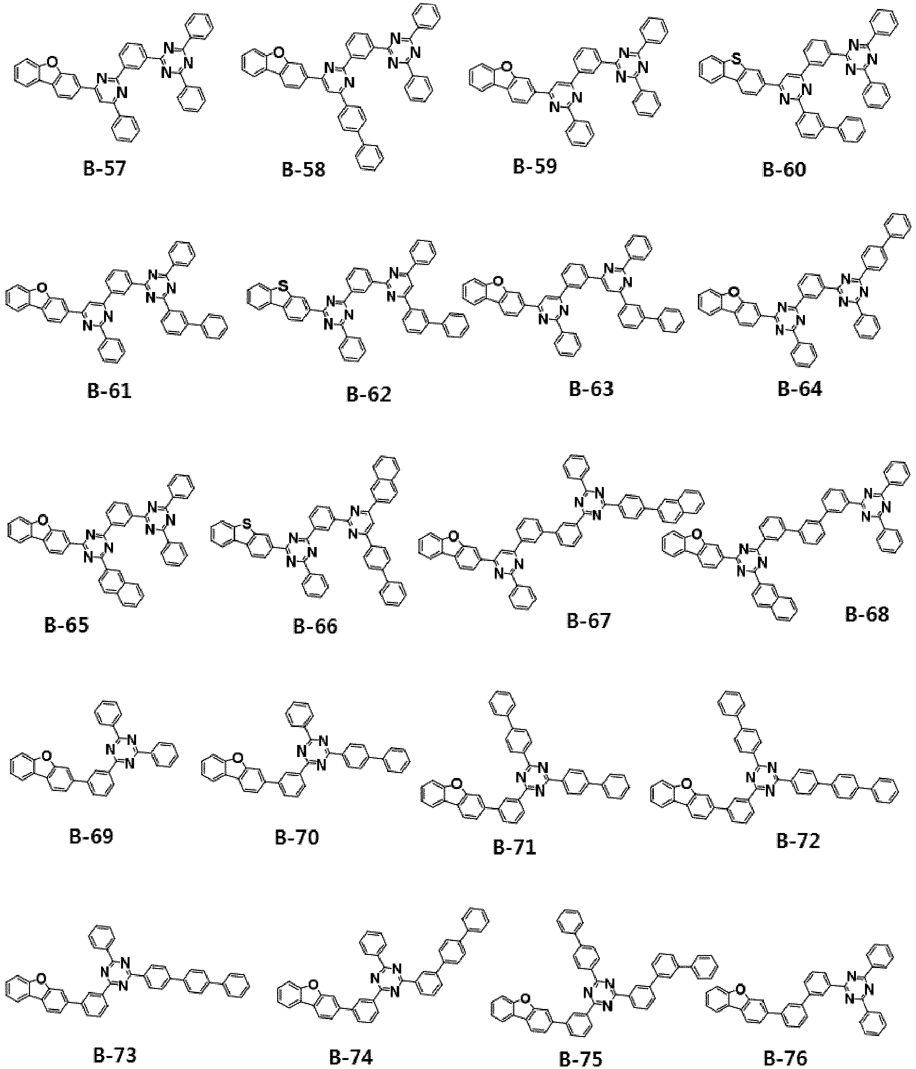

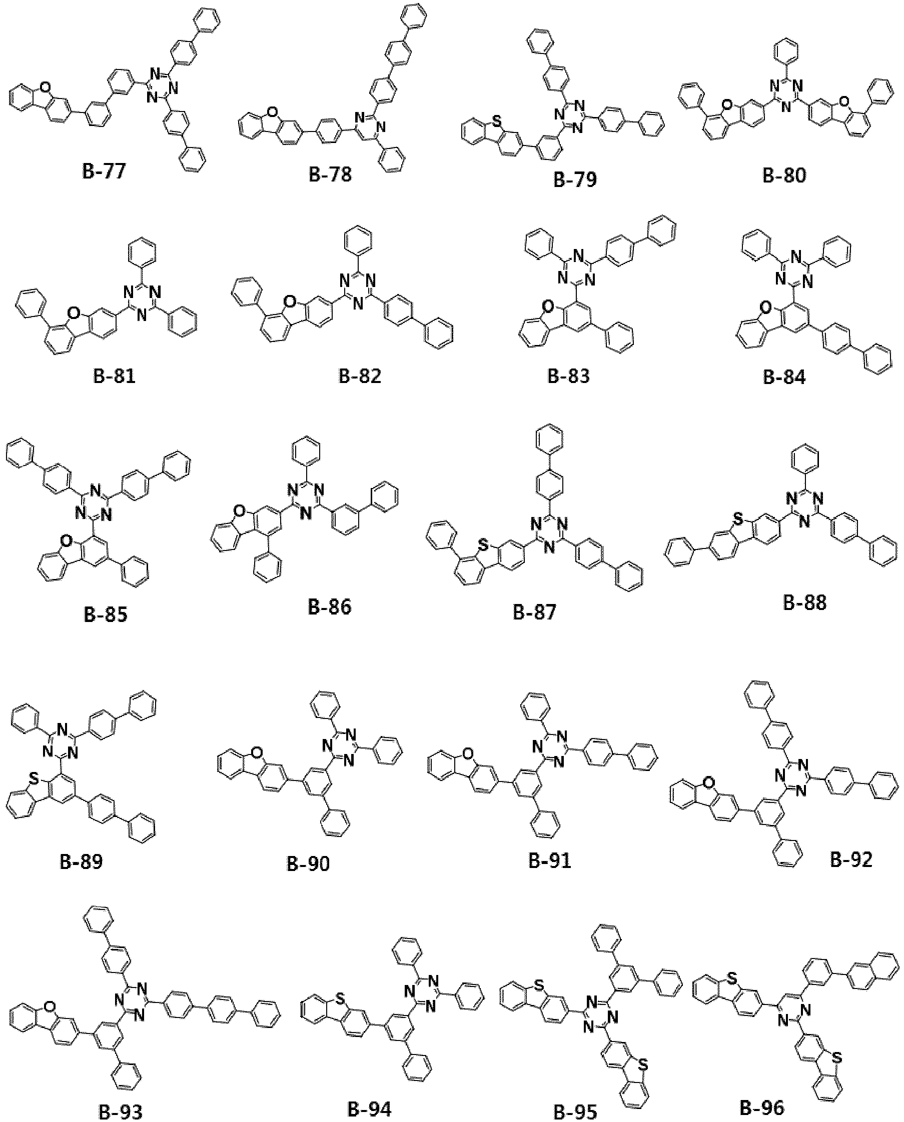

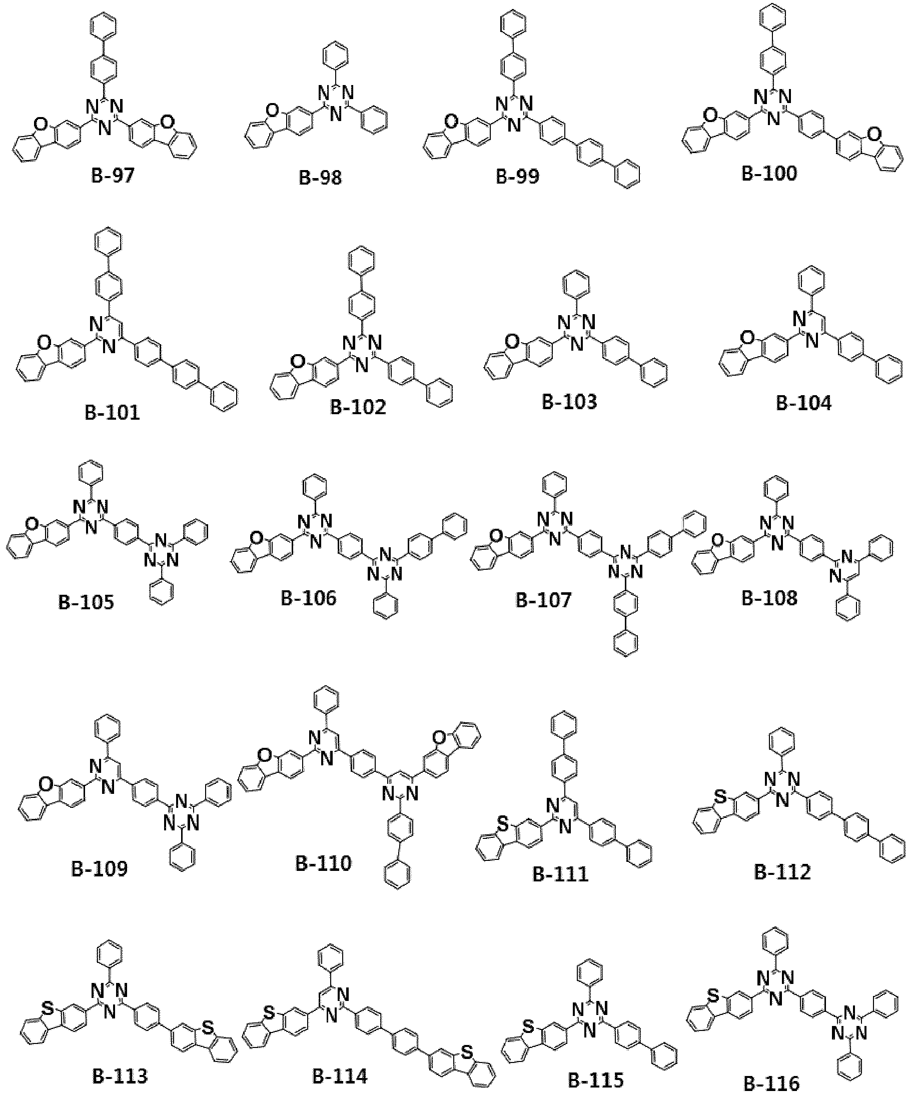

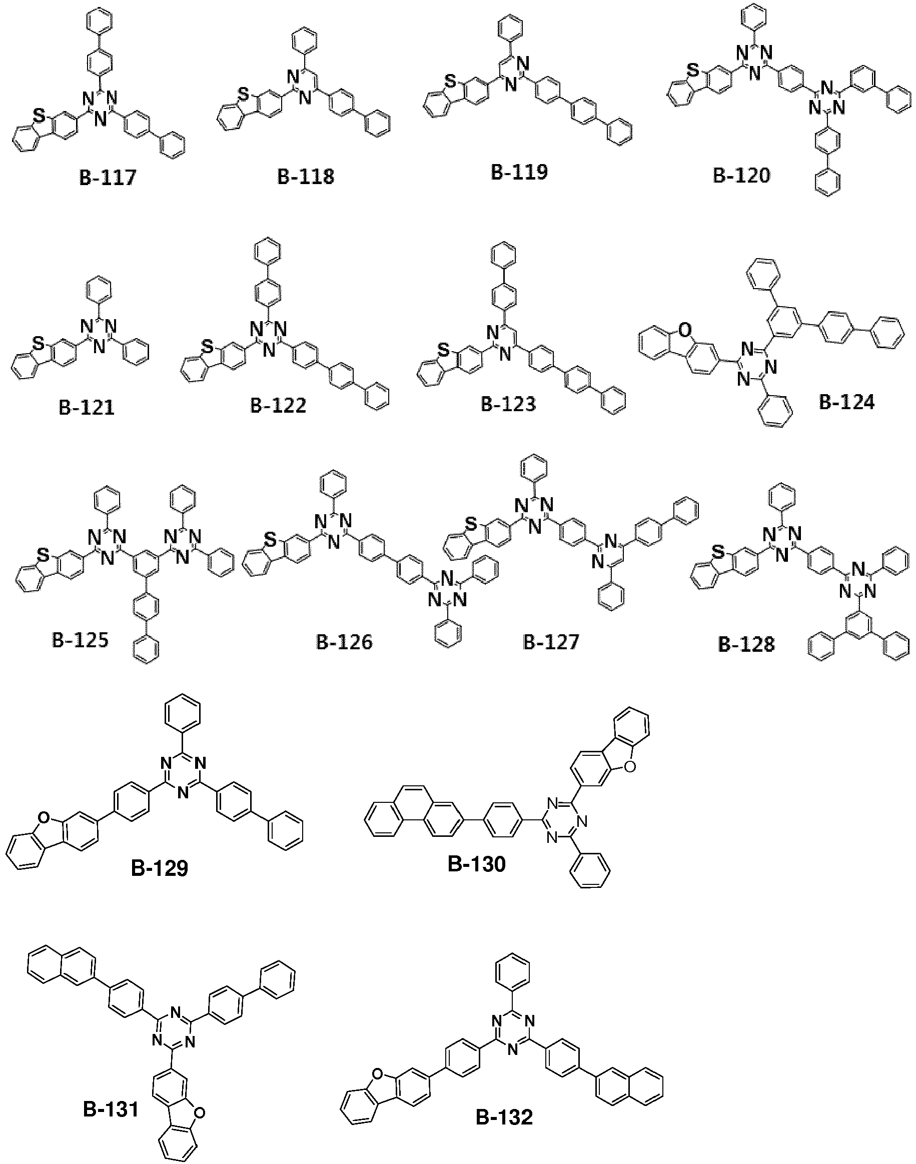

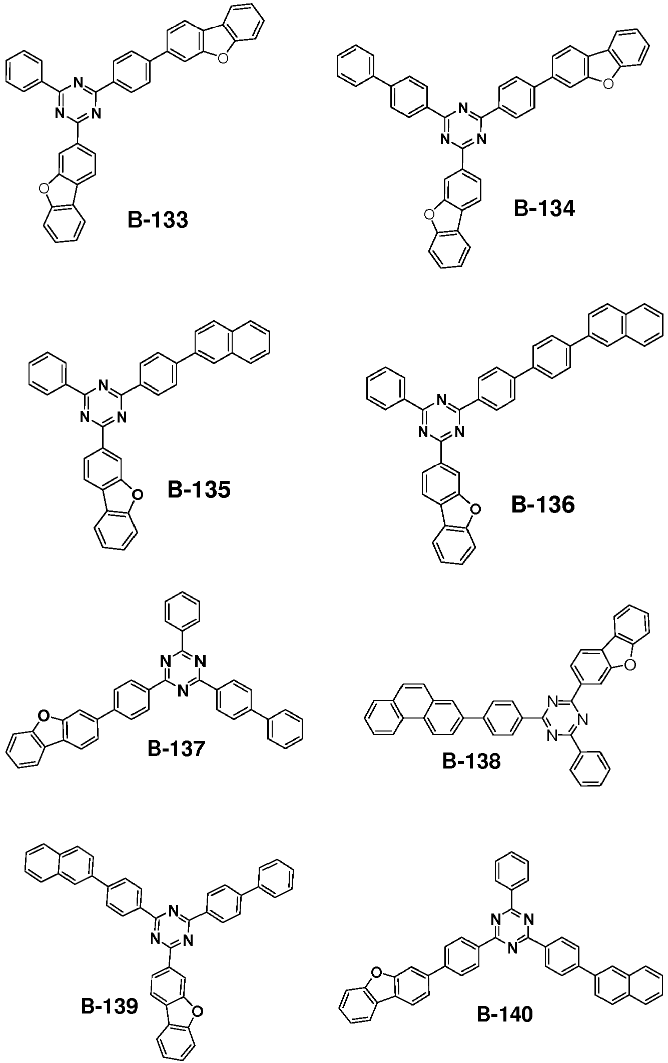

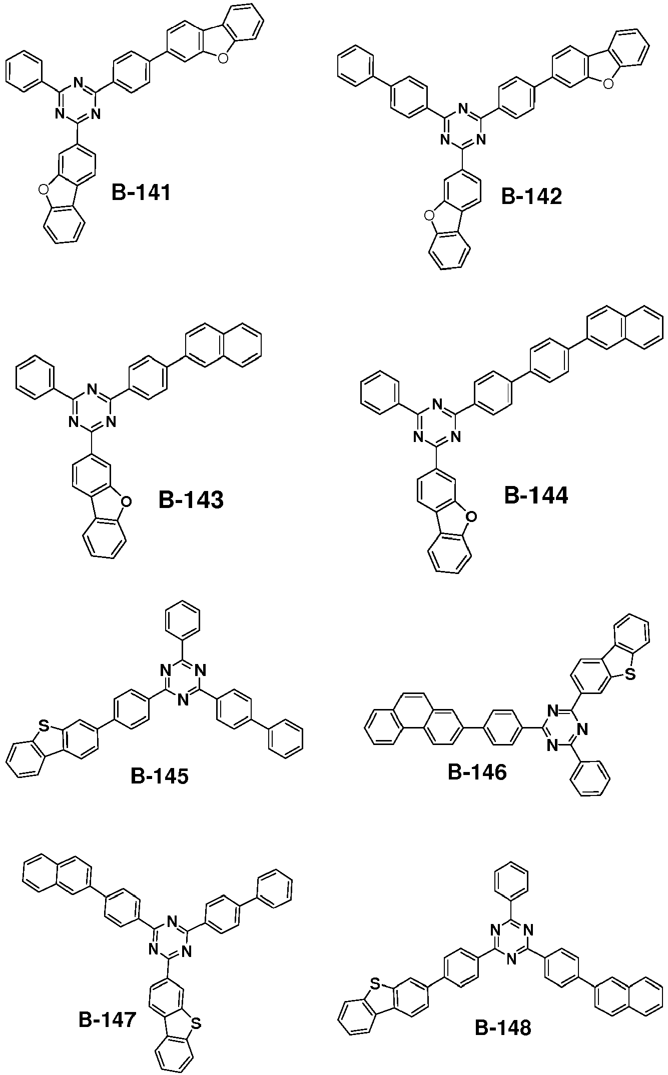

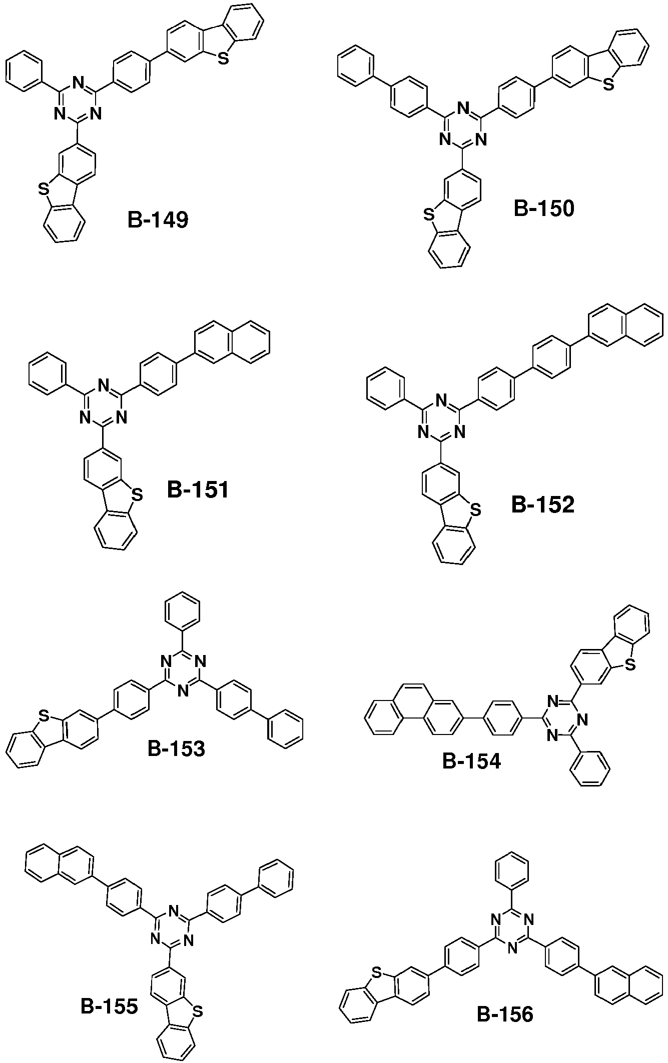

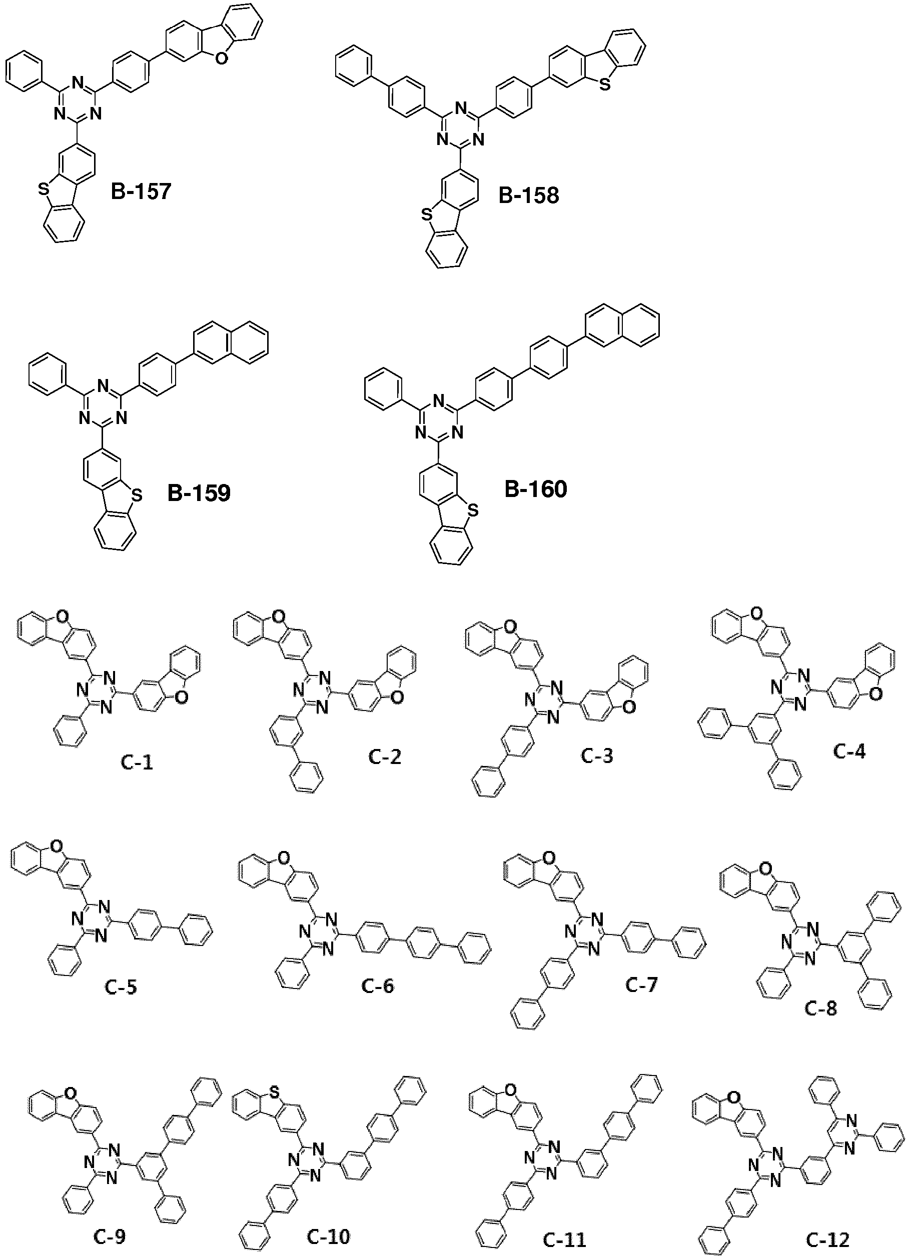

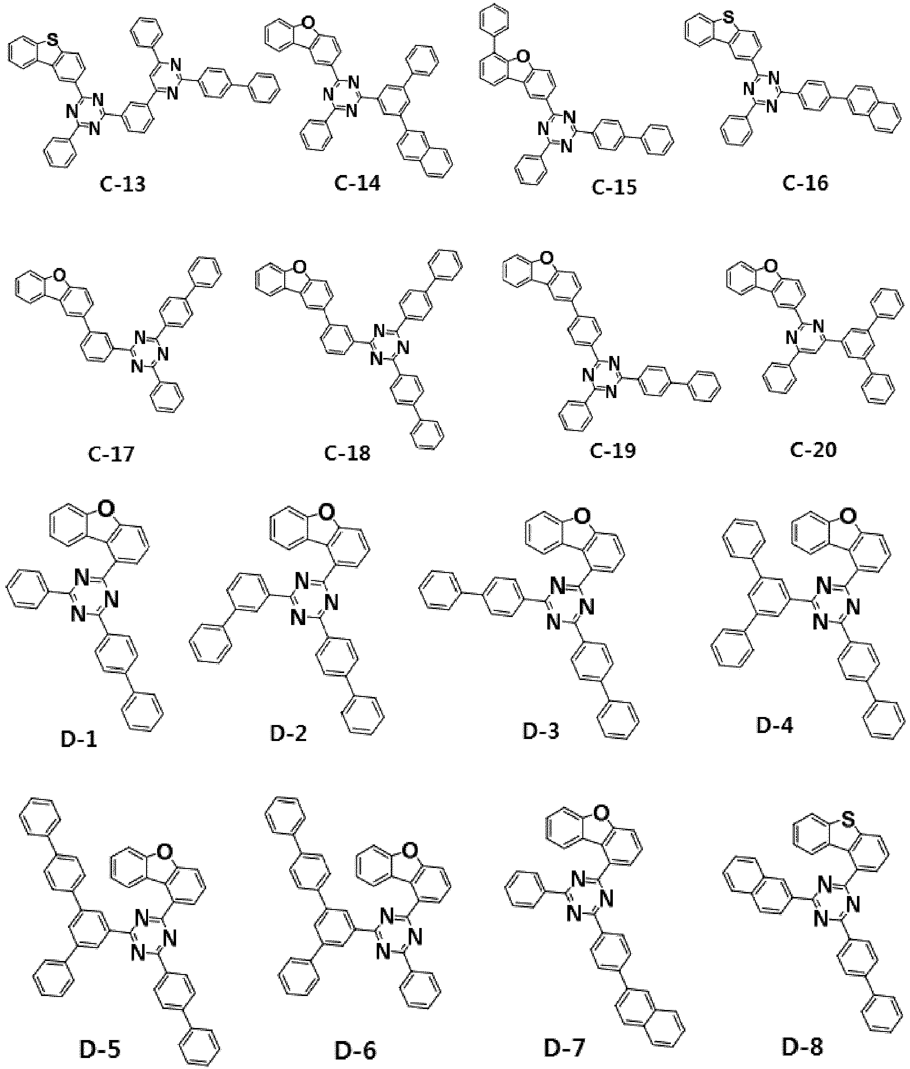

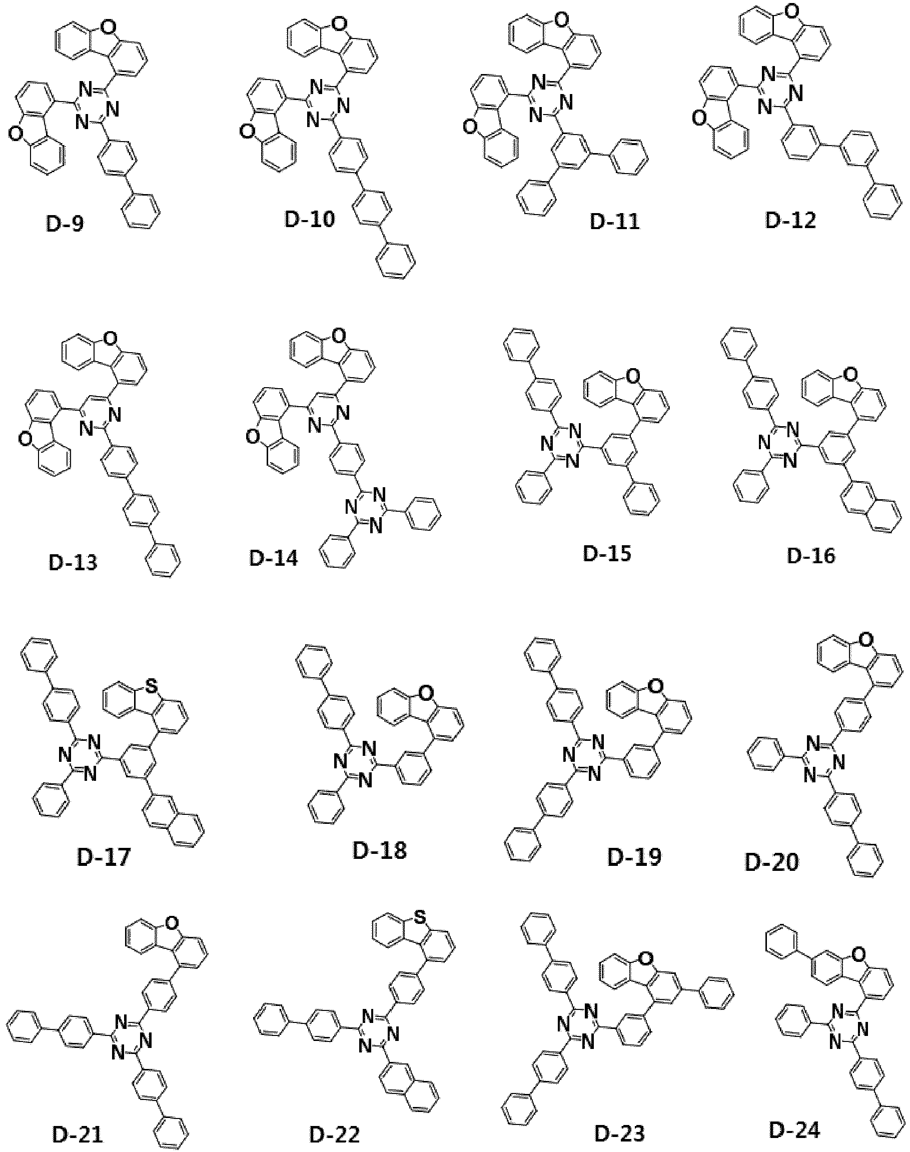

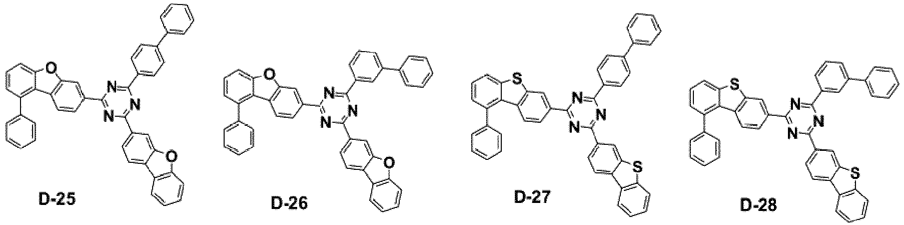

The first compound and the second compound may be included in a weight ratio of, for example, about 1:99 to about 99:1. Within the range, a desirable weight ratio may be adjusted using a hole transport capability of the first compound and an electron transport capability of the second compound to realize bipolar characteristics and thus to improve efficiency and life-span. Within the range, they may be for example included in a weight ratio of about 10:90 to 90:10, about 20:80 to 80:20, about 30:70 to 70:30, about 40:60 to 60:40, or about 50:50. For example, they may be included in a weight ratio of about 50:50 to 60:40, for example, about 50:50 or about 60:40.

According to an embodiment of the present invention, a composition according to an example embodiment includes a compound represented by Chemical Formula 1A-1-b as the first compound and a compound represented by Chemical Formula 2A or Chemical Formula 2B as the second compound.

According to an embodiment of the present invention, in Chemical Formula 1A-1-b, Ar may be a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted phenanthrenyl group, a substituted or unsubstituted triphenylenyl group, a substituted or unsubstituted fluorenyl group, a substituted or unsubstituted dibenzofuranyl group, a substituted or unsubstituted dibenzothiophenyl group, a substituted or unsubstituted carbazolyl group, or a combination thereof, L^{a} and L¹ to L⁴ may independently be a single bond, a substituted or unsubstituted phenylene group, a substituted or unsubstituted biphenylene group, a substituted or unsubstituted terphenylene group, or a substituted or unsubstituted naphthylene group, R^{a}, R¹, R², and R⁴ may independently be hydrogen, deuterium, a cyano group, a substituted or unsubstituted C1 to C30 alkyl group, a substituted or unsubstituted C6 to C30 aryl group, a substituted or unsubstituted C2 to C30 heterocyclic group, or a combination thereof, and R^{b} and R^{c} may independently be a substituted or unsubstituted phenyl group, a substituted or unsubstituted biphenyl group, a substituted or unsubstituted anthracenyl group, a substituted or unsubstituted naphthyl group, a substituted or unsubstituted phenanthrenyl group, a substituted or unsubstituted triphenylenyl group, a substituted or unsubstituted fluorenyl group, a substituted or unsubstituted carbazolyl group, a substituted or unsubstituted dibenzofuranyl group, or a substituted or unsubstituted dibenzothiophenyl group, and

in Chemical Formula 2A and Chemical Formula 2B, Z¹ to Z³ may independently be N or CR^{d}, at least two of Z¹ to Z³ are N, L⁵ to L⁷ may independently be a single bond, a substituted or unsubstituted phenylene group, a substituted or unsubstituted biphenylene group, a substituted or unsubstituted terphenylene group, or a substituted or unsubstituted naphthylene group, R⁶ and R⁷ may independently be a substituted or unsubstituted phenyl group, a substituted or unsubstituted biphenyl group, a substituted or unsubstituted terphenyl group, a substituted or unsubstituted quaterphenyl group, a substituted or unsubstituted naphthyl group, a substituted or unsubstituted anthracenyl group, a substituted or unsubstituted phenanthrenyl group, a substituted or unsubstituted triphenylenyl group, a substituted or unsubstituted fluorenyl group, a substituted or unsubstituted pyridinyl group, a substituted or unsubstituted pyrimidinyl group, a substituted or unsubstituted triazinyl group, a substituted or unsubstituted dibenzofuranyl group, or a substituted or unsubstituted dibenzothiophenyl group, X¹ and X² may independently be O or S, and R^{d}, R^{e1}, R^{e2}, R^{f1}, R^{f2}, R^{g1}, R^{g2}, R^{h1}, and R^{h2} may each independently be hydrogen, deuterium, a substituted or unsubstituted C1 to C30 alkyl group, a substituted or unsubstituted C6 to C30 aryl group, a substituted or unsubstituted C2 to C30 heterocyclic group, a substituted or unsubstituted silyl group, a substituted or unsubstituted amine group, a halogen, a cyano group, or a combination thereof.

According to an embodiment of the present invention, Chemical Formula 2B may be represented by Chemical Formula 2B-1.

According to an embodiment of the present invention, the composition may further include at least one other compound in addition to the first compound and the second compound.

According to an embodiment of the present invention, the composition may further include a dopant. The dopant may be for example a phosphorescent dopant, for example a red, green, or blue phosphorescent dopant, for example a red phosphorescent dopant.

The dopant is a material mixed in a small amount with the first compound and the second compound to cause light emission. The dopant may be a material such as a metal complex that emits light by multiple excitations into a triplet or more. The dopant may be, for example an inorganic, organic, or organic/inorganic compound, and one or more kinds thereof may be used.

Examples of the dopant may be a phosphorescent dopant and examples of the phosphorescent dopant may be an organic metal compound including Ir, Pt, Os, Ti, Zr, Hf, Eu, Tb, Tm, Fe, Co, Ni, Ru, Rh, Pd, or a combination thereof. The phosphorescent dopant may be, for example a compound represented by Chemical Formula Z.

### [Chemical Formula Z]

L⁸MX⁴

In Chemical Formula Z, M is a metal, and L⁸ and X⁴ may be the same or different, and are a ligand to form a complex compound with M.

The M may be for example Ir, Pt, Os, Ti, Zr, Hf, Eu, Tb, Tm, Fe, Co, Ni, Ru, Rh, Pd, or a combination thereof, and the L⁸ and X⁴ may be, for example a bidendate ligand.

The composition may be applied by, for example, a dry film formation method such as chemical vapor deposition (CVD).

Hereinafter, an organic optoelectronic device including the composition is described.

An organic optoelectronic device according to an example embodiment may be a device to convert electrical energy into photo energy or vice versa, and may be, for example, an organic photoelectric device, an organic light emitting diode, an organic solar cell, an organic photo conductor drum, etc.

Herein, an organic light emitting diode as one example of an organic optoelectronic device is described referring to drawings, which merely serves to illustrate the invention and is not binding.

FIGS. 1 and 2 illustrate cross-sectional views of organic light emitting diodes according to example embodiments.

Referring to FIG. 1, an organic optoelectronic device 100 according to an example embodiment includes an anode 120 and a cathode 110 facing each other, and an organic layer 105 disposed between the anode 120 and the cathode 110.

The anode 120 may be made of a conductor having a large work function to help hole injection, and may be for example a metal, a metal oxide and/or a conductive polymer. The anode 120 may be, for example a metal such as nickel, platinum, vanadium, chromium, copper, zinc, gold, and the like or an alloy thereof; metal oxide such as zinc oxide, indium oxide, indium tin oxide (ITO), indium zinc oxide (IZO), and the like; a combination of metal and oxide such as ZnO and Al or SnO₂ and Sb; a conductive polymer such as poly(3-methylthiophene), poly(3,4-(ethylene-1,2-dioxy)thiophene) (PEDOT), polypyrrole, and polyaniline.

The cathode 110 may be made of a conductor having a small work function to help electron injection, and may be for example a metal, a metal oxide and/or a conductive polymer. The cathode 110 may be for example a metal such as magnesium, calcium, sodium, potassium, titanium, indium, yttrium, lithium, gadolinium, aluminum, silver, tin, lead, cesium, barium, and the like or an alloy thereof; a multi-layer structure material such as LiF/Al, LiO₂/Al, LiF/Ca, LiF/Al and BaF₂/Ca.

The organic layer 105 may include a light emitting layer 130 including the composition.

The composition may be for example a red light emitting composition.

The light emitting layer 130 may include, for example the first compound and the second compound as a phosphorescent host.

Referring to FIG. 2, an organic light emitting diode 200 according to an example embodiment further includes a hole auxiliary layer 140 in addition to the light emitting layer 130. The hole auxiliary layer 140 may further increase hole injection and/or hole mobility while blocking electrons between the anode 120 and the light emitting layer 130.

The hole auxiliary layer 140 may include, for example, at least one compound of Group D, below.

For example, the hole auxiliary layer 140 may include a hole transport layer between the anode 120 and the light emitting layer 130 and a hole transport auxiliary layer between the light emitting layer 130 and the hole transport layer, and at least one compound of Group D may be included in the hole transport auxiliary layer.

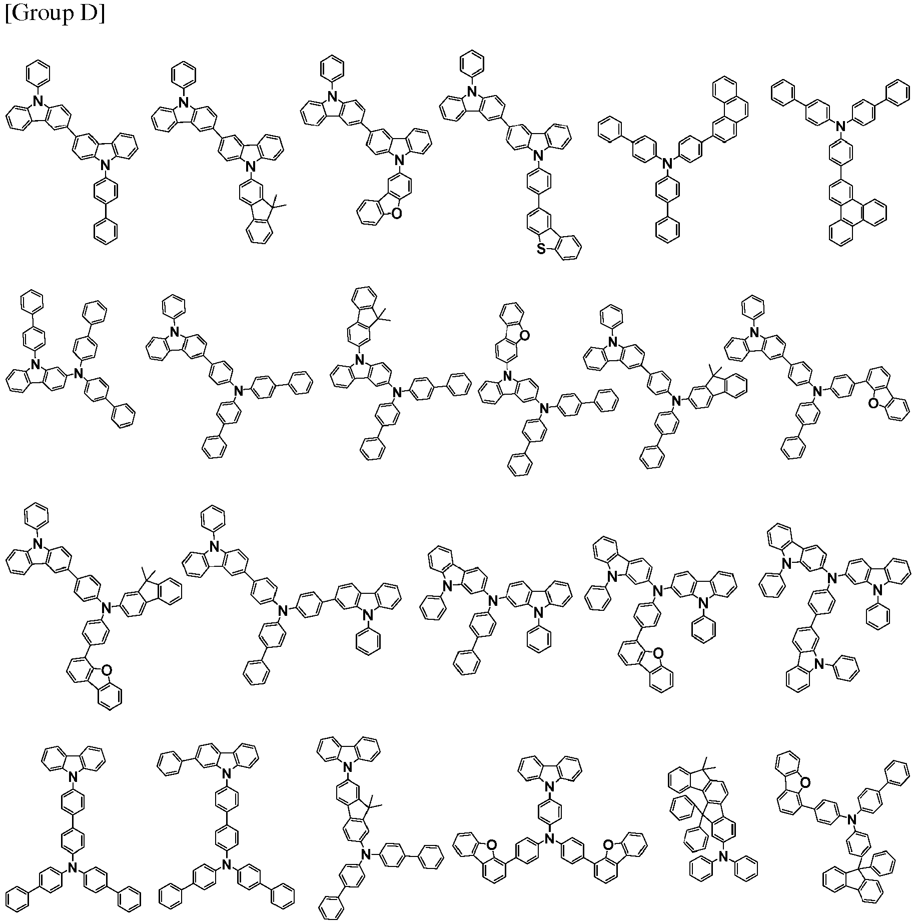

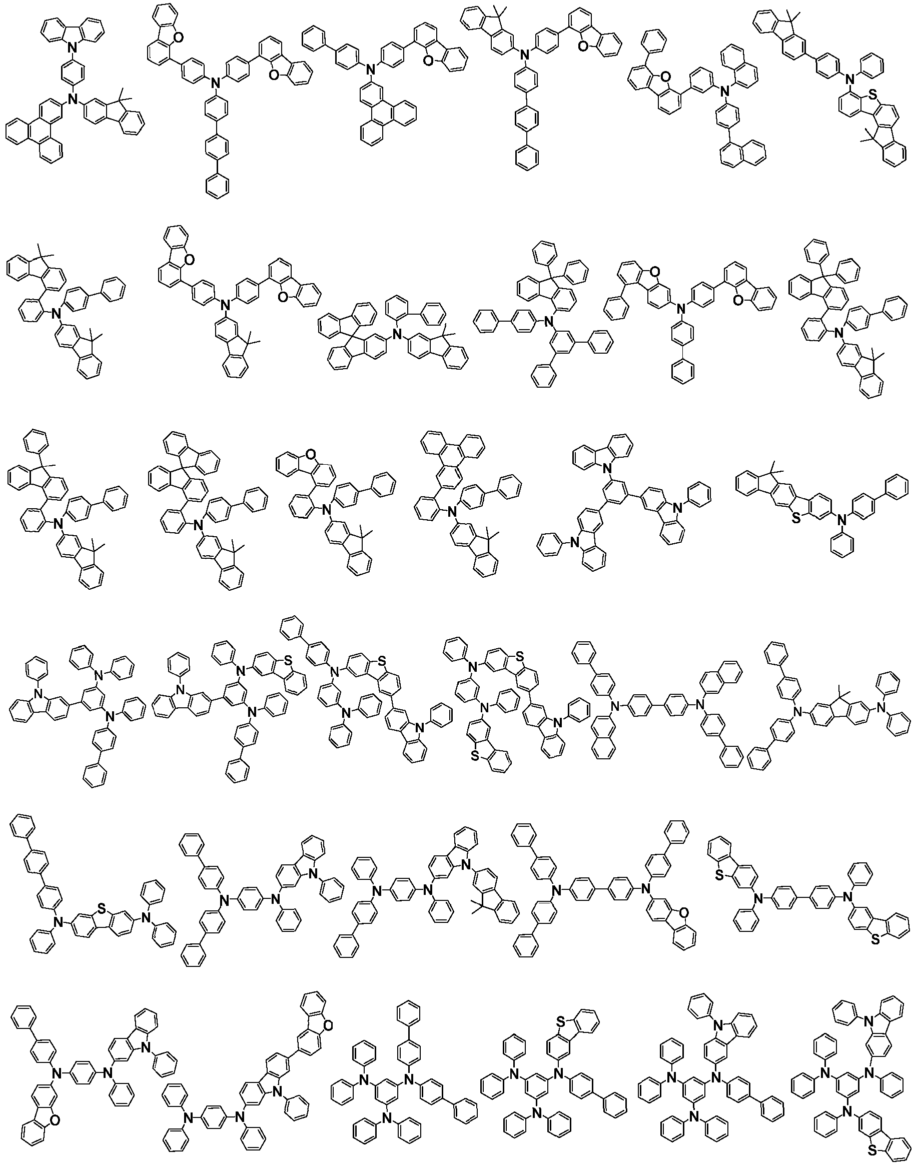

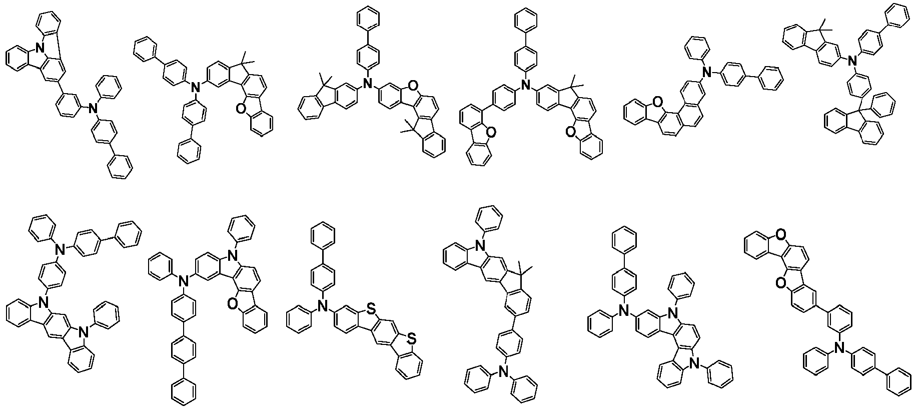

In the hole transport auxiliary layer, known compounds disclosed in U.S. 5,061,569 B2, JP1993-009471A, WO1995-009147A1, JP1995-126615A, JP1998-095973A, and the like and compounds similar thereto may be used in addition to the compound.

In an embodiment, in FIG. 1 or 2, an organic light emitting diode may further include an electron transport layer, an electron injection layer, or a hole injection layer as the organic layer 105.

The organic light emitting diodes 100 and 200 may be manufactured by forming an anode or a cathode on a substrate, forming an organic layer using a dry film formation method such as a vacuum deposition method (evaporation), sputtering, plasma plating, and ion plating, and forming a cathode or an anode thereon.

The organic light emitting diode may be applied to an organic light emitting display device.

The following Examples and Comparative Examples are provided in order to highlight characteristics of one or more embodiments, but it will be understood that the Examples and Comparative Examples are not to be construed as limiting the scope of the embodiments, nor are the Comparative Examples to be construed as being outside the scope of the embodiments. Further, it will be understood that the embodiments are not limited to the particular details described in the Examples and Comparative Examples.

### (Preparation of First Compound)

Synthesis Example 1: Synthesis of Compound A-2

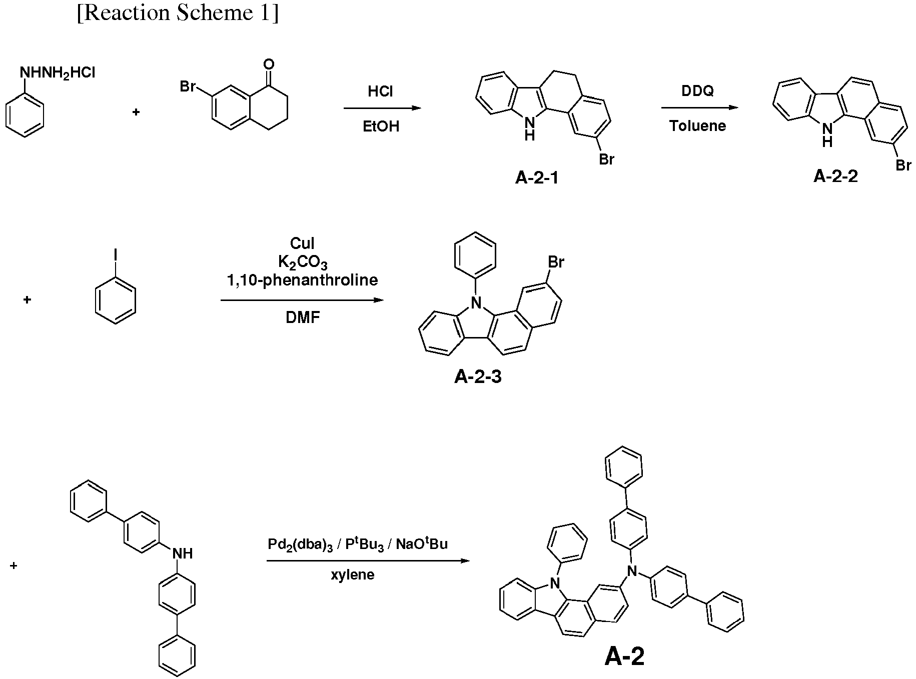

### a) Synthesis of Intermediate A-2-1

Phenylhydrazine hydrochloride (70.0 g, 484.1 mmol) and 7-bromo-3,4-dihydro-2H-naphthalen-1-one (108.9 g, 484.1 mmol) were put in a round-bottomed flask and then dissolved in ethanol (1200 ml). 60 mL of hydrochloric acid was slowly added in a dropwise fashion thereto at room temperature, and the obtained mixture was stirred at 90 °C for 12 hours. When a reaction was complete, the solvent was removed therefrom under a reduced pressure, and an extract was obtained therefrom by using an excess amount of EA. After removing the organic solvent under a reduced pressure, the extract was stirred in a small amount of methanol and then filtered to obtain 95.2 g of Intermediate A-2-1 (66 %).

### b) Synthesis of Intermediate A-2-2

Intermediate A-2-1 (95.2 g, 319.3 mmol) and 2,3-dichloro-5,6-dicyano-1,4-benzoquinone (108.7 g, 478.9 mmol) were put in a round-bottomed flask and dissolved in 600 ml of toluene. The solution was stirred at 80 °C for 12 hours. When a reaction was complete, a reaction solvent was removed, and the rest thereof was treated through column chromatography to obtain 41.3 g of Intermediate A-2-2 (44 %).

### c) Synthesis of Intermediate A-2-3

Intermediate A-2-2 (41.3 g, 139.0 mmol), iodobenzene (199.2 g, 976.0 mmol), CuI (5.31 g, 28.0 mmol), K₂CO₃ (28.9 g, 209.0 mmol), and 1,10-phenanthroline (5.03 g, 28.0 mmol) were put in a round-bottomed flask and dissolved in 500 ml of DMF. The solution was stirred at 180 °C for 12 hours. When a reaction was complete, the reaction solvent was removed therefrom under a reduced pressure, and then a product therefrom was dissolved in dichloromethane and silica gel-filtered. After concentrating dichloromethane, the product was recrystallized with hexane to obtain 39.0 g of Intermediate A-2-3 (75 %).

### d) Synthesis of Compound A-2

Intermediate A-2-3 (23.2 g, 62.5 mmol), bis-biphenyl-4-yl-amine (21.1 g, 65.6 mmol), sodium t-butoxide (NaOtBu) (9.0 g, 93.8 mmol), Pd₂(dba)₃ (3.4 g, 3.7 mmol), and tri t-butylphosphine (P(tBu)₃) (4.5 g, 50 % in toluene) were put in xylene (300 mL) and then heated and refluxed under a nitrogen flow for 12 hours. After removing the xylene, 200 mL of methanol was added thereto to crystallize a solid, the solid was filtered, dissolved in toluene, and filtered again with silica gel/Celite, and then the organic solvent in an appropriate amount was concentrated to obtain 29 g of Compound A-2 (76 %).

### LC/MS calculated for: C46H32N2 Exact Mass: 612.26 found for 612.32 [M+H]

### Synthesis Example 2: Synthesis of Compound A-3

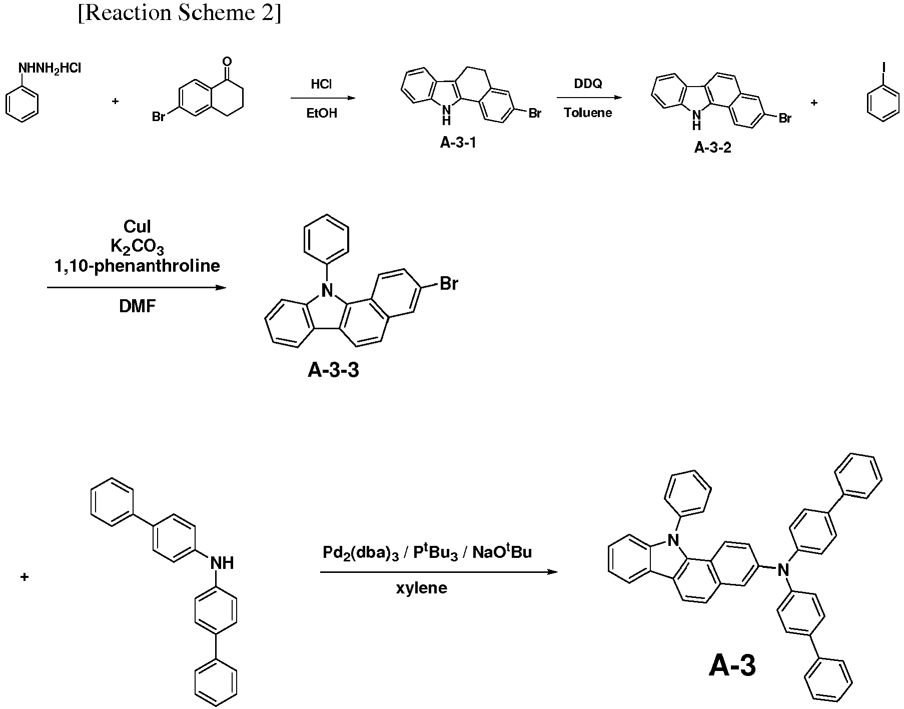

### a) Synthesis of Intermediate A-3-1

Intermediate A-3-1 was synthesized according to the same method as the a) of Synthesis Example 1 by respectively using phenylhydrazinehydrochloride and 6-bromo-3,4-dihydro-2H-naphthalen-1-one by 1.0 equivalent.

### b) Synthesis of Intermediate A-3-2

Intermediate A-3-2 was synthesized according to the same method as the b) of Synthesis Example 1 by using Intermediate A-3-1 and 2,3-dichloro-5,6-dicyano-1,4-benzoquinone in an equivalent ratio of 1:1.5.

### c) Synthesis of Intermediate A-3-3

Intermediate A-3-3 was synthesized according to the same method as the c) of Synthesis Example 1 by using Intermediate A-3-2 and iodobenzene in an equivalent ratio of 1:3.

### d) Synthesis of Compound A-3

Compound A-3 was synthesized according to the same method as the d) of Synthesis Example 1 by using Intermediate A-3-3 and bis-biphenyl-4-yl-amine in an equivalent ratio of 1:1.

### LC/MS calculated for: C46H32N2 Exact Mass: 612.26 found for 612.28 [M+H]

Synthesis Example 3: Synthesis of Compound A-5

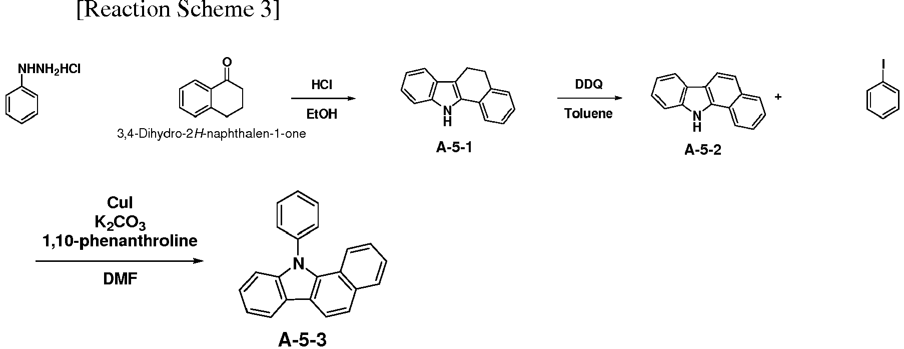

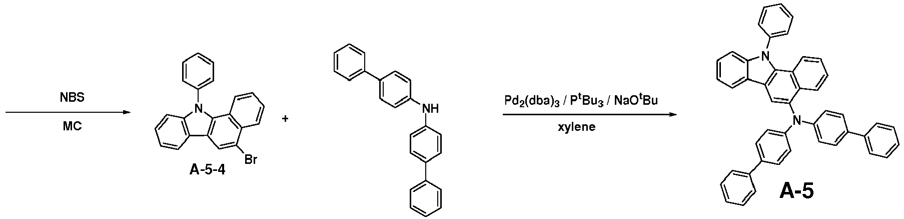

### a) Synthesis of Intermediate A-5-1

Intermediate A-5-1 was synthesized according to the same method as the a) of Synthesis Example 1 by respectively using phenylhydrazinehydrochloride and 3,4-dihydro-2H-naphthalen-1-one by 1.0 equivalent.

### b) Synthesis of Intermediate A-5-2

Intermediate A-5-2 was synthesized according to the same method as the b) of Synthesis Example 1 by respectively using Intermediate A-5-1 and 2,3-dichloro-5,6-dicyano-1,4-benzoquinone in an equivalent ratio of 1:1.5.

### c) Synthesis of Intermediate A-5-3

Intermediate A-5-3 was synthesized according to the same method as the c) of Synthesis Example 1 by using Intermediate A-5-2 and iodobenzene in an equivalent ratio of 1:3.

### d) Synthesis of Intermediate A-5-4

Intermediate A-5-3 (23.6 g, 80.6 mmol) was put in a round-bottomed flask and then dissolved in 300 mL of dichloromethane. Subsequently, N-bromosuccinimide (NBS) (14.1 g, 79.0 mmol) was dissolved in 100 mL of DMF, the solution was slowly added to the above solution in a dropwise fashion, and the mixed solution was stirred at room temperature for 2 hours. When a reaction was complete, the reaction solvent was removed, and a product therefrom was treated through column chromatography to obtain 25 g of Intermediate A-5-4 (83 %).

### e) Synthesis of Compound A-5

Compound A-5 was synthesized according to the same method as the d) of Synthesis Example 1 by using Intermediate A-5-4 and bis-biphenyl-4-yl-amine in an equivalent ratio of 1:1.

### LC/MS calculated for: C46H32N2 Exact Mass: 612.26 found for 612.33 [M+H]

Synthesis Example 4: Synthesis of Compound A-7

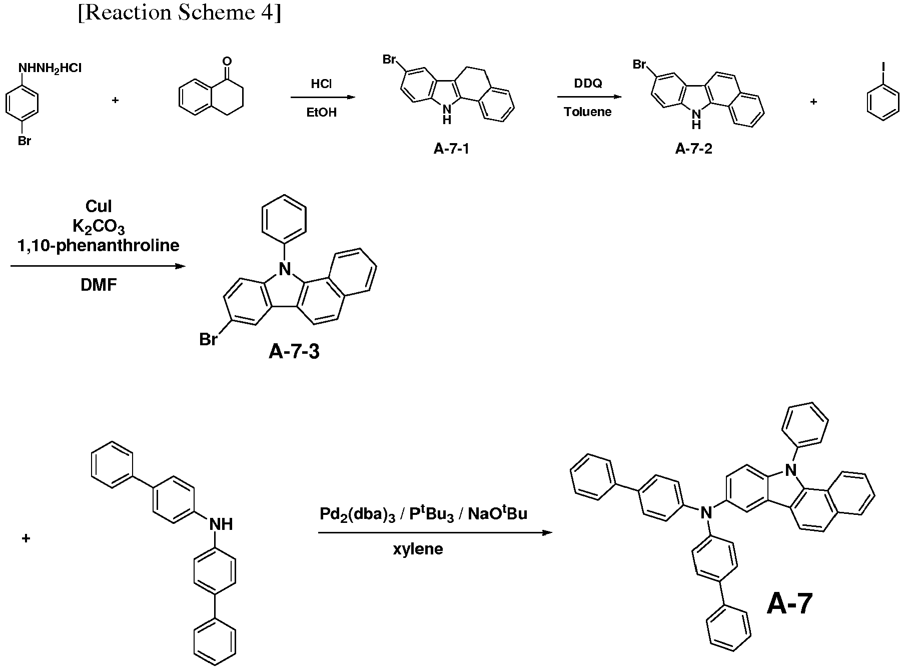

### a) Synthesis of Intermediate A-7-1

Intermediate A-7-1 was synthesized according to the same method as the a) of Synthesis Example 1 by respectively using 4-bromophenylhydrazinehydrochloride and 3,4-dihydro-2H-naphthalen-1-one by 1.0 equivalent.

### b) Synthesis of Intermediate A-7-2

Intermediate A-7-2 was synthesized according to the same method as the b) of Synthesis Example 1 by using Intermediate A-7-1 and 2,3-dichloro-5,6-dicyano-1,4-benzoquinone in an equivalent ratio of 1:1.5.

### c) Synthesis of Intermediate A-7-3

Intermediate A-7-3 was synthesized according to the same method as the c) of Synthesis Example 1 by using Intermediate A-7-2 and iodobenzene in an equivalent ratio of 1:3.

### d) Synthesis of Compound A-7

Compound A-7 was synthesized according to the same method as the d) of Synthesis Example 1 by using Intermediate A-7-3 and bis-biphenyl-4-yl-amine in an equivalent ratio of 1:1.

### LC/MS calculated for: C46H32N2 Exact Mass: 612.26 found for 612.30 [M+H]

Synthesis Example 5: Synthesis of Compound A-8

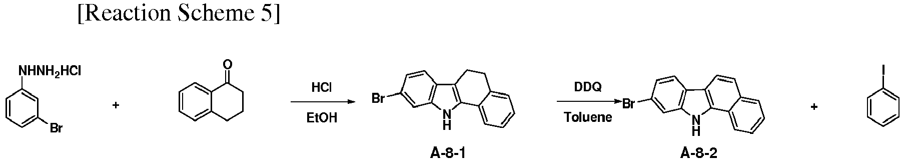

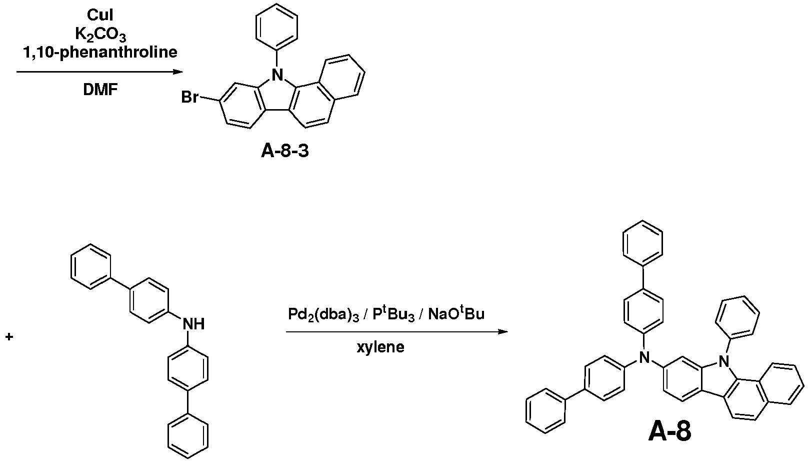

### a) Synthesis of Intermediate A-8-1

Intermediate A-8-1 was synthesized according to the same method as the a) of Synthesis Example 1 by respectively using 3-bromophenylhydrazinehydrochloride and 3,4-dihydro-2H-naphthalen-1-one by 1.0 equivalent.

### b) Synthesis of Intermediate A-8-2

Intermediate A-8-2 was synthesized according to the same method as the b) of Synthesis Example 1 by using Intermediate A-8-1 and 2,3-dichloro-5,6-dicyano-1,4-benzoquinone in an equivalent ratio of 1:1.5.

### c) Synthesis of Intermediate A-8-3

Intermediate A-8-3 was synthesized according to the same method as the c) of Synthesis Example 1 by using Intermediate A-8-2 and iodobenzene in an equivalent ratio of 1:3.

### d) Synthesis of Compound A-8

Compound A-8 was synthesized according to the same method as the d) of Synthesis Example 1 by using Intermediate A-8-3 and bis-biphenyl-4-yl-amine in an equivalent ratio of 1:1.

### LC/MS calculated for: C46H32N2 Exact Mass: 612.26 found for 612.33 [M+H]

Synthesis Example 6: Synthesis of Compound A-11

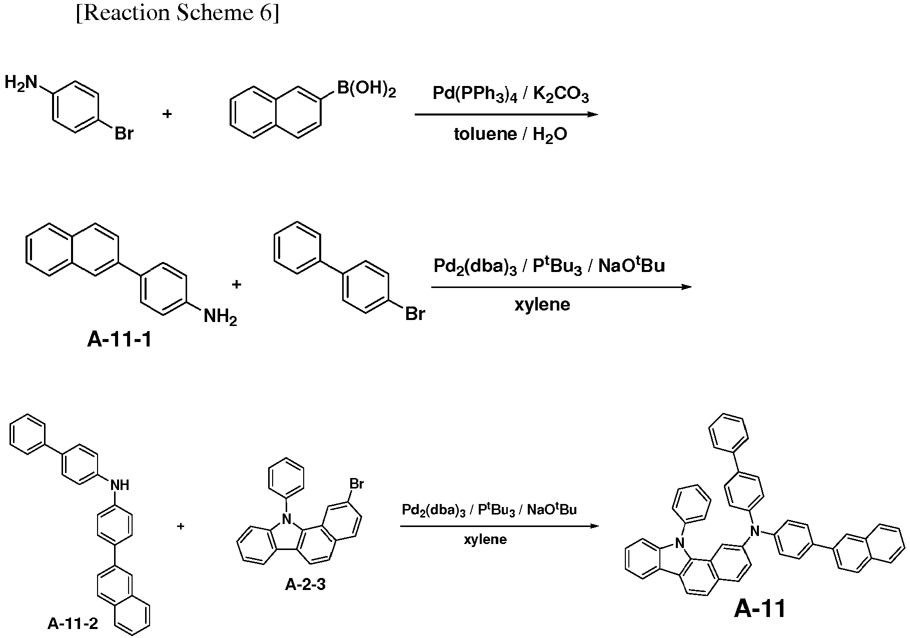

### a) Synthesis of Intermediate A-11-1

4-bromo-phenylamine (50.0 g, 290.7 mmol), 2-naphthalene boronic Acid (59.9 g, 171.9 mmol), K₂CO₃ (80.4 g, 581.3 mmol), and Pd(PPh₃)₄ (10.1 g, 8.7 mmol) were put in a round-bottomed flask and dissolved in 800 ml of toluene and 400 ml of distilled water, and the solution was stirred at 80 °C for 12 hours. When a reaction was complete, an aqueous layer was removed therefrom, and the rest thereof was treated through column chromatography to obtain 40.0 g of Intermediate A-11-1 (63 %).

### b) Synthesis of Intermediate A-11-2

Intermediate A-11-1 (17.7 g, 80.8 mmol), 4-bromo-biphenyl (18.8 g, 80.8 mmol), sodium t-butoxide (NaOtBu) (11.6 g, 121.1 mmol), Pd₂(dba)₃ (4.4 g, 4.8 mmol), and tri t-butylphosphine (P(tBu)₃) (5.9 g, 50 % in toluene) were added to xylene (400 mL) and then heated and refluxed together under a nitrogen flow for 12 hours. After removing the xylene, the rest thereof was treated through column chromatography to obtain 20.0 g of Intermediate A-11-2 (67 %).

c) Synthesis of Compound A-11

Compound A-11 was synthesized according to the same method as the d) of Synthesis Example 1 by using Intermediate A-11-2 and Intermediate A-2-3 in an equivalent ratio of 1:1.

### LC/MS calculated for: C50H34N2 Exact Mass: 662.27 found for 662.31 [M+H]

Synthesis Example 7: Synthesis of Compound A-12

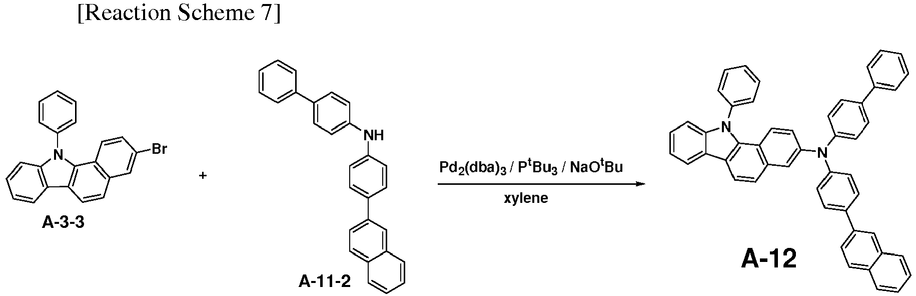

Compound A-12 was synthesized according to the same method as the d) of Synthesis Example 1 by using Intermediate A-3-3 and Intermediate A-11-2 in an equivalent ratio of 1:1.

LC/MS calculated for: C50H34N2 Exact Mass: 662.27 found for 662.30 [M+H]

Synthesis Example 8: Synthesis of Compound A-29

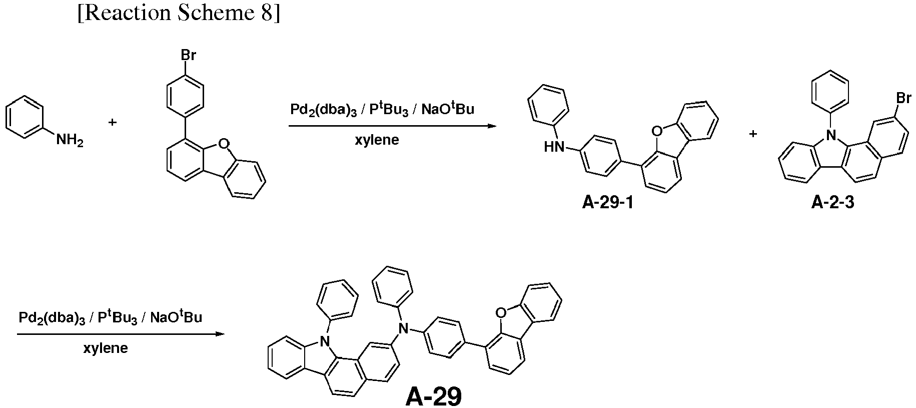

### a) Synthesis of Intermediate A-29-1

Aniline (8.3 g, 89.5 mmol), 4-(4-bromo-phenyl)-dibenzofuran (23.1 g, 71.5 mmol), sodium t-butoxide (NaOtBu) (12.9 g, 134.2 mmol), Pd₂(dba)₃ (4.9 g, 5.4 mmol), and tri t-butylphosphine (P(tBu)₃) (6.5 g, 50 % in toluene) were added to xylene (400 mL) and then heated and refluxes together under a nitrogen flow for 12 hours. After removing the xylene, the rest thereof was treated through column chromatography to obtain 20.0 g of Intermediate A-29-1 (67 %).

### b) Synthesis of Compound A-29

Compound A-29 was synthesized according to the same method as the d) of Synthesis Example 1 by using Intermediate A-29-1 and Intermediate A-2-3 in an equivalent ratio of 1:1.

LC/MS calculated for: C46H30N2O Exact Mass: 626.24 found for 626.28 [M+H]

Synthesis Example 9: Synthesis of Compound A-38

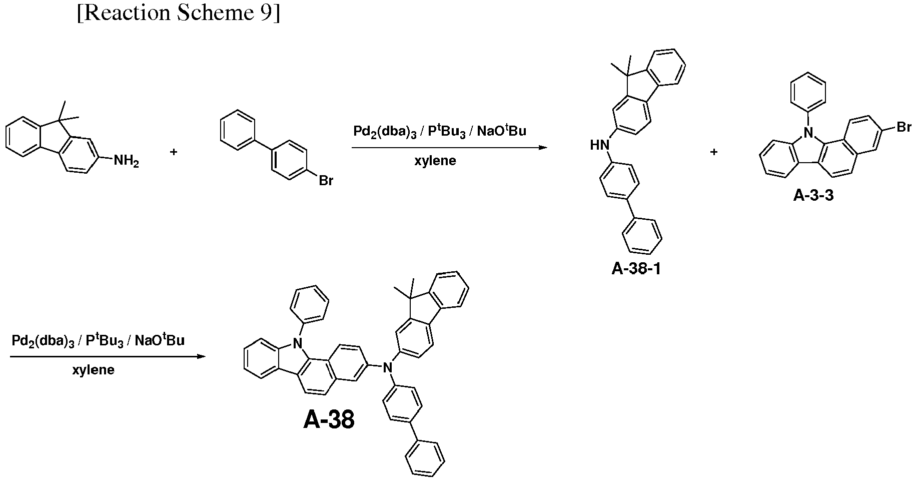

### a) Synthesis of Intermediate A-38-1

9,9-Dimethyl-9H-fluoren-2-ylamine (17.4 g, 83.0 mmol), 4-bromo-biphenyl (15.5 g, 66.4 mmol), sodium t-butoxide (NaOtBu) (12.0 g, 124.5 mmol), Pd₂(dba)₃ (4.6 g, 5.0 mmol), and tri t-butylphosphine (P(tBu)₃) (6.0 g, 50 % in toluene) were put in xylene (400 mL) and then heated and refluxed under a nitrogen flow for 12 hours. After removing the xylene, the rest thereof was treated through column chromatography to obtain 18.0 g of Intermediate A-38-1 (60 %).

### b) Synthesis of Compound A-38

Compound A-38 was synthesized according to the same method as the d) of Synthesis Example 1 by using Intermediate A-38-1 and Intermediate A-3-3 in an equivalent ratio of 1:1.

LC/MS calculated for: C49H36N2 Exact Mass: 652.29 found for 652.33 [M+H]

Synthesis Example 10: Synthesis of Compound A-51

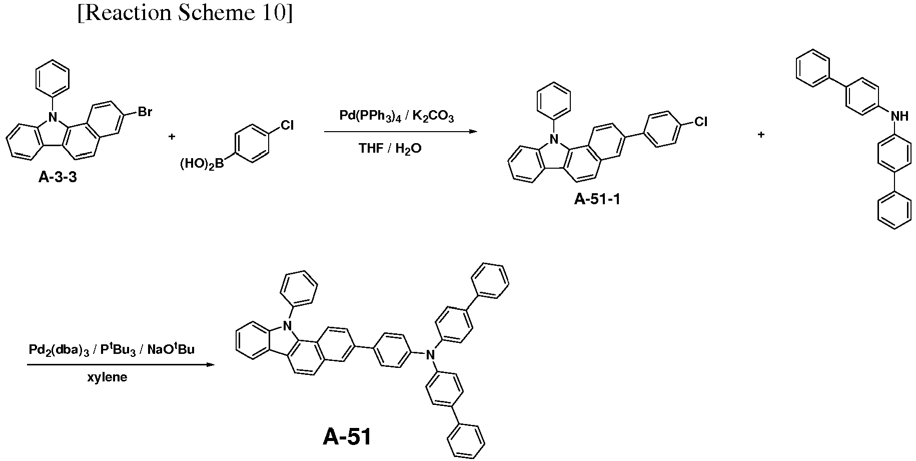

### a) Synthesis of Intermediate A-51-1

Intermediate A-3-3 (30.0 g, 80.6 mmol), 4-chlorophenyl boronic acid (15.1 g, 96.7 mmol), K₂CO₃ (22.3 g, 161.2 mmol), and Pd(PPh₃)₄ (2.8 g, 2.4 mmol) were put in a round-bottomed flask and then dissolved in 200 ml of tetrahydrofuran and 100 ml of distilled water, and the solution was stirred at 80 °C for 12 hours. When a reaction was complete, an aqueous layer was removed, and the rest thereof was treated through column chromatography to obtain 27.0 g of Intermediate A-51-1 (83 %).

### b) Synthesis of Compound A-51

Compound A-51 was synthesized according to the same method as the d) of Synthesis Example 1 by using Intermediate A-51-1 and bis-biphenyl-4-yl-amine in an equivalent ratio of 1:1.

LC/MS calculated for: C52H36N2 Exact Mass: 688.29 found for 688.34 [M+H]

Synthesis Example 11: Synthesis of Compound A-65

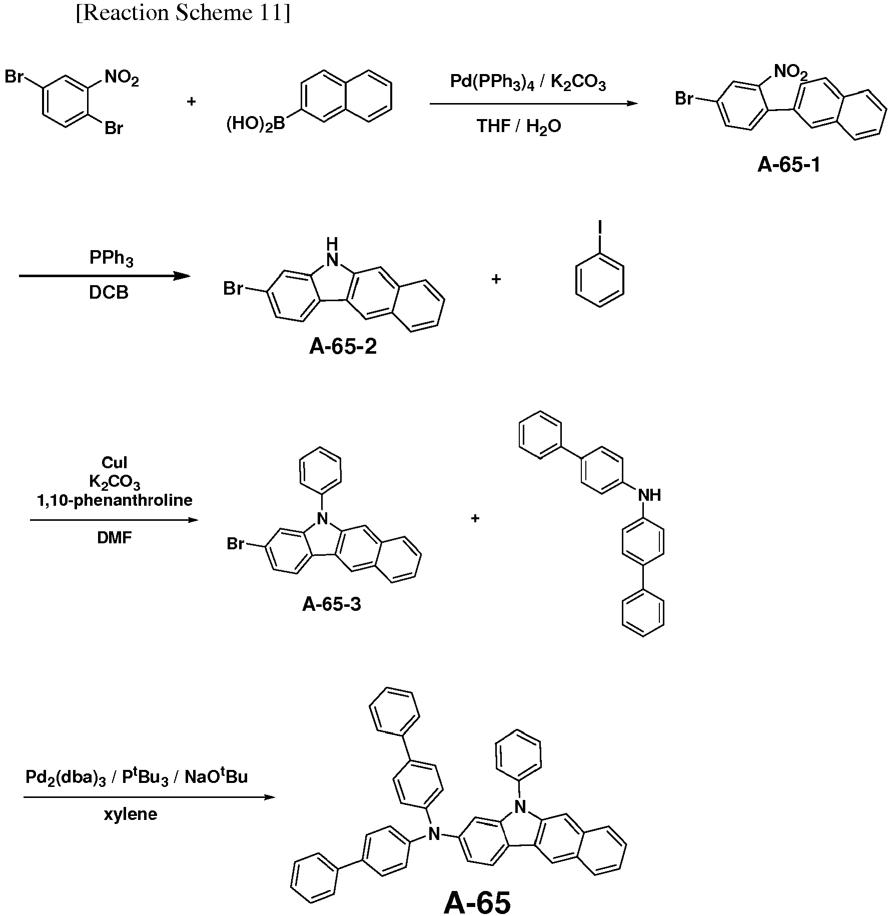

### a) Synthesis of Intermediate A-65-1

1,4-dibromo-2-nitro-benzene (30.0 g, 106.8 mmol), 2-naphthalene boronic acid (18.4 g, 106.8 mmol), K₂CO₃ (29.5 g, 213.6 mmol), and Pd(PPh₃)₄ (3.7 g, 3.2 mmol) were put in a round-bottomed flask and then dissolved in 300 mL of tetrahydrofuran and 150 mL of distilled water, and the solution was stirred at 80 °C for 12 hours. When a reaction was complete, an aqueous layer was removed, and the rest thereof was treated through column chromatography to obtain 27.0 g of Intermediate A-65-1 (77 %).

### b) Synthesis of Intermediate A-65-2

Intermediate A-65-1 (27.0 g, 82.3 mmol) and triphenylphosphine (86.3 g, 329.1 mmol) were put in a round-bottomed flask and then dissolved in 300 mL of 1,2-dichlorobenzene, and the solution was stirred at 180 °C for 12 hours. When a reaction was complete, a solvent was removed therefrom, and the rest thereof was treated through column chromatography to obtain 18.0 g of Intermediate A-65-2 (74 %).

### c) Synthesis of Intermediate A-65-3

Intermediate A-65-3 was synthesized according to the same method as the c) of Synthesis Example 1 by using Intermediate A-65-2 and iodobenzene in an equivalent ratio of 1:3.

### d) Synthesis of Compound A-65

Intermediate A-65 was synthesized according to the same method as the d) of Synthesis Example 1 by using Intermediate A-65-3 and bis-biphenyl-4-yl-amine in an equivalent ratio of 1:1.

LC/MS calculated for: C46H32N2 Exact Mass: 612.26 found for 612.30 [M+H]

Synthesis Example 12: Synthesis of Compound A-72

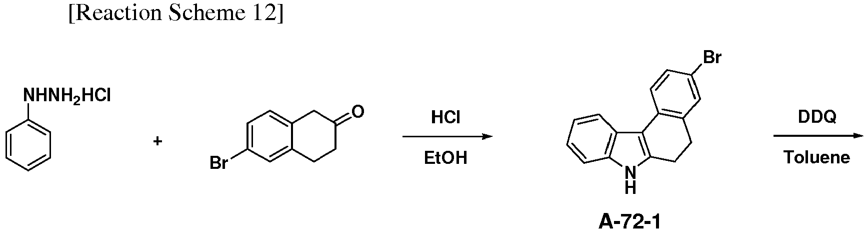

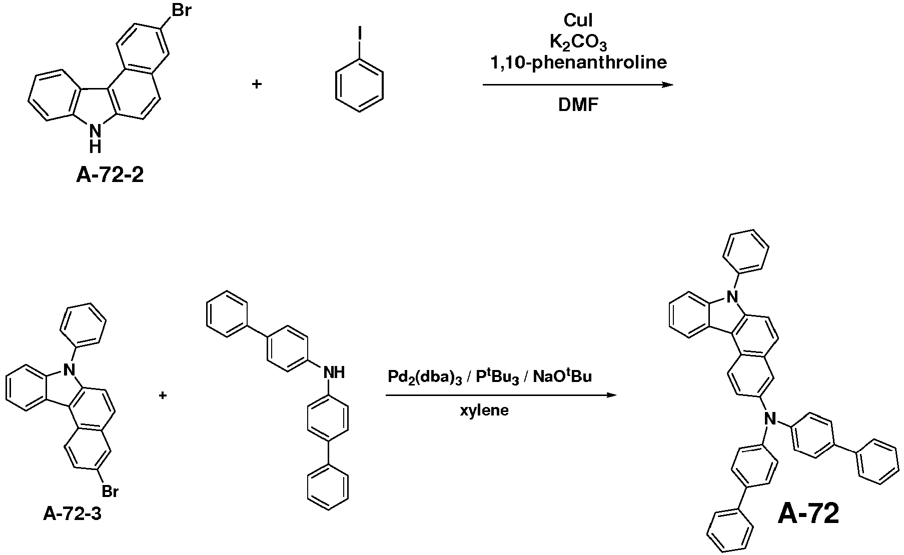

### a) Synthesis of Intermediate A-72-1

Intermediate A-72-1 was synthesized according to the same method as the a) of Synthesis Example 1 by using phenylhydrazinehydrochloride and 6-bromo-3,4-dihydro-1H-naphthalen-2-one by 1.0 equivalent.

### b) Synthesis of Intermediate A-72-2

Intermediate A-72-2 was synthesized according to the same method as the b) of Synthesis Example 1 by using Intermediate A-72-1 and 2,3-dichloro-5,6-dicyano-1,4-benzoquinone in an equivalent ratio of 1:1.5.

### c) Synthesis of Intermediate A-72-3

Intermediate A-72-3 was synthesized according to the same method as the c) of Synthesis Example 1 by using Intermediate A-72-2 and iodobenzene in an equivalent ratio of 1:3.

### d) Synthesis of Compound A-72

Intermediate A-72 was synthesized according to the same method as the d) of Synthesis Example 1 by using Intermediate A-72-3 and bis-biphenyl-4-yl-amine in an equivalent ratio of 1:1.

LC/MS calculated for: C46H32N2 Exact Mass: 612.26 found for 612.31 [M+H]

Synthesis Example 13: Synthesis of Compound A-77

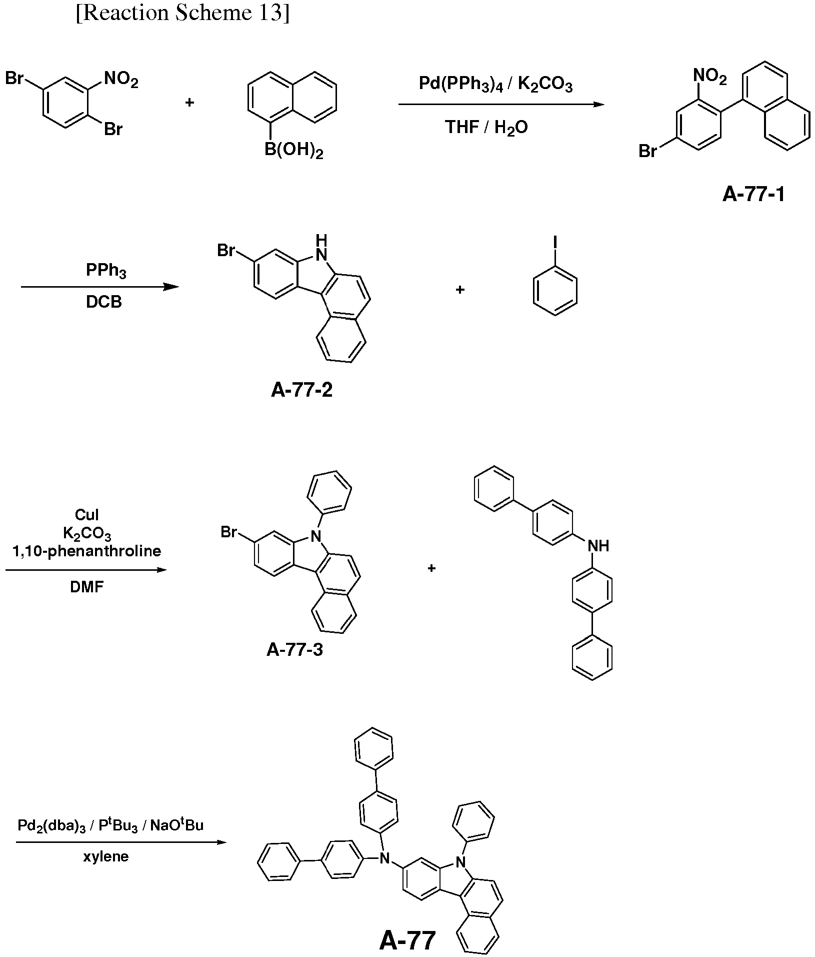

### a) Synthesis of Intermediate A-77-1

Intermediate A-77-1 was synthesized according to the same method as the a) of Synthesis Example 11 by using 1,4-dibromo-2-nitro-benzene and 1 -naphthalene boronic acid by 1.0 equivalent.

### b) Synthesis of Intermediate A-77-2

Intermediate A-77-2 was synthesized according to the same method as the b) of Synthesis Example 11 by using Intermediate A-77-1 and triphenylphosphine in an equivalent ratio of 1:4.

### c) Synthesis of Intermediate A-77-3

Intermediate A-77-3 was synthesized according to the same method as the c) of Synthesis Example 1 by using Intermediate A-77-2 and iodobenzene in an equivalent ratio of 1:3.

### d) Synthesis of Compound A-77

Compound A-77 was synthesized according to the same method as the d) of Synthesis Example 1 by using Intermediate A-77-3 and bis-biphenyl-4-yl-amine in an equivalent ratio of 1:1.

LC/MS calculated for: C46H32N2 Exact Mass: 612.26 found for 612.29 [M+H]

Comparative Synthesis Example 1: Synthesis of Comparative Compound V-1

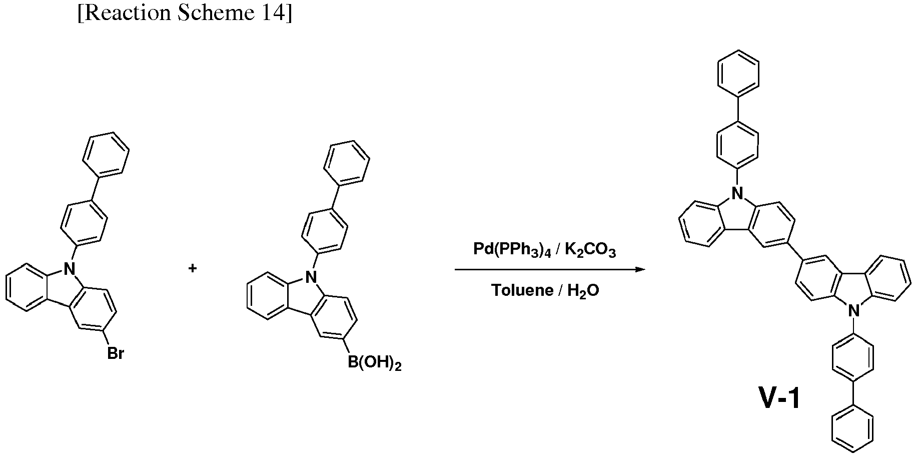

The compound, biphenylcarbazolyl bromide (12.33 g, 30.95 mmol) was dissolved in 200 mL of toluene in an nitrogen atmosphere, biphenylcarbazolylboronic acid (12.37 g, 34.05 mmol) and tetrakis(triphenylphosphine)palladium (1.07 g, 0.93 mmol) are added thereto, and the obtained mixture was stirred. Subsequently, potassium carbonate saturated in water (12.83 g, 92.86 mmol) was added thereto, and the obtained mixture was heated and refluxed at 90 °C for 12 hours. When a reaction was complete, water was added to the reaction solution, and an extract was obtained by using dichloromethane (DCM), filtered after removing moisture therefrom by using anhydrous MgSO₄, and concentrated under a reduced pressure. A residue obtained therefrom was separated and purified through flash column chromatography to obtain Compound V-1 (18.7 g, 92 %).

LC/MS calculated for: C48H32N2 Exact Mass: 636.26 found for 636.30 [M+H]

Comparative Synthesis Example 2: Synthesis of Comparative Compound V-2

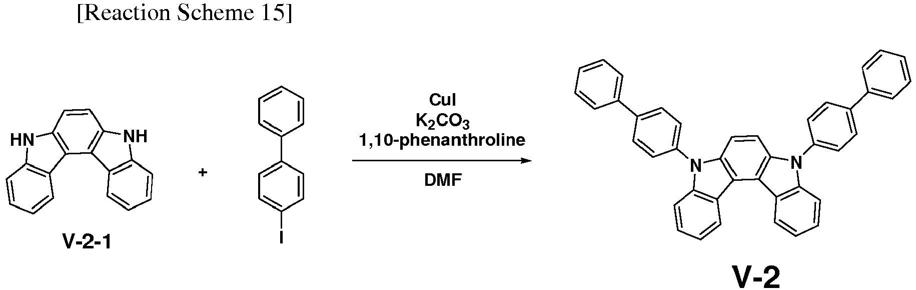

8 g (31.2 mmol) of Intermediate V-2-1 (5,8-dihydro-indolo[2,3-C]carbazole), 20.5 g (73.32 mmol) of 4-iodobiphenyl, 1.19 g (6.24 mmol) of CuI, 1.12 g (6.24 mmol) of 1,10-phenanthroline, and 12.9 g (93.6 mmol) of K₂CO₃ were put in a round-bottomed flask, 50 ml of DMF was added thereto to dissolve them, and the solution was refluxed and stirred under a nitrogen atmosphere for 24 hours. When a reaction was complete, distilled water was added thereto, and a precipitate therefrom was filtered. The solid was dissolved in 250 ml of xylene, filtered with silica gel, and precipitated into a white solid to obtain 16.2 g of Compound V-2 (a yield of 93 %).

LC/MS calculated for: C42H28N2 Exact Mass: 560.23 found for 560.27 [M+H]

### (Preparation of Second Compound)

Synthesis Example 14: Synthesis of Compound B-1

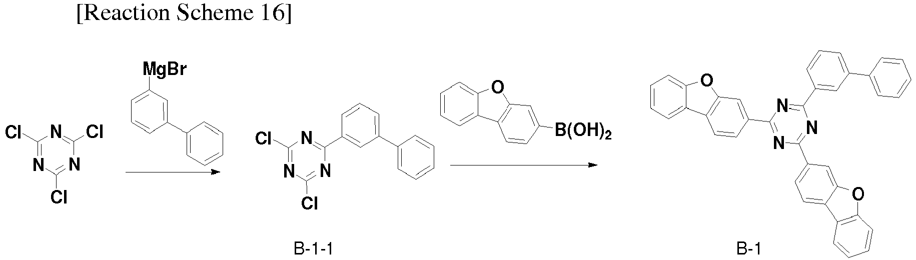

### a) Synthesis of Intermediate B-1-1

15 g (81.34 mmol) of cyanuric chloride was dissolved in 200 mL of anhydrous tetrahydrofuran in a 500 mL round-bottomed flask, 1 equivalent of a 3-biphenyl magnesium bromide solution (0.5 M tetrahydrofuran) was added thereto in a dropwise fashion at 0 °C under a nitrogen atmosphere, and the mixture was slowly heated up to room temperature. Subsequently, the reaction solution was stirred at the room temperature for 1 hour and then poured into 500 mL of ice water to separate layers. An organic layer was separated therefrom and then treated with anhydrous magnesium sulfate and concentrated. The concentrated residue was recrystallized with tetrahydrofuran and methanol to obtain 17.2 g of Intermediate B-1-1.

### b) Synthesis of Compound B-1

17.2 g (56.9 mmol) of Intermediate B-1-1 was added to 200 mL of tetrahydrofuran and 100 mL of distilled water in a 500 mL round-bottomed flask, 2 equivalent of dibenzofuran-3-boronic acid (CAS: 395087-89-5), 0.03 equivalent of tetrakistriphenylphosphine palladium, and 2 equivalent of potassium carbonate were added thereto, and the mixture was heated and refluxed under a nitrogen atmosphere. After 18 hours, the reaction solution was cooled down, and a solid precipitated therein was filtered and washed with 500 mL of water. The solid was recrystallized with 500 mL of monochlorobenzene to obtain 12.87 g of Compound B-1.

LC/MS calculated for: C39H23N3O2 Exact Mass: 565.1790 found for: 566.18 [M+H]

Synthesis Example 15: Synthesis of Compound B-3

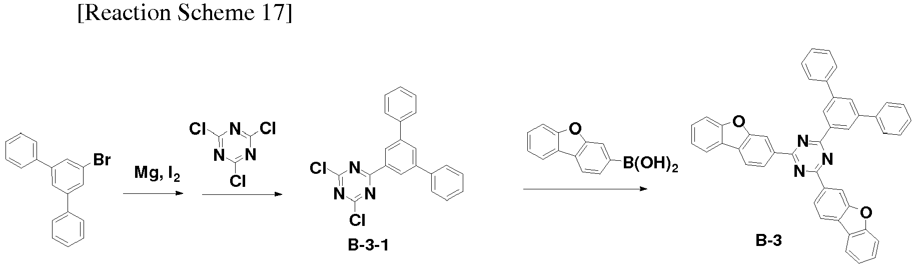

### a) Synthesis of Intermediate B-3-1

Magnesium (7.86 g, 323 mmol) and iodine (1.64 g, 6.46 mmol) were added to 0.1 L of tetrahydrofuran (THF) in a nitrogen atmosphere, the mixture was stirred for 30 minutes, 1-bromo-3,5-diphenylbenzene (100 g, 323 mmol) dissolved in 0.3 L of THF was slowly added thereto in a dropwise fashion at 0 °C over 30 minutes. This obtained mixed solution was slowly added in a dropwise fashion to 64.5 g (350 mmol) of cyanuric chloride dissolved in 0.5 L of THF at 0 °C over 30 minutes. When a reaction was complete, water was added to the reaction solution, and an extract was obtained by using dichloromethane (DCM), filtered after removing moisture therefrom with anhydrous MgSO₄, and concentrated under a reduced pressure. The obtained residue was separated and purified through flash column chromatography to obtain Intermediate B-3-1 (79.4 g, 65 %).

### b) Synthesis of Compound B-3

Compound B-3 was synthesized according to the same method as the b) of Synthesis Example 14 by using Intermediate B-3-1.

LC/MS calculated for: C45H27N3O2 Exact Mass: 641.2103 found for 642.21 [M+H]

Synthesis Example 16: Synthesis of Compound B-17

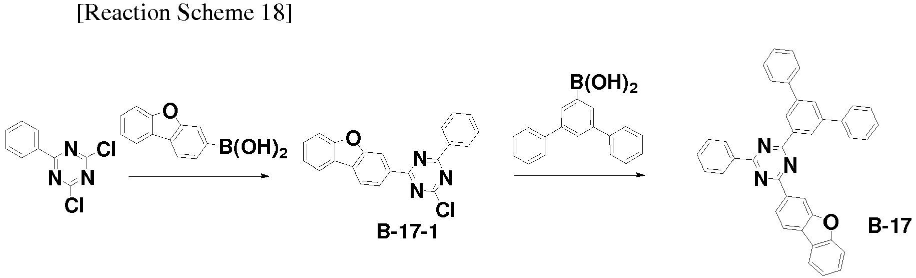

### a) Synthesis of Intermediate B-17-1

22.6 g (100 mmol) of 2,4-dichloro-6-phenyltriazine was added to 100 mL of tetrahydrofuran, 100 mL of toluene, and 100 mL of distilled water in a 500 mL round-bottomed flask, 0.9 equivalent of dibenzofuran-3-boronic acid (CAS No.: 395087-89-5), 0.03 equivalent of tetrakistriphenylphosphine palladium, and 2 equivalent of potassium carbonate were added thereto, and the mixture was heated and refluxed under a nitrogen atmosphere. After 6 hours, the reaction solution was cooled down, an aqueous layer was removed therefrom, and an organic layer therein was dried under a reduced pressure. A solid obtained therefrom was washed with water and hexane and recrystallized with 200 mL of toluene to obtain 21.4 g of Intermediate B-17-1 (60 % of a yield).

### b) Synthesis of Compound B-17

Intermediate B-17-1 (56.9 mmol) was added to 200 mL of tetrahydrofuran and 100 mL of distilled water in a 500 mL round-bottomed flask, 1.1 equivalent of 3,5-diphenylbenzeneboronic acid (CAS No.: 128388-54-5), 0.03 equivalent of tetrakistriphenylphosphine palladium, and 2 equivalent of potassium carbonate were added thereto, and the mixture was heated and refluxed under a nitrogen atmosphere. After 18 hours, the reaction solution was cooled down, and a solid precipitated therein was filtered and washed with 500 mL of water. The solid was recrystallized with 500 mL of monochlorobenzene to obtain Compound B-17.

LC/MS calculated for: C39H25N3O Exact Mass: 555.1998 found for 556.21 [M+H]

Synthesis Example 17: Synthesis of Compound B-20

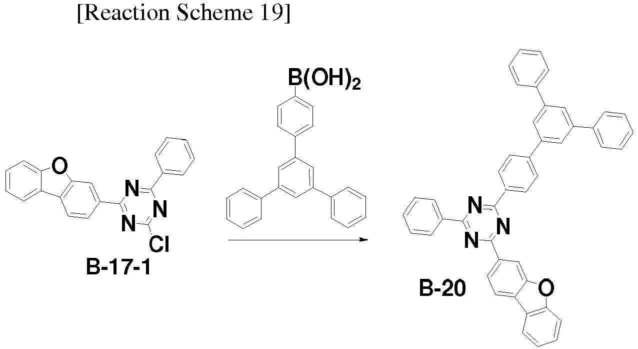

Compound B-20 was synthesized according to the same method as the b) of Synthesis Example 16 by using Intermediate B-17-1 and 1.1 equivalent of (5'-phenyl[1,1':3',1"-terphenyl]-4-yl)-boronic acid (CAS No.: 491612-72-7).

LC/MS calculated for: C45H29N3O Exact Mass: 627.2311 found for 628.24 [M+H]

Synthesis Example 18: Synthesis of Compound B-23

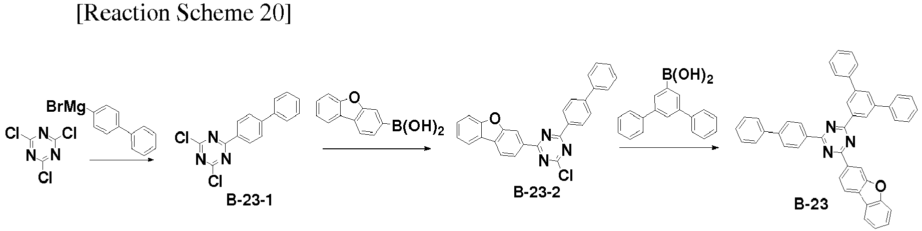

### a) Synthesis of Intermediate B-23-1

15 g (81.34 mmol) of cyanuric chloride is dissolved in 200 mL of anhydrous tetrahydrofuran in a 500 mL round-bottomed flask, 1 equivalent of a 4-biphenyl magnesium bromide solution (0.5 M tetrahydrofuran) was added thereto in a dropwise fashion at 0 °C under a nitrogen atmosphere, and the mixture was slowly heated up to room temperature. The reaction solution was stirred at the room temperature for 1 hour and then poured into 500 mL of ice water to separate layers. An organic layer was separated, treated with anhydrous magnesium sulfate, and concentrated. The concentrated residue was recrystallized with tetrahydrofuran and methanol to obtain 17.2 g of Intermediate B-23-1.

### b) Synthesis of Intermediate B-23-2

Intermediate B-23-2 was synthesized according to the same method as the a) of Synthesis Example 16 by using Intermediate B-23-1.

### c) Synthesis of Compound B-23

Compound B-23 was synthesized according to the same method as the b) of Synthesis Example 16 by using Intermediate B-23-2 and 1.1 equivalent of 3,5-diphenylbenzeneboronic acid.

LC/MS calculated for: C45H29N3O Exact Mass: 627.2311 found for 628.24 [M+H]

Synthesis Example 19: Synthesis of Compound B-24

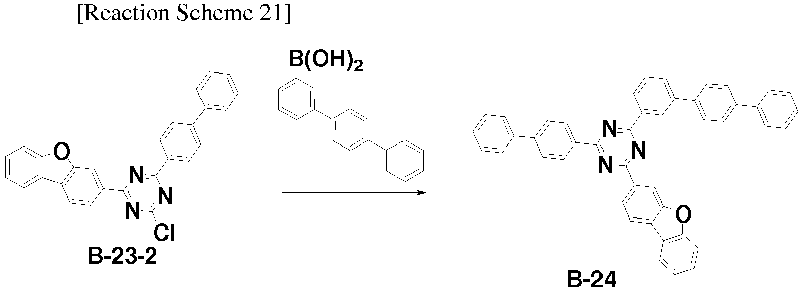

Compound B-24 was synthesized according to the same method as the b) of Synthesis Example 16 by using Intermediate B-23-2 and 1.1 equivalent of B-[1,1':4',1"-terphenyl]-3-yl boronic acid.

LC/MS calculated for: C45H29N3O Exact Mass: 627.2311 found for 628.24 [M+H]

Synthesis Example 20: Synthesis of Compound B-71

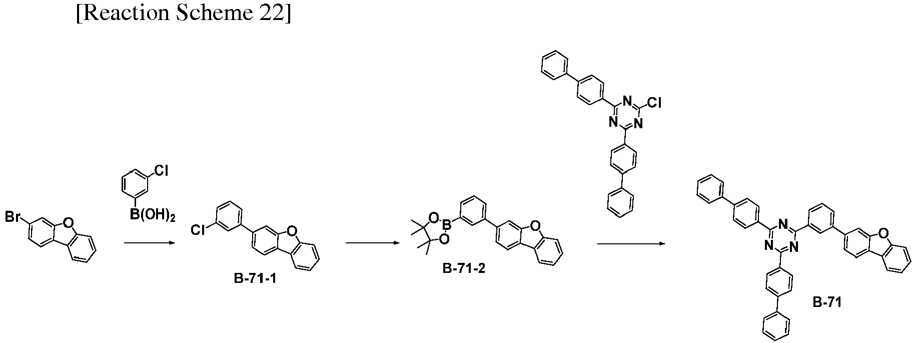

### a) Synthesis of Intermediate B-71-1

14.06 g (56.90 mmol) of 3-bromo-dibenzofuran, 200 mL of tetrahydrofuran, and 100 mL of distilled water were put in a 500 mL round-bottomed flask, 1 equivalent of 3'-chloro-phenylboronic acid, 0.03 equivalent of tetrakistriphenylphosphine palladium, and 2 equivalent of potassium carbonate were added thereto, and the mixture was heated and refluxed under a nitrogen atmosphere. After 18 hours, the reaction solution was cooled down, and a solid precipitated therein was filtered and washed with 500 mL of water. The solid was recrystallized with 500 mL of monochlorobenzene to obtain 12.05 g of Intermediate B-71-1. (A yield of 76 %)

### b) Synthesis of Intermediate B-71-2

24.53 g (88.02 mmol) of Intermediate B-71-1 was added to 250 mL of DMF in a 500 mL round-bottomed flask, 0.05 equivalent of dichlorodiphenylphosphinoferrocene palladium, 1.2 equivalent of bispinacolato diboron, and 2 equivalent of potassium acetate were added thereto, and the mixture was heated and refluxed under a nitrogen atmosphere for 18 hours. The reaction solution was cooled down and then added to 1 L of water in a dropwise fashion to obtain a solid. The solid was dissolved in boiling toluene, treated with activated carbon, and filtered with silica gel, and the filtrate was concentrated. The concentrated solid was stirred with a small amount of hexane and filtered to obtain 22.81 g of Intermediate B-71-2. (A yield of 70 %)

### c) Synthesis of Compound B-71

Compound B-71 was synthesized according to the same method as the b) of Synthesis Example 16 by using Intermediate B-71-2 and 2,4-bis([1,1'-biphenyl]-4-yl)-6-chloro-1,3,5-triazine respectively by 1.0 equivalent.

LC/MS calculated for: C45H29N3O Exact Mass: 627.2311 found for 628.25 [M+H]

Synthesis Example 21: Synthesis of Compound B-124

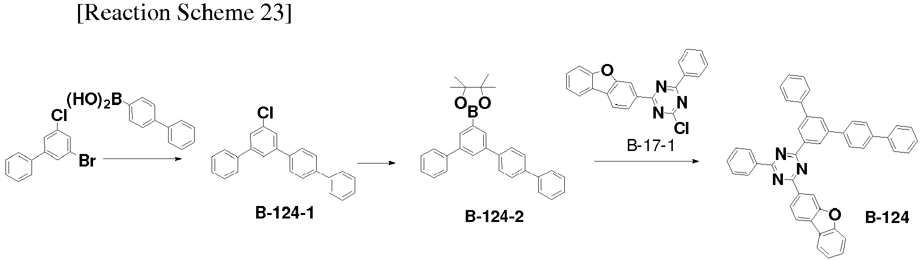

### a) Synthesis of Intermediate B-124-1

Intermediate B-124-1 was synthesized according to the same method as a) of Synthesis Example 16 by using 1-bromo-3-chloro-5-phenylbenzene and biphenyl-4-boronic acid respectively by 1.1 equivalent. Herein, a product therefrom was not recrystallized but purified through flash column by using hexane.

### b) Synthesis of Intermediate B-124-2

30 g (88.02 mmol) of Intermediate B-124-1 was added to 250 mL of DMF in a 500 mL round-bottomed flask, 0.05 equivalent of dichlorodiphenylphosphinoferrocene palladium, 1.2 equivalent of bispinacolato diboron, and 2 equivalent of potassium acetate were added thereto, and the mixture was heated and refluxed under a nitrogen atmosphere for 18 hours. The reaction solution was cooled down and then added to 1 L of water in a dropwise fashion to obtain a solid. The solid was dissolved in boiling toluene, treated with activated carbon, and filtered with silica gel, and the filtrate was concentrated. The concentrated solid was stirred with a small amount of hexane and filtered to obtain 28.5 g of Intermediate B-124-2 (70 % of a yield).

### c) Synthesis of Compound B-124

Compound B-124 was synthesized according to the same method as b) of Synthesis Example 16 by using Intermediate B-124-2 and Intermediate B-17-1 respectively by 1.0 equivalent.

LC/MS calculated for: C45H29N3O Exact Mass: 627.2311 found for 628.22 [M+H]

Synthesis Example 22: Synthesis of Compound B-129

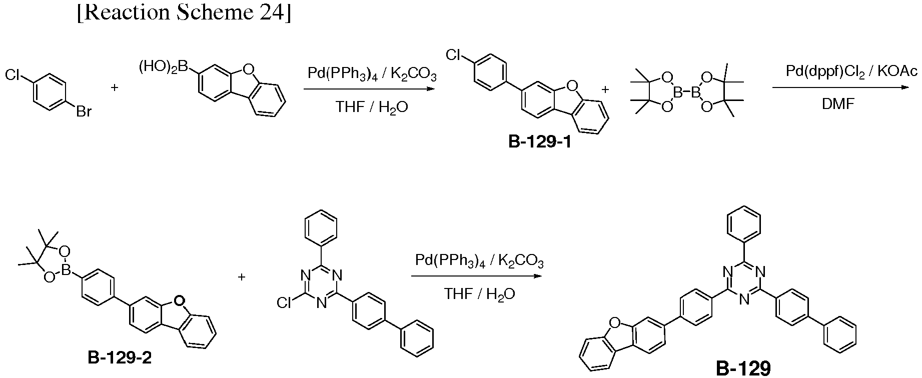

### a) Synthesis of Intermediate B-129-1

Intermediate B-129-1 was synthesized according to the same method as the a) of Synthesis Example 20 by using 1-bromo-4-chloro-benzene and 3-dibenzofuranylboronic acid respectively by 1.0 equivalent.

### b) Synthesis of Intermediate B-129-2

Intermediate B-129-2 was synthesized according to the same method as the b) of Synthesis Example 20 by using Intermediate B-129-1 and bispinacolato diboron in an equivalent ratio of 1:1.2.

### c) Synthesis of Compound B-129

Compound B-129 was synthesized according to the same method as the b) of Synthesis Example 16 by using Intermediate B-129-2 and 2-chloro-4-(biphenyl-4-yl)6-phenyl-1,3,5-triazine respectively by 1.0 equivalent.

LC/MS calculated for: C39H25N3O Exact Mass: 551.20 found for 551.24 [M+H]

Synthesis Example 23: Synthesis of Compound B-131

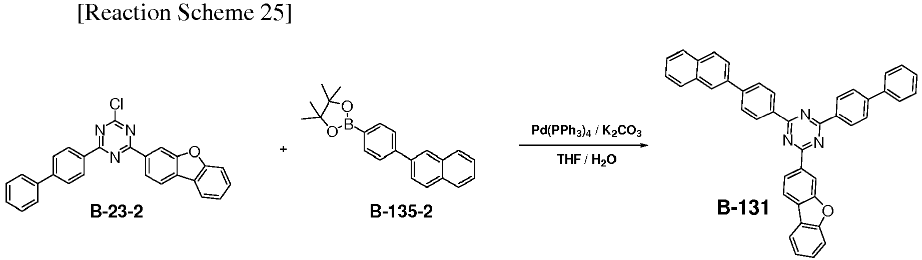

Compound B-131 was synthesized according to the same method as the b) of Synthesis Example 16 by using Intermediate B-23-2 and Intermediate B-135-2 respectively by 1.0 equivalent.

LC/MS calculated for: C43H27N3O Exact Mass: 601.22 found for 601.26 [M+H]

Synthesis Example 24: Synthesis of Compound B-133

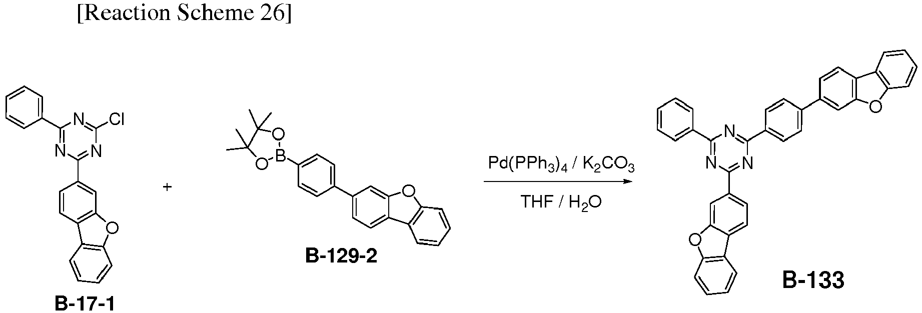

Compound B-133 was synthesized according to the same method as the b) of Synthesis Example 16 by using Intermediate B-17-1 and Intermediate B-129-2 respectively by 1.0 equivalent.

LC/MS calculated for: C39H23N3O2 Exact Mass: 565.18 found for 565.22 [M+H]

Synthesis Example 25: Synthesis of Compound B-135

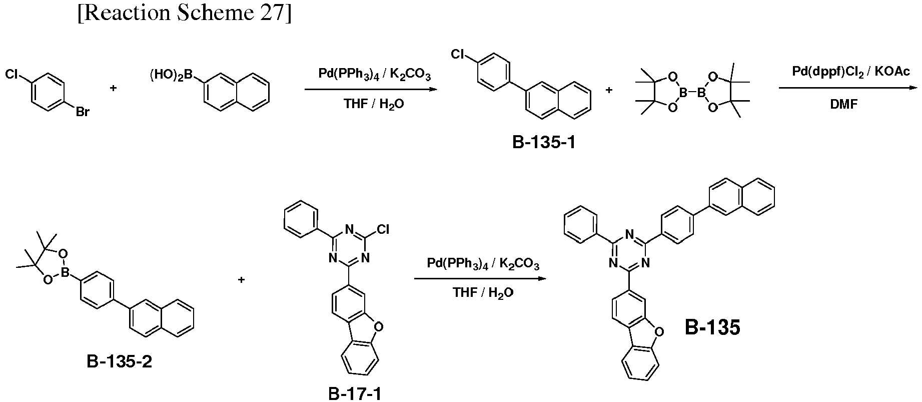

### a) Synthesis of Intermediate B-135-1

Intermediate B-135-1 was synthesized according to the same method as the a) of Synthesis Example 20 by using 1 -bromo-4-chloro-benzene and 2-naphthalene boronic acid respectively by 1.0 equivalent.

### b) Synthesis of Intermediate B-135-2

Intermediate B-135-2 was synthesized according to the same method as the b) of Synthesis Example 20 by using Intermediate B-135-1 and bispinacolato diboron in an equivalent ratio of 1:1.2.

### c) Synthesis of Compound B-135

Compound B-135 was synthesized according to the same method as the b) of Synthesis Example 16 by using Intermediate B-135-2 and Intermediate B-17-1 respectively by 1.0 equivalent.

LC/MS calculated for: C37H23N3O Exact Mass: 525.18 found for 525.22 [M+H]

Synthesis Example 26: Synthesis of Compound D-25

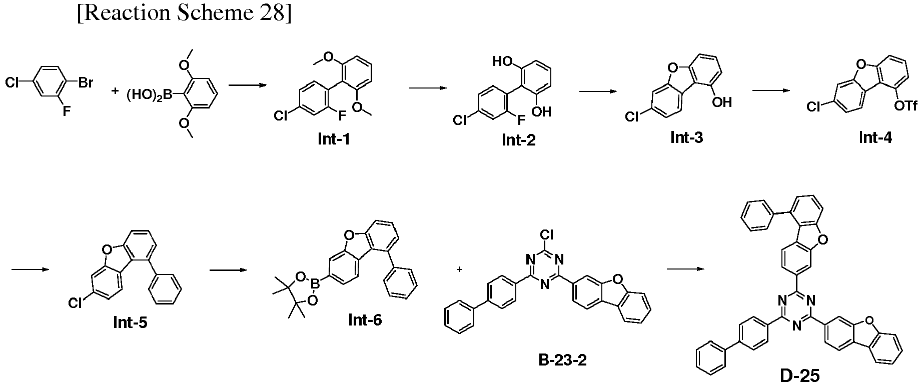

### a) Synthesis of Intermediate 1

1-bromo-4-chloro-2-fluorobenzene (61 g, 291 mmol), 2,6-dimethoxyphenylboronic acid (50.4 g, 277 mmol), K₂CO₃ (60.4 g, 437 mmol) and Pd(PPh₃)₄ (10.1 g, 8.7 mmol) were put in a round-bottomed flask and then dissolved in 500 ml of THF and 200 ml of distilled water, and the solution was refluxed and stirred at 60 °C for 12 hours. When a reaction was complete, an aqueous layer was removed, and the rest thereof was treated through column chromatography (hexane: DCM 20 %) to obtain 38 g of Intermediate 1 (51 %).

### b) Synthesis of Intermediate 2

Intermediate 1 (38 g, 142 mmol) and pyridine hydrochloride (165 g, 1425 mmol) were put in a round-bottomed flask and then refluxed and stirred at 200 °C for 24 hours. When a reaction was complete, the resultant is cooled down to room temperature and then slowly poured into distilled water, and the mixture was stirred for 1 hour. A solid therein was filtered to obtain 23 g of Intermediate 2 (68 %).

### c) Synthesis of Intermediate 3

Intermediate 2 (23 g, 96 mmol) and K₂CO₃ (20 g, 144 mmol) were put in a round-bottomed flask and dissolved in 100 ml of NMP, and the solution was refluxed and stirred at 180 °C for 12 hours. When a reaction was complete, the mixture was poured into an excess amount of distilled water. A solid therein was filtered, dissolved in ethylacetate, and then dried with MgSO₄, and an organic layer was removed therefrom under a reduced pressure. Subsequently, column chromatography (hexane: EA 30 %) was used to obtain 16 g of Intermediate 3 (76 %).

### d) Synthesis of Intermediate 4

Intermediate 3 (16 g, 73 mmol) and pyridine (12 ml, 146 mmol) were put in a round-bottomed flask and dissolved in 200 ml of DCM. The solution was cooled down to 0 °C, and trifluoromethanesulfonic anhydride (14.7 ml, 88 mmol) was slowly added thereto in a dropwise fashion. The mixture was stirred for 6 hour, and when a reaction was complete, an excess amount of distilled water was added thereto, and the obtained mixture was stirred for 30 minutes and extracted with DCM. Subsequently, an organic solvent was removed under a reduced pressure, and the rest thereof was vacuum-dried to obtain 22.5 g of Intermediate 4 (88 %).

### e) Synthesis of Intermediate 5

14.4 g of Intermediate 5 (81 %) was synthesized according to the same method as Synthesis Example 1 by using Intermediate 4 (22.5 g, 64 mmol), phenylboronic acid (7.8 g, 64 mmol), K₂CO₃ (13.3 g, 96 mmol), and Pd(PPh₃)₄ (3.7 g, 3.2 mmol).

### f) Synthesis of Intermediate 6

Intermediate 5 (22.5 g, 80 mmol), bis(pinacolato)diboron (24.6 g, 97 mmol), Pd(dppf)Cl₂ (2 g, 2.4 mmol), tricyclohexylphosphine (3.9 g, 16 mmol), and potassium acetate (16 g, 161 mmol) were put in a round-bottomed flask and dissolved in 320 ml of DMF. The mixture was refluxed and stirred at 120 °C for 10 hours. When a reaction was complete, the mixture was poured into an excess amount of distilled water, and the obtained mixture was stirred for one hour. A solid therein was filtered and dissolved in DCM. MgSO₄ was used to remove moisture therefrom, and an organic solvent was filtered by using a silica gel pad and removed under a reduced pressure. A solid was recrystallized with EA and hexane to obtain 26.9 g of Intermediate 6 (90 %).

### g) Synthesis of Compound D-25

15.5 g of Compound D-25 (70 %) was synthesized according to the same method as the b) of Synthesis Example 16 by using Intermediate B-23-2 (15 g, 35 mmol), Intermediate 6 (12.8 g, 35 mmol), K₂CO₃ (7.2 g, 52 mmol), and Pd(PPh₃)₄ (2 g, 1.7 mmol) under a nitrogen condition in a round-bottomed flask.

LC/MS calculated for: C45H27N3O2 Exact Mass: 641.21 found for 641.25 [M+H]

Synthesis Example 27: Synthesis of Compound D-3

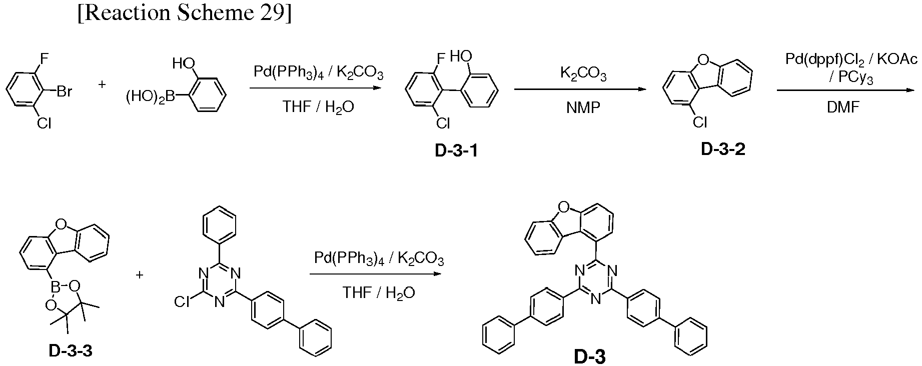

### a) Synthesis of Intermediate D-3-1

Intermediate D-3-1 was synthesized according to the same method as the a) of Synthesis Example 26 by using 2-bromo-1-chloro-3-fluoro-benzene and 2-hydroxyphenylboronic acid respectively by 1.0 equivalent.

### b) Synthesis of Intermediate D-3-2

Intermediate D-3-2 was synthesized according to the same method as the c) of Synthesis Example 26 by using Intermediate D-3-1 and K₂CO₃ in an equivalent ratio of 1:1.5.

### c) Synthesis of Intermediate D-3-3

Intermediate D-3-3 was synthesized according to the same method as the f) of Synthesis Example 26 by using Intermediate D-3-2 and bis(pinacolato)diboron in an equivalent ratio of 1:1.2.

### d) Synthesis of Compound D-3

Compound D-3 was synthesized according to the same method as the b) of Synthesis Example 16 by using Intermediate D-3-3 and 2,4-bis([1,1'-biphenyl]-4-yl)-6-chloro-1,3,5-triazine respectively by 1.0 equivalent.

LC/MS calculated for: C39H25N3O Exact Mass: 551.20 found for 551.24 [M+H]

### (Manufacture of Organic Light Emitting Diode)

### Example 1

A glass substrate coated with ITO (indium tin oxide) as a 1500 Å-thick thin film was washed with distilled water. After washing with the distilled water, the glass substrate was ultrasonic wave-washed with a solvent such as isopropyl alcohol, acetone, methanol, and the like and dried and then moved to a plasma cleaner, cleaned by using oxygen plasma for 10 minutes, and moved to a vacuum depositor. This obtained ITO transparent electrode was used as an anode, Compound A was vacuum-deposited on the ITO substrate to form a 700 Å-thick hole injection layer, Compound B was deposited to be 50 Å thick on the injection layer, and Compound C was deposited to be 1020 Å thick to form a hole transport layer. On the hole transport layer, a 400 Å-thick hole transport auxiliary layer was formed by depositing Compound C-1. On the hole transport auxiliary layer, a 400 Å-thick light emitting layer was formed by vacuum-depositing Compounds A-2 and B-3 as a host simultaneously and 2 wt% of [Ir(piq)₂acac] as a dopant. Herein Compound A-2 and Compound B-3 were used in a weight ratio of 5:5, and their ratio in the following Examples was separately provided. Subsequently, on the light emitting layer, a 300 Å-thick electron transport layer was formed by simultaneously vacuum-depositing the compound D and Liq in a ratio of 1:1, and on the electron transport layer, Liq and A1 were sequentially vacuum-deposited to be 15 Å thick and 1200 Å thick, manufacturing an organic light emitting diode.

The organic light emitting diode had a five-layered organic thin layer, and specifically the following structure.

ITO/Compound A (700 Å)/Compound B (50 Å)/Compound C (700 Å)/Compound C-1 (400 Å)ÆML[Compound A-2 : B-3: [Ir(piq)₂acac] (2 wt%)] (400 Å)/ Compound D : Liq (300 Å)/Liq (15 Å)/Al (1200 Å).

Compound A: N4,N4'-diphenyl-N4,N4'-bis(9-phenyl-9H-carbazol-3-yl)biphenyl-4,4'-diamine

Compound B: 1,4,5,8,9,11-hexaazatriphenylene-hexacarbonitrile (HAT-CN)

Compound C: N-(biphenyl-4-yl)-9,9-dimethyl-N-(4-(9-phenyl-9H-carbazol-3-yl)phenyl)-9H- fluoren-2-amine

Compound C-1: N,N-di([1,1'-biphenyl]-4-yl)-7,7-dimethyl-7H-fluoreno[4,3-b]benzofuran-10-amine

Compound D: 8-(4-(4,6-di(naphthalen-2-yl)-1,3,5-triazin-2-yl)phenyl)quinoline

### Examples 2 to 14

Each organic light emitting diode was manufactured according to the same method as Example 1 except for changing compositions as shown in Table 1.

### Comparative Examples 1 and 2

Each organic light emitting diode was manufactured according to the same method as Example 1 except for changing compositions as shown in Table 1.

### Evaluation

Power efficiency of the organic light emitting diodes according to Examples 1 to 14 and Comparative Examples 1 and 2 were evaluated.

### Specific measurement methods are as follows, and the results are shown in Table 1.

### (1) Measurement of Current Density Change Depending on Voltage Change

The obtained organic light emitting diodes were measured regarding a current value flowing in the unit device, while increasing the voltage from 0 V to 10 V using a current-voltage meter (Keithley 2400), and the measured current value was divided by area to provide the results.

### (2) Measurement of Luminance Change Depending on Voltage Change

Luminance was measured by using a luminance meter (Minolta Cs-1000A), while the voltages of the organic light emitting diodes were increased from 0 V to 10 V.

### (3) Measurement of Power Efficiency

Power efficiency (cd/A) at the same current density (10 mA/cm²) were calculated by using the luminance, current density, and voltages (V) from the items (1) and (2).

### (4) Measurement of Life-span

The results were obtained by measuring a time when current efficiency (cd/A) was decreased down to 97 %, while luminance (cd/m²) was maintained to be 9000 cd/m².

### (5) Measurement of Driving Voltage

A driving voltage of each diode was measured using a current-voltage meter (Keithley 2400) at 15 mA/cm².

**[Table 1]**

| | First host | Second host | First host: Second host Ratio (wt:wt) | Color | Power efficiency (cd/A) | Driving voltage (V) | Life-span T97 (h) |
|---|---|---|---|---|---|---|---|
| Example 1 | A-2 | B-3 | 5:5 | red | 21.3 | 3.71 | 98 |
| Example 2 | A-2 | B-20 | 5:5 | red | 21.0 | 3.53 | 80 |
| Example 3 | A-2 | B-23 | 5:5 | red | 20.5 | 3.75 | 107 |
| Example 4 | A-2 | B-124 | 5:5 | red | 20.8 | 3.78 | 112 |
| Example 5 | A-2 | B-129 | 5:5 | red | 22.0 | 3.63 | 117 |
| Example 6 | A-2 | B-129 | 6:4 | red | 21.5 | 3.65 | 96 |
| Example 7 | A-2 | B-133 | 5:5 | red | 22.0 | 3.68 | 121 |
| Example 8 | A-2 | B-135 | 5:5 | red | 21.2 | 3.70 | 120 |
| Example 9 | A-2 | B-135 | 6:4 | red | 20.6 | 3.72 | 122 |
| Example 10 | A-2 | D-25 | 5:5 | red | 22.2 | 3.55 | 124 |
| Example 11 | A-2 | D-3 | 5:5 | red | 20.0 | 3.78 | 100 |
| Example 12 | A-2 | D-3 | 6:4 | red | 19.7 | 3.80 | 80 |
| Example 13 | A-11 | B-135 | 5:5 | red | 21.2 | 3.68 | 127 |
| Example 14 | A-29 | B-135 | 5:5 | red | 21.5 | 3.75 | 115 |
| Comparativ e Example 1 | V-1 | B-20 | 5:5 | red | 15.6 | 4.77 | 4 |
| Comparativ e Example 2 | V-2 | B-20 | 5:5 | red | 19.0 | 4.1 | 34 |

Referring to Table 1, organic light emitting diodes according to Examples 1 to 14 exhibited remarkably improved driving voltage, efficiency, and life-span compared with those of Comparative Examples 1 and 2.

By way of summation and review, an organic light emitting diode (OLED) has recently drawn attention due to an increase in demand for flat panel displays. The organic light emitting diode converts electrical energy into light by applying current to an organic light emitting material. Performance of an organic light emitting diode may be affected by organic materials disposed between electrodes.

As described above, embodiments may provide a composition for an organic optoelectronic device capable of realizing an organic optoelectronic device having high efficiency and a long life-span.

### <Description of Symbols>

- 100, 200:: organic light emitting diode
- 105:: organic layer
- 110:: cathode
- 120:: anode
- 130:: light emitting layer
- 140:: hole auxiliary layer

Example embodiments have been disclosed herein, and although specific terms are employed, they are used and are to be interpreted in a generic and descriptive sense only and not for purpose of limitation. In some instances, as would be apparent to one of ordinary skill in the art as of the filing of the present application, features, characteristics, and/or elements described in connection with a particular embodiment may be used singly or in combination with features, characteristics, and/or elements described in connection with other embodiments unless otherwise specifically indicated. Accordingly, it will be understood by those of skill in the art that various changes in form and details may be made without departing from the spirit and scope of the present invention as set forth in the following claims.

## Claims

1. A composition, comprising:
a first compound, the first compound being represented by a combination of Chemical Formula 1 and Chemical Formula 2 bonded together; and
a second compound, the second compound being represented by Chemical Formula 3, 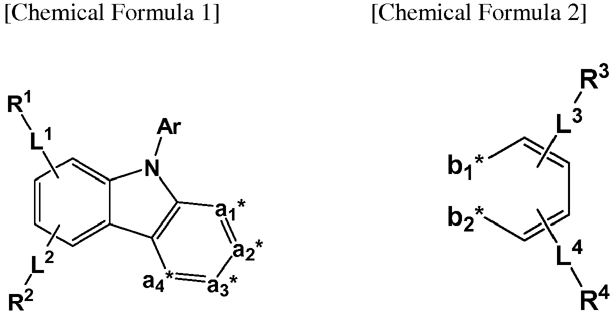
wherein, in Chemical Formula 1 and Chemical Formula 2,
Ar is a substituted or unsubstituted C6 to C30 aryl group, a substituted or unsubstituted C2 to C30 heterocyclic group, or a combination thereof,
adjacent two of a₁^{∗} to a₄^{∗} are bonded with b₁^{∗} and b₂^{∗}, respectively, and remaining two of a₁^{∗} to a₄^{∗} not bonding with b₁^{∗} and b₂^{∗} are each independently C-L^{a}-R^{a},
L^{a} and L¹ to L⁴ are each independently a single bond, a substituted or unsubstituted C6 to C20 arylene group, a substituted or unsubstituted divalent C2 to C20 heterocyclic group, or a combination thereof,
R^{a} and R¹ to R⁴ are each independently hydrogen, deuterium, a cyano group, a substituted or
unsubstituted amine group, a substituted or unsubstituted C1 to C30 alkyl group, a substituted or unsubstituted C6 to C30 aryl group, a substituted or unsubstituted C2 to C30 heterocyclic group, or a combination thereof, provided that at least one of R^{a} and R¹ to R⁴ is a group represented by Chemical Formula A: 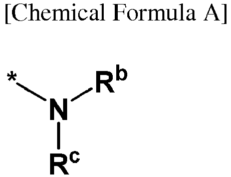
wherein, in Chemical Formula A,
R^{b} and R^{c} are each independently a substituted or unsubstituted C6 to C30 aryl group, a substituted or unsubstituted C2 to C30 heterocyclic group, or a combination thereof, and ^{∗} is a linking point with L^{a} and L¹ to L⁴; 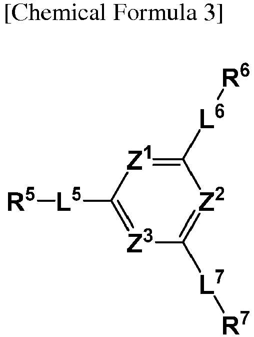
wherein, in Chemical Formula 3,
Z¹ to Z³ are each independently N or CR^{d}, wherein R^{d} is hydrogen, deuterium, a substituted or unsubstituted C1 to C30 alkyl group, a substituted or unsubstituted C6 to C30 aryl group, a substituted or unsubstituted C3 to C30 heterocyclic group, a substituted or unsubstituted silyl group, a substituted or unsubstituted amine group, a halogen, a cyano group, or a combination thereof, provided that at least two of Z¹ to Z³ are N,
L⁵ to L⁷ are each independently a single bond, a substituted or unsubstituted C6 to C20 arylene group, a substituted or unsubstituted divalent C2 to C20 heterocyclic group, or a combination thereof,
R⁵ to R⁷ are each independently a substituted or unsubstituted C6 to C30 aryl group, a substituted or unsubstituted C2 to C30 heterocyclic group, or a combination thereof, provided that at least one of R⁵ to R⁷ is a group represented by Chemical Formula B: 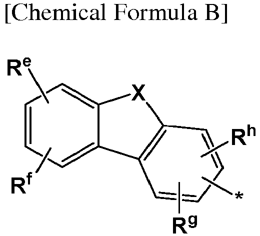
wherein, in Chemical Formula B,
X is O or S,
R^{e} to R^{h} are each independently hydrogen, deuterium, a substituted or unsubstituted C1 to C30 alkyl group, a substituted or unsubstituted C6 to C30 aryl group, a substituted or unsubstituted C2 to C30 heterocyclic group, a substituted or unsubstituted silyl group, a substituted or
unsubstituted amine group, a halogen, a cyano group, or a combination thereof,
R^{e} and R^{f} are each separate or are bonded with each other to form a ring,
R^{g} and R^{h} are each separate or are bonded with each other to form a ring, and
^{∗} is a linking point with one of L⁵ to L⁷.

2. The composition as claimed in claim 1, wherein the first compound is represented by one of Chemical Formula 1A to Chemical Formula 1C: 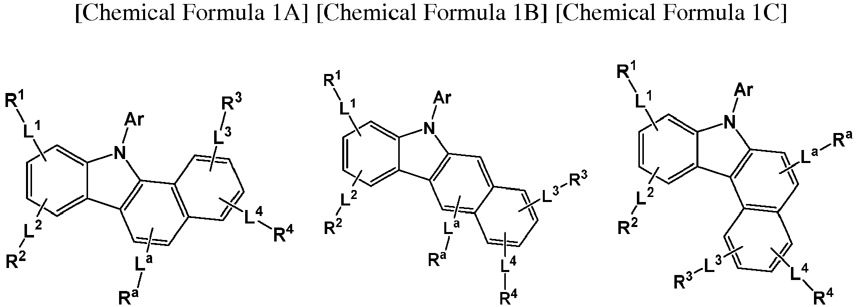 wherein, in Chemical Formula 1A to Chemical Formula 1C,
Ar is a substituted or unsubstituted C6 to C30 aryl group, a substituted or unsubstituted C2 to C30 heterocyclic group, or a combination thereof,
L^{a} and L¹ to L⁴ are each independently a single bond, a substituted or unsubstituted C6 to C20 arylene group, a substituted or unsubstituted divalent C2 to C20 heterocyclic group, or a combination thereof,
R^{a} and R¹ to R⁴ are each independently hydrogen, deuterium, a cyano group, a substituted or unsubstituted amine group, a substituted or unsubstituted C1 to C30 alkyl group, a substituted or unsubstituted C6 to C30 aryl group, a substituted or unsubstituted C2 to C30 heterocyclic group, or a combination thereof, provided that at least one of R^{a} and R¹ to R⁴ is a group represented by Chemical Formula A: 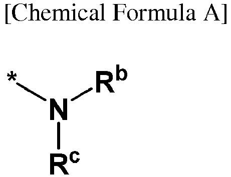
wherein, in Chemical Formula A,
R^{b} and R^{c} are each independently a substituted or unsubstituted C6 to C30 aryl group, a substituted or unsubstituted C2 to C30 heterocyclic group, or a combination thereof, and
^{∗} is a linking point with L^{a} and L¹ to L⁴.

3. The composition as claimed in claim 1 or 2, wherein the first compound is represented by one of Chemical Formula 1A-1 to Chemical Formula 1A-3, Chemical Formula 1B-1 to Chemical Formula 1B-3, and Chemical Formula 1C-1 to Chemical Formula 1C-3: 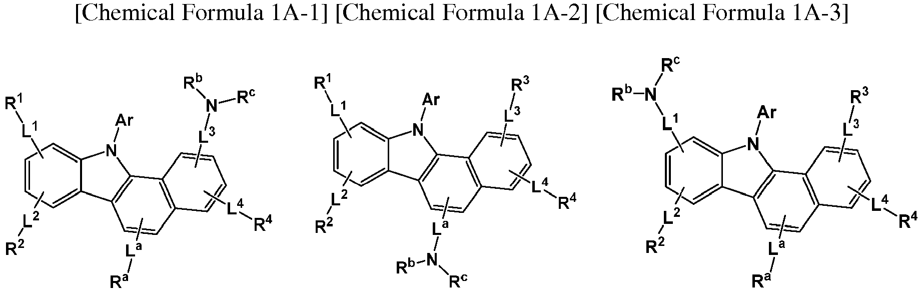 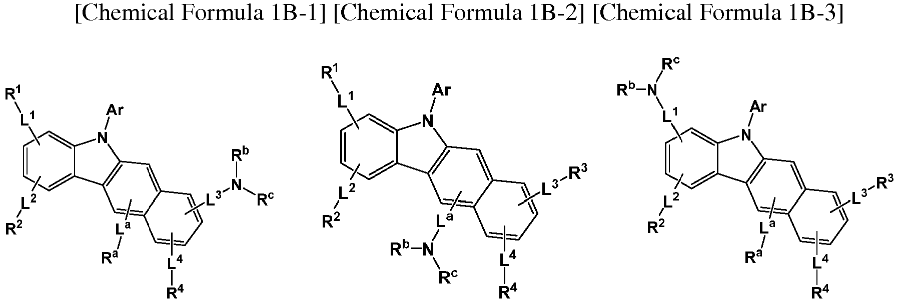 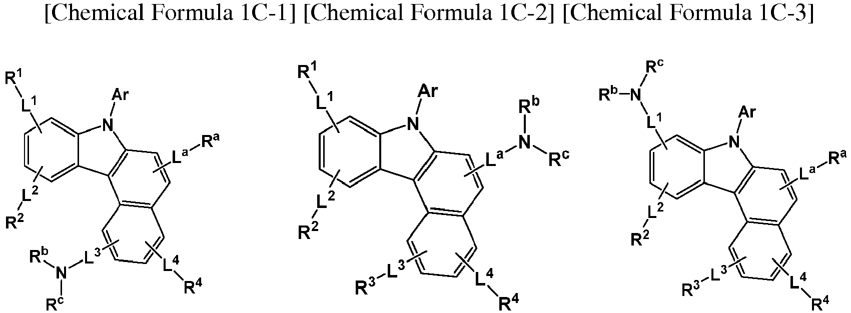 wherein, in Chemical Formula 1A-1 to Chemical Formula 1A-3, Chemical Formula 1B-1 to Chemical Formula 1B-3 and Chemical Formula 1C-1 to Chemical Formula 1C-3,
Ar is a substituted or unsubstituted C6 to C30 aryl group, a substituted or unsubstituted C2 to C30 heterocyclic group, or a combination thereof,
L^{a} and L¹ to L⁴ are each independently a single bond, a substituted or unsubstituted C6 to C20 arylene group, a substituted or unsubstituted divalent C2 to C20 heterocyclic group, or a combination thereof,
R^{a} and R¹ to R⁴ are each independently hydrogen, deuterium, a cyano group, a substituted or unsubstituted amine group, a substituted or unsubstituted C1 to C30 alkyl group, a substituted or unsubstituted C6 to C30 aryl group, a substituted or unsubstituted C2 to C30 heterocyclic group, or a combination thereof, and
R^{b} and R^{c} are each independently a substituted or unsubstituted phenyl group, a substituted or unsubstituted biphenyl group, a substituted or unsubstituted anthracenyl group, a substituted or unsubstituted naphthyl group, a substituted or unsubstituted phenanthrenyl group, a substituted or unsubstituted triphenylenyl group, a substituted or unsubstituted fluorenyl group, a substituted or unsubstituted carbazolyl group, a substituted or unsubstituted dibenzofuranyl group, or a substituted or unsubstituted dibenzothiophenyl group.

4. The composition as claimed in any of the claims 1 to 3, wherein the first compound is represented by Chemical Formula 1A-1-b: 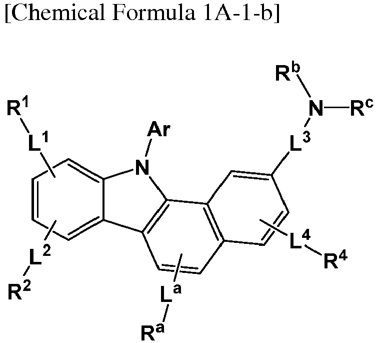 wherein, in Chemical Formula 1A-1-b,
Ar is a substituted or unsubstituted C6 to C30 aryl group, a substituted or unsubstituted C2 to C30 heterocyclic group, or a combination thereof,
L^{a} and L¹ to L⁴ are each independently a single bond, a substituted or unsubstituted C6 to C20 arylene group, a substituted or unsubstituted divalent C2 to C20 heterocyclic group, or a combination thereof,
R^{a}, R¹, R², and R⁴ are each independently hydrogen, deuterium, a cyano group, a substituted or unsubstituted C1 to C30 alkyl group, a substituted or unsubstituted C6 to C30 aryl group, a substituted or unsubstituted C2 to C30 heterocyclic group, or a combination thereof, and
R^{b} and R^{c} are each independently a substituted or unsubstituted phenyl group, a substituted or unsubstituted biphenyl group, a substituted or unsubstituted anthracenyl group, a substituted or unsubstituted naphthyl group, a substituted or unsubstituted phenanthrenyl group, a substituted or unsubstituted triphenylenyl group, a substituted or unsubstituted fluorenyl group, a substituted or unsubstituted carbazolyl group, a substituted or unsubstituted dibenzofuranyl group, or a substituted or unsubstituted dibenzothiophenyl group.

5. The composition as claimed in any of claims 1 to 4, wherein the second compound is represented by one of Chemical Formula 2A to Chemical Formula 2C: 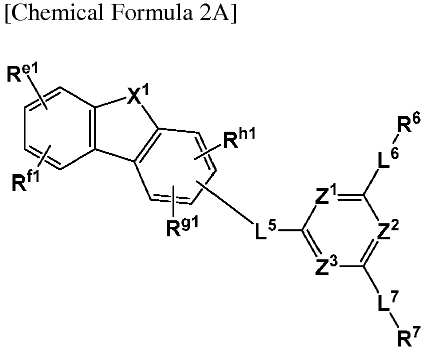 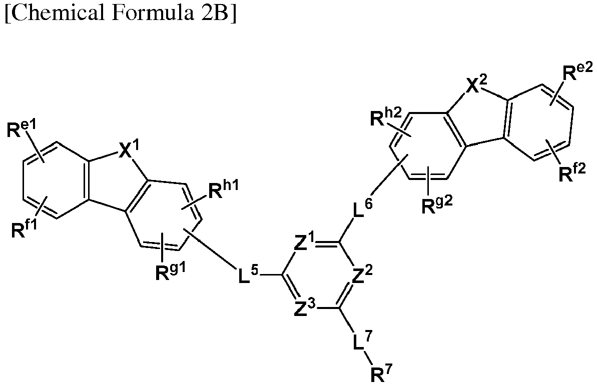 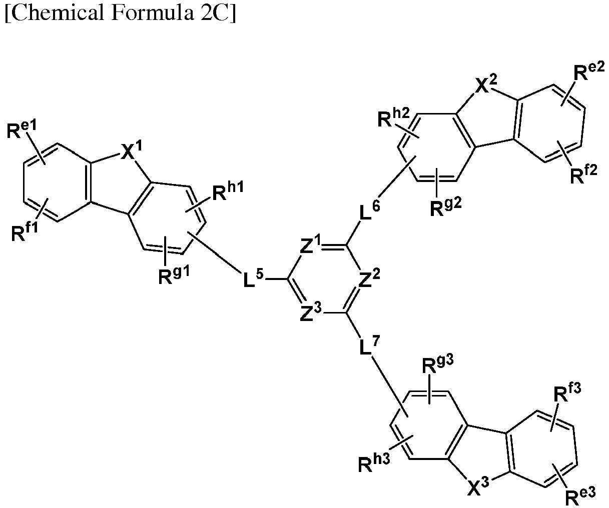 wherein, in Chemical Formulae 2A to 2C,
Z¹ to Z³ are each independently N or CR^{d}, wherein R^{d} is hydrogen, deuterium, a substituted or unsubstituted C1 to C30 alkyl group, a substituted or unsubstituted C6 to C30 aryl group, a substituted or unsubstituted C3 to C30 heterocyclic group, a substituted or unsubstituted silyl group, a substituted or unsubstituted amine group, a halogen, a cyano group, or a combination thereof, provided that at least two of Z¹ to Z³ are N,
L⁵ to L⁷ are each independently a single bond, a substituted or unsubstituted C6 to C20 arylene group, a substituted or unsubstituted divalent C2 to C20 heterocyclic group, or a combination thereof,
R⁶ and R⁷ are each independently a substituted or unsubstituted C6 to C30 aryl group, a substituted or unsubstituted C2 to C30 heterocyclic group, or a combination thereof,
X¹ to X³ are each independently O or S, and
R^{e1} to R^{e3}, R^{f1} to R^{f3}, R^{g1} to R^{g3} and R^{h1} to R^{h3} are each independently hydrogen, deuterium, a substituted or unsubstituted C1 to C30 alkyl group, a substituted or unsubstituted C6 to C30 aryl group, a substituted or unsubstituted C2 to C30 heterocyclic group, a substituted or unsubstituted silyl group, a substituted or unsubstituted amine group, a halogen, a cyano group, or a combination thereof.

6. The composition as claimed in any of claims 1 to 5, wherein:
the second compound is represented by Chemical Formula 2A or Chemical Formula 2B, and R⁶ and R⁷ are each independently a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted phenanthrenyl group, a substituted or unsubstituted triphenylenyl group, a substituted or unsubstituted fluorenyl group, a substituted or unsubstituted pyridinyl group, a substituted or unsubstituted pyrimidinyl group, a substituted or unsubstituted triazinyl group, or a combination thereof.

7. The composition as claimed in any of claims 1 to 6, wherein Chemical Formula B is represented by one of Chemical Formula B-1 to Chemical Formula B-4: 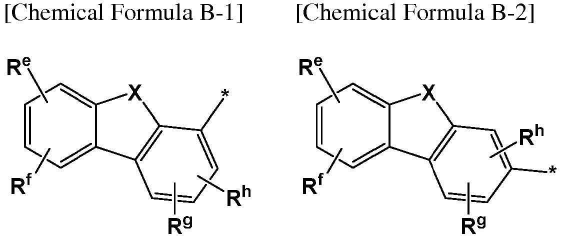 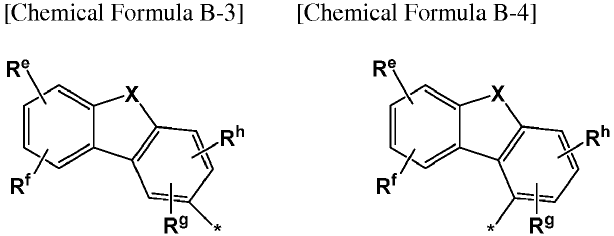 wherein, in Chemical Formula B-1 to Chemical Formula B-4,
X is O or S,
R^{e} to R^{h} are each independently hydrogen, deuterium, a substituted or unsubstituted C1 to C30 alkyl group, a substituted or unsubstituted C6 to C30 aryl group, a substituted or unsubstituted C3 to C30 heterocyclic group, a substituted or unsubstituted silyl group, a substituted or unsubstituted amine group, a halogen, a cyano group, or a combination thereof,
R^{e} and R^{f} are each separate or are bonded with each other to form a ring,
R^{g} and R^{h} are each separate or are bonded with each other to form a ring, and
^{∗} is a linking point with one of L⁵ to L⁷.

8. The composition as claimed in any of claims 1 to 7, wherein R⁵ to R⁷ are each independently a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted phenanthrenyl group, a substituted or unsubstituted triphenylenyl group, a substituted or unsubstituted fluorenyl group, a substituted or unsubstituted pyridinyl group, a substituted or unsubstituted pyrimidinyl group, a substituted or unsubstituted triazinyl group, the group represented by Chemical Formula B, or a combination thereof.

9. The composition as claimed in any of claims 1 to 8, wherein:
the first compound is represented by Chemical Formula 1A-1-b, and
the second compound is represented by Chemical Formula 2A or Chemical Formula 2B, 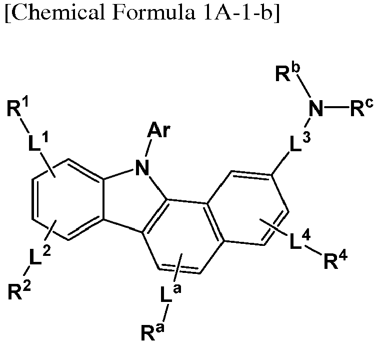
wherein, in Chemical Formula 1A-1-b,
Ar is a substituted or unsubstituted phenyl group, a substituted or unsubstituted biphenyl group, a substituted or unsubstituted terphenyl group, a substituted or unsubstituted naphthyl group, a substituted or unsubstituted anthracenyl group, a substituted or unsubstituted phenanthrenyl group, a substituted or unsubstituted triphenylenyl group, a substituted or
unsubstituted fluorenyl group, a substituted or unsubstituted dibenzofuranyl group, a substituted or unsubstituted dibenzothiophenyl group, a substituted or unsubstituted carbazolyl group, or a combination thereof,
L^{a} and L¹ to L⁴ are each independently a single bond, a substituted or unsubstituted phenylene group, a substituted or unsubstituted biphenylene group, a substituted or unsubstituted terphenylene group, or a substituted or unsubstituted naphthylene group, R^{a}, R¹, R², and R⁴ are each independently hydrogen, deuterium, a cyano group, a substituted or unsubstituted C1 to C30 alkyl group, a substituted or unsubstituted C6 to C30 aryl group, a substituted or unsubstituted C2 to C30 heterocyclic group, or a combination thereof, and
R^{b} and R^{c} are each independently a substituted or unsubstituted phenyl group, a substituted or unsubstituted biphenyl group, a substituted or unsubstituted anthracenyl group, a substituted or unsubstituted naphthyl group, a substituted or unsubstituted phenanthrenyl group, a substituted or unsubstituted triphenylenyl group, a substituted or unsubstituted fluorenyl group, a substituted or unsubstituted carbazolyl group, a substituted or unsubstituted
dibenzofuranyl group, or a substituted or unsubstituted dibenzothiophenyl group; 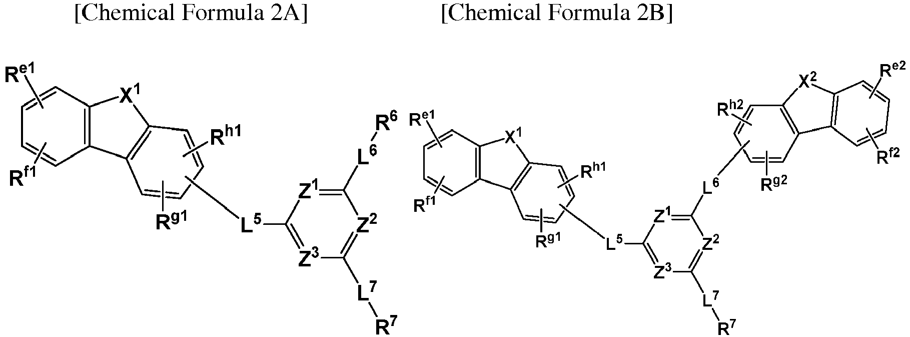
wherein, in Chemical Formula 2A and Chemical Formula 2B,
Z¹ to Z³ are each independently N or CR^{d}, provided that at least two of Z¹ to Z³ are N,
L⁵ to L⁷ are each independently a single bond, a substituted or unsubstituted phenylene group,
a substituted or unsubstituted biphenylene group, a substituted or unsubstituted terphenylene group, or a substituted or unsubstituted naphthylene group,
R⁶ and R⁷ are each independently a substituted or unsubstituted phenyl group, a substituted or
unsubstituted biphenyl group, a substituted or unsubstituted terphenyl group, a substituted or
unsubstituted quaterphenyl group, a substituted or unsubstituted naphthyl group, a substituted or unsubstituted anthracenyl group, a substituted or unsubstituted phenanthrenyl group, a substituted or unsubstituted triphenylenyl group, a substituted or unsubstituted fluorenyl group, a substituted or unsubstituted pyridinyl group, a substituted or unsubstituted pyrimidinyl group, a substituted or unsubstituted triazinyl group, a substituted or
unsubstituted dibenzofuranyl group, or a substituted or unsubstituted dibenzothiophenyl group, X¹ and X² are each independently O or S, and
R^{d}, R^{e1}, R^{e2}, R^{f1}, R^{f2}, R^{g1}, R^{g2}, R^{h1}, and R^{h2} are each independently hydrogen, deuterium, a substituted or unsubstituted C1 to C30 alkyl group, a substituted or unsubstituted C6 to C30 aryl group, a substituted or unsubstituted C2 to C30 heterocyclic group, a substituted or
unsubstituted silyl group, a substituted or unsubstituted amine group, a halogen, a cyano group, or a combination thereof.

10. The composition as claimed in any of claims 1 to 9, wherein the second compound is represented by Chemical Formula 2B-1: 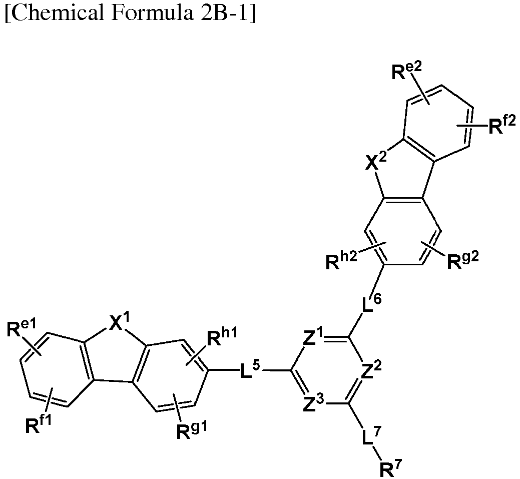 wherein, in Chemical Formula 2B-1,
Z¹ to Z³ are each independently N or CR^{d}, provided that at least two of Z¹ to Z³ are N,
L⁵ to L⁷ are each independently a single bond, a substituted or unsubstituted phenylene group, a substituted or unsubstituted biphenylene group, a substituted or unsubstituted terphenylene group, or a substituted or unsubstituted naphthylene group,
R⁷ is a substituted or unsubstituted phenyl group, a substituted or unsubstituted biphenyl group, a substituted or unsubstituted terphenyl group, a substituted or unsubstituted quaterphenyl group, a substituted or unsubstituted naphthyl group, a substituted or unsubstituted anthracenyl group, a substituted or unsubstituted phenanthrenyl group, a substituted or unsubstituted triphenylenyl group, a substituted or unsubstituted fluorenyl group, a substituted or unsubstituted pyridinyl group, a substituted or unsubstituted pyrimidinyl group, a substituted or unsubstituted triazinyl group, a substituted or unsubstituted dibenzofuranyl group, or a substituted or unsubstituted dibenzothiophenyl group, X¹ and X² are each independently O or S, and
R^{d}, R^{e1}, R^{e2}, R^{f1}, R^{f2}, R^{g1}, R^{g2}, R^{h1}, and R^{h2} are each independently hydrogen, deuterium, a substituted or unsubstituted C1 to C30 alkyl group, a substituted or unsubstituted C6 to C30 aryl group, a substituted or unsubstituted C2 to C30 heterocyclic group, a substituted or unsubstituted silyl group, a substituted or unsubstituted amine group, a halogen, a cyano group, or a combination thereof.

11. An organic optoelectronic device, comprising:
an anode and a cathode facing each other; and
at least one organic layer disposed between the anode and the cathode, wherein the organic layer includes the composition as claimed in any one of claims 1 to 10.

12. The organic optoelectronic device as claimed in claim 11, wherein:
the organic layer includes a light emitting layer, and
the light emitting layer includes the composition.

13. The organic optoelectronic device as claimed in claim 11 or 12, wherein the first compound and the second compound are included as a phosphorescent host of the light emitting layer.

14. The organic optoelectronic device as claimed in claim 11, wherein the composition is a red light emitting composition.

15. A display device comprising the organic optoelectronic device as claimed in claim 11.

## Patentansprüche

1. Zusammensetzung mit:
einer ersten Verbindung, wobei die erste Verbindung durch eine Kombination der chemischen Formel 1 und der chemischen Formel 2 dargestellt ist, die aneinander gebunden sind; und
einer zweiten Verbindung, wobei die zweite Verbindung durch die chemische Formel 3 dargestellt ist, 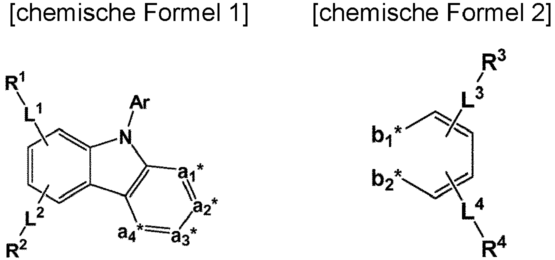
wobei in der chemischen Formel 1 und in der chemischen Formel 2,
Ar eine substituierte oder unsubstituierte C6- bis C30-Arylgruppe, eine substituierte oder unsubstituierte heterozyklische C2- bis C30-Gruppe oder eine Kombination davon ist,
benachbarte zwei der Komponenten a1* bis a₄* an b₁* bzw. b₂* gebunden sind und die verbleibenden zwei Komponenten von a₁* bis a₄*, die nicht an b₁* und b₂* gebunden sind, jeweils unabhängig voneinander C-L^{a}-R^{a} sind,
L^{a} und L¹ bis L⁴ jeweils unabhängig voneinander eine Einfachbindung, eine substituierte oder unsubstituierte C6- bis C20-Arylengruppe, eine substituierte oder unsubstituierte zweiwertige heterozyklische C2- bis C20-Gruppe oder eine Kombination davon sind,
R^{a} und R¹ bis R⁴ jeweils unabhängig voneinander Wasserstoff, Deuterium, eine Cyanogruppe, eine substituierte oder unsubstituierte Amingruppe, eine substituierte oder unsubstituierte C1- bis C30-Alkylgruppe, eine substituierte oder unsubstituierte C6- bis C30-Arylgruppe, eine substituierte oder unsubstituierte heterozyklische C2- bis C30-Gruppe oder eine Kombination davon sind, mit der Maßgabe, dass mindestens eine der Komponenten R^{a} und R¹ bis R⁴ eine durch die chemische Formel A dargestellte Gruppe ist: 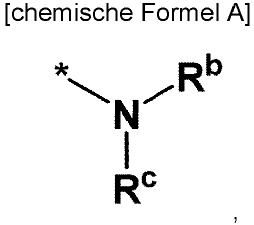
wobei in der chemischen Formel A,
R^{b} und R^{c} jeweils unabhängig voneinander eine substituierte oder unsubstituierte C6- bis C30-Arylgruppe, eine substituierte oder unsubstituierte heterozyklische C2- bis C30-Gruppe oder eine Kombination davon sind, und
* eine Bindungsstelle mit L^{a} und L¹ bis L⁴ ist; 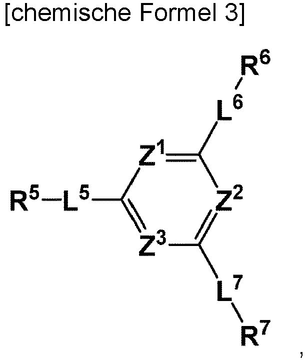
wobei in der chemischen Formel 3,
Z¹ bis Z³ jeweils unabhängig voneinander N oder CR^{d} sind, wobei R^{d} Wasserstoff, Deuterium, eine substituierte oder unsubstituierte C1- bis C30-Alkylgruppe, eine substituierte oder unsubstituierte C6- bis C30-Arylgruppe, eine substituierte oder unsubstituierte heterozyklische C3- bis C30-Gruppe, eine substituierte oder unsubstituierte Silylgruppe, eine substituierte oder unsubstituierte Amingruppe, ein Halogen, eine Cyanogruppe oder eine Kombination davon ist, mit der Maßgabe, dass mindestens zwei der Komponenten Z¹ bis Z³ N sind,
L⁵ bis L⁷ jeweils unabhängig voneinander eine Einfachbindung, eine substituierte oder unsubstituierte C6- bis C20-Arylengruppe, eine substituierte oder unsubstituierte zweiwertige heterozyklische C2- bis C20-Gruppe oder eine Kombination davon sind,
R⁵ bis R⁷ jeweils unabhängig voneinander eine substituierte oder unsubstituierte C6- bis C30-Arylgruppe, eine substituierte oder unsubstituierte heterozyklische C2- bis C30-Gruppe oder eine Kombination davon sind, mit der Maßgabe, dass mindestens eine der Komponenten R⁵ bis R⁷ eine durch die chemische Formel B dargestellte Gruppe ist: 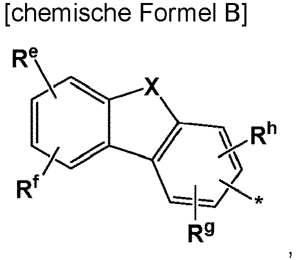
wobei in der chemischen Formel B,
X O oder S ist,
R^{e} bis R^{h} jeweils unabhängig voneinander Wasserstoff, Deuterium, eine substituierte oder unsubstituierte C1- bis C30-Alkylgruppe, eine substituierte oder unsubstituierte C6- bis C30-Arylgruppe, eine substituierte oder unsubstituierte heterocyklische C2- bis C30-Gruppe, eine substituierte oder unsubstituierte Silylgruppe, eine substituierte oder unsubstituierte Amingruppe, ein Halogen, eine Cyanogruppe oder eine Kombination davon sind,
R^{e} und R^{f} jeweils separat oder unter Bildung eines Rings miteinander verbunden sind,
R^{g} und R^{h} jeweils separat oder unter Bildung eines Rings miteinander verbunden sind, und
* eine Bindungsstelle mit einer Komponente von L⁵ bis L⁷ ist.

2. Zusammensetzung nach Anspruch 1, wobei die erste Verbindung durch eine der chemischen Formeln 1A bis 1C dargestellt ist: 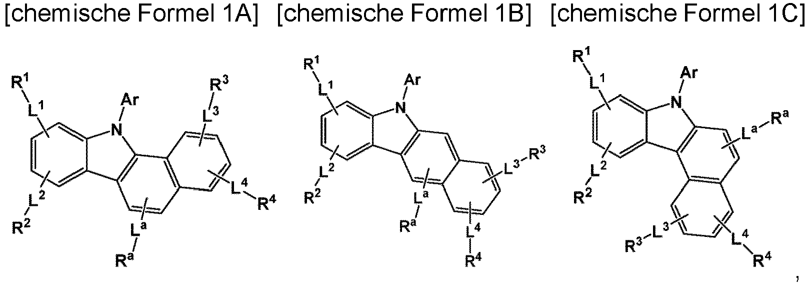 wobei in den chemischen Formeln 1A bis 1C,
Ar eine substituierte oder unsubstituierte C6- bis C30-Arylgruppe, eine substituierte oder unsubstituierte heterozyklische C2- bis C30-Gruppe oder eine Kombination davon ist,
L^{a} und L¹ bis L⁴ jeweils unabhängig voneinander eine Einfachbindung, eine substituierte oder unsubstituierte C6- bis C20-Arylengruppe, eine substituierte oder unsubstituierte zweiwertige heterozyklische C2- bis C20-Gruppe oder eine Kombination davon sind,
R^{a} und R¹ bis R⁴ jeweils unabhängig voneinander Wasserstoff, Deuterium, eine Cyanogruppe, eine substituierte oder unsubstituierte Amingruppe, eine substituierte oder unsubstituierte C1- bis C30-Alkylgruppe, eine substituierte oder unsubstituierte C6- bis C30-Arylgruppe, eine substituierte oder unsubstituierte heterozyklische C2- bis C30-Gruppe oder eine Kombination davon sind, mit der Maßgabe, dass mindestens eine der Komponenten R^{a} und R¹ bis R⁴ eine durch die chemische Formel A dargestellte Gruppe ist: 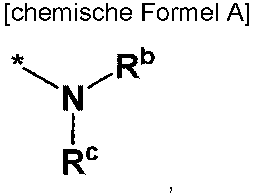
wobei in der chemischen Formel A,
R^{b} und R^{c} jeweils unabhängig voneinander eine substituierte oder unsubstituierte C6- bis C30-Arylgruppe, eine substituierte oder unsubstituierte heterozyklische C2- bis C30-Gruppe oder eine Kombination davon sind, und
* eine Bindungsstelle mit L^{a} und L¹ bis L⁴ ist.

3. Zusammensetzung nach Anspruch 1 oder 2, wobei die erste Verbindung durch eine der folgenden Formeln dargestellt ist: chemische Formel 1A-1 bis chemische Formel 1A-3, chemische Formel 1B-1 bis chemische Formel 1B-3 und chemische Formel 1C-1 bis chemische Formel 1C-3: 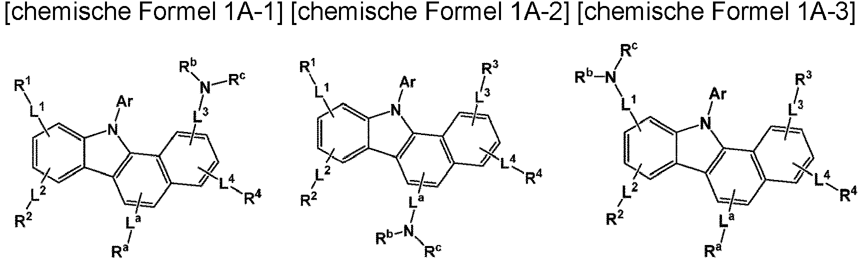 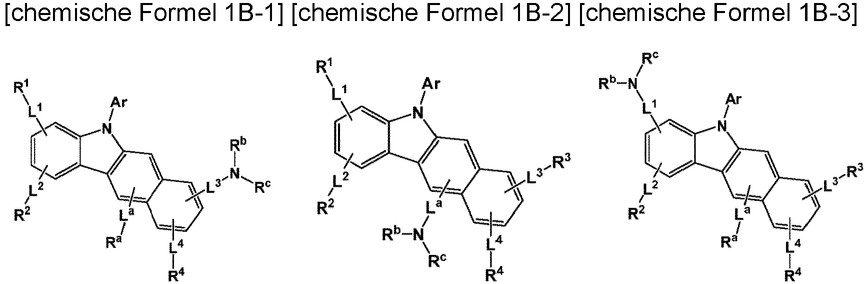 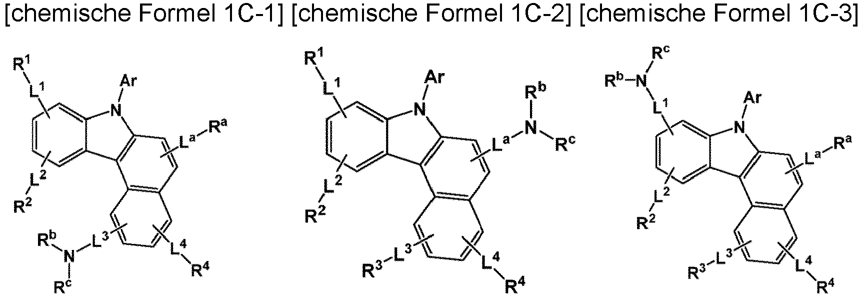
wobei in den chemischen Formeln 1A-1 bis 1A-3, den chemischen Formeln 1B-1 bis 1B-3 und den chemischen Formeln 1C-1 bis 1C-3,
Ar eine substituierte oder unsubstituierte C6- bis C30-Arylgruppe, eine substituierte oder unsubstituierte heterozyklische C2- bis C30-Gruppe oder eine Kombination davon ist,
L^{a} und L¹ bis L⁴ jeweils unabhängig voneinander eine Einfachbindung, eine substituierte oder unsubstituierte C6- bis C20-Arylengruppe, eine substituierte oder unsubstituierte zweiwertige heterozyklische C2- bis C20-Gruppe oder eine Kombination davon sind,
R^{a} und R¹ bis R⁴ jeweils unabhängig voneinander Wasserstoff, Deuterium, eine Cyanogruppe, eine substituierte oder unsubstituierte Amingruppe, eine substituierte oder unsubstituierte C1- bis C30-Alkylgruppe, eine substituierte oder unsubstituierte C6- bis C30-Arylgruppe, eine substituierte oder unsubstituierte heterozyklische C2- bis C30-Gruppe oder eine Kombination davon sind, und
R^{b} und R^{c} jeweils unabhängig voneinander eine substituierte oder unsubstituierte Phenylgruppe, eine substituierte oder unsubstituierte Biphenylgruppe, eine substituierte oder unsubstituierte Anthracenylgruppe, eine substituierte oder unsubstituierte Naphthylgruppe, eine substituierte oder unsubstituierte Phenanthrenylgruppe, eine substituierte oder unsubstituierte Triphenylenylgruppe, eine substituierte oder unsubstituierte Fluorenylgruppe, eine substituierte oder unsubstituierte Carbazolylgruppe, eine substituierte oder unsubstituierte Dibenzofuranylgruppe oder eine substituierte oder unsubstituierte Dibenzothiophenylgruppe sind.

4. Zusammensetzung nach einem der Ansprüche 1 bis 3, wobei die erste Verbindung durch die chemische Formel 1A-1-b dargestellt ist: 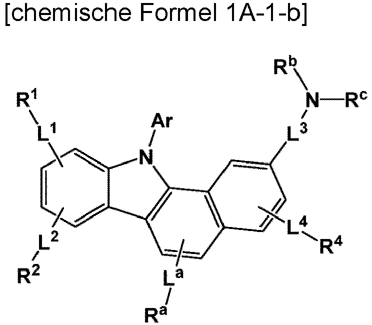
wobei in der chemischen Formel 1A-1-b
Ar eine substituierte oder unsubstituierte C6- bis C30-Arylgruppe, eine substituierte oder unsubstituierte heterozyklische C2- bis C30-Gruppe oder eine Kombination davon ist,
L^{a} und L¹ bis L⁴ jeweils unabhängig voneinander eine Einfachbindung, eine substituierte oder unsubstituierte C6- bis C20-Arylengruppe, eine substituierte oder unsubstituierte zweiwertige heterozyklische C2- bis C20-Gruppe oder eine Kombination davon sind,
R^{a}, R¹, R² und R⁴ jeweils unabhängig voneinander Wasserstoff, Deuterium, eine Cyanogruppe, eine substituierte oder unsubstituierte C1- bis C30-Alkylgruppe, eine substituierte oder unsubstituierte C6- bis C30-Arylgruppe, eine substituierte oder unsubstituierte heterozyklische C2- bis C30-Gruppe oder eine Kombination davon sind, und
R^{b} und R^{c} jeweils unabhängig voneinander eine substituierte oder unsubstituierte Phenylgruppe, eine substituierte oder unsubstituierte Biphenylgruppe, eine substituierte oder unsubstituierte Anthracenylgruppe, eine substituierte oder unsubstituierte Naphthylgruppe, eine substituierte oder unsubstituierte Phenanthrenylgruppe, eine substituierte oder unsubstituierte Triphenylenylgruppe, eine substituierte oder unsubstituierte Fluorenylgruppe, eine substituierte oder unsubstituierte Carbazolylgruppe, eine substituierte oder unsubstituierte Dibenzofuranylgruppe oder eine substituierte oder unsubstituierte Dibenzothiophenylgruppe sind.

5. Zusammensetzung nach einem der Ansprüche 1 bis 4, wobei die zweite Verbindung durch eine der chemischen Formeln 2A bis 2C dargestellt ist: 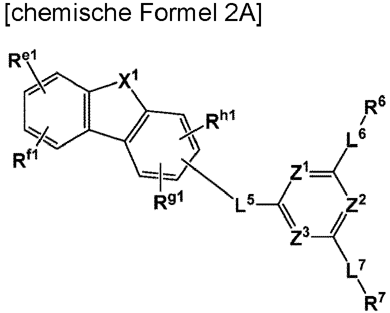 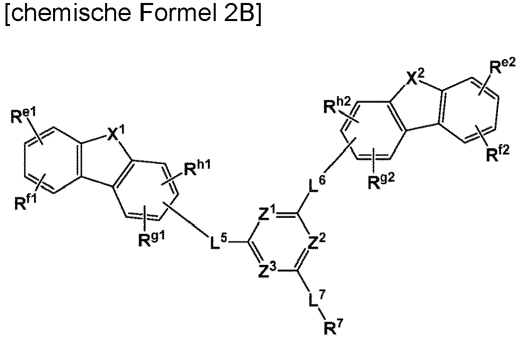 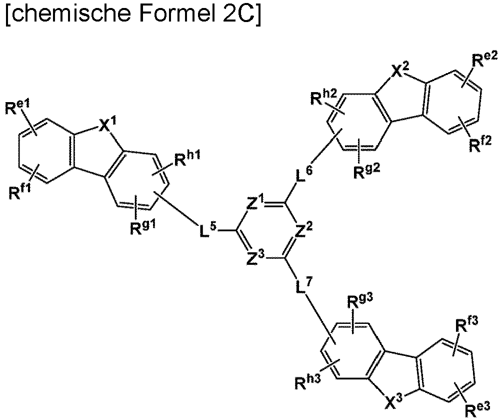
wobei in den chemischen Formeln 2A bis 2C
Z¹ bis Z³ jeweils unabhängig voneinander N oder CR^{d} sind, wobei R^{d} Wasserstoff, Deuterium, eine substituierte oder unsubstituierte C1- bis C30-Alkylgruppe, eine substituierte oder unsubstituierte C6- bis C30-Arylgruppe, eine substituierte oder unsubstituierte heterozyklische C3- bis C30-Gruppe, eine substituierte oder unsubstituierte Silylgruppe, eine substituierte oder unsubstituierte Amingruppe, ein Halogen, eine Cyanogruppe oder eine Kombination davon ist, mit der Maßgabe, dass mindestens zwei Komponenten von Z¹ bis Z³ N sind,
L⁵ bis L⁷ jeweils unabhängig voneinander eine Einfachbindung, eine substituierte oder unsubstituierte C6- bis C20-Arylengruppe, eine substituierte oder unsubstituierte zweiwertige heterozyklische C2- bis C20-Gruppe oder eine Kombination davon sind,
R⁶ und R⁷ jeweils unabhängig voneinander eine substituierte oder unsubstituierte C6- bis C30-Arylgruppe, eine substituierte oder unsubstituierte heterozyklische C2- bis C30- Gruppe oder eine Kombination davon sind,
X¹ bis X³ jeweils unabhängig voneinander O oder S sind, und
R^{e1} bis R^{e3}, R^{f1} bis R^{f3}, R^{g1} bis R^{g3} und R^{h1} bis R^{h3} jeweils unabhängig voneinander Wasserstoff, Deuterium, eine substituierte oder unsubstituierte C1-bis C30-Alkylgruppe, eine substituierte oder unsubstituierte C6- bis C30-Arylgruppe, eine substituierte oder unsubstituierte heterozyklische C2- bis C30-Gruppe, eine substituierte oder unsubstituierte Silylgruppe, eine substituierte oder unsubstituierte Amingruppe, ein Halogen, eine Cyanogruppe oder eine Kombination davon sind.

6. Zusammensetzung nach einem der Ansprüche 1 bis 5, wobei:
die zweite Verbindung durch die chemische Formel 2A oder die chemische Formel 2B dargestellt ist und R⁶ und R⁷ jeweils unabhängig voneinander eine substituierte oder unsubstituierte Phenylgruppe, eine substituierte oder unsubstituierte Biphenylgruppe, eine substituierte oder unsubstituierte Terphenylgruppe, eine substituierte oder unsubstituierte Naphthylgruppe, eine substituierte oder unsubstituierte Anthracenylgruppe, eine substituierte oder unsubstituierte Phenanthrenylgruppe, eine substituierte oder unsubstituierte Triphenylenylgruppe, eine substituierte oder unsubstituierte Fluorenylgruppe, eine substituierte oder unsubstituierte Pyridinylgruppe, eine substituierte oder unsubstituierte Pyrimidinylgruppe, eine substituierte oder unsubstituierte Triazinylgruppe oder eine Kombination davon sind.

7. Zusammensetzung nach einem der Ansprüche 1 bis 6, wobei die chemische Formel B durch eine der chemischen Formeln B-1 bis B-4 dargestellt ist: 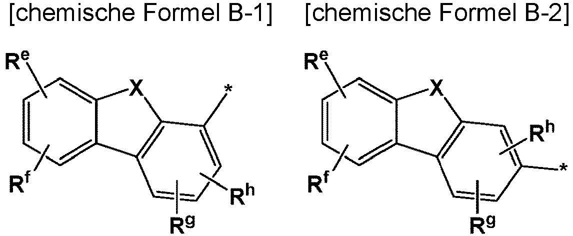 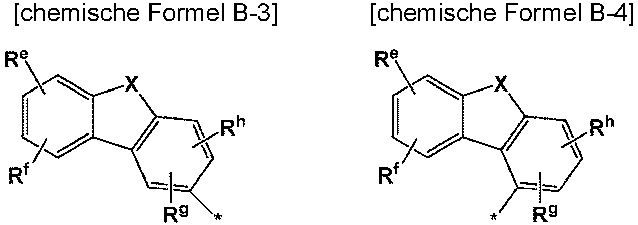 wobei in den chemischen Formeln B-1 bis B-4
X O oder S ist,
R^{e} bis R^{h} jeweils unabhängig voneinander Wasserstoff, Deuterium, eine substituierte oder unsubstituierte C1- bis C30-Alkylgruppe, eine substituierte oder unsubstituierte C6- bis C30-Arylgruppe, eine substituierte oder unsubstituierte heterozyklische Gruppe, eine substituierte oder unsubstituierte Silylgruppe, eine substituierte oder unsubstituierte Amingruppe, ein Halogen, eine Cyanogruppe oder eine Kombination davon sind,
R^{e} und R^{f} jeweils separat oder unter Bildung eines Rings miteinander verbunden sind,
R^{g} und R^{h} jeweils separat oder unter Bildung eines Rings miteinander verbunden sind, und
* eine Bindungsstelle mit einer Komponente von L⁵ bis L⁷ ist.

8. Zusammensetzung nach einem der Ansprüche 1 bis 7, wobei R⁵ bis R⁷ jeweils unabhängig voneinander eine substituierte oder unsubstituierte Phenylgruppe, eine substituierte oder unsubstituierte Biphenylgruppe, eine substituierte oder unsubstituierte Terphenylgruppe, eine substituierte oder unsubstituierte Naphthylgruppe, eine substituierte oder unsubstituierte Anthracenylgruppe, eine substituierte oder unsubstituierte Phenanthrenylgruppe, eine substituierte oder unsubstituierte Triphenylenylgruppe, eine substituierte oder unsubstituierte Fluorenylgruppe, eine substituierte oder unsubstituierte Pyridinylgruppe, eine substituierte oder unsubstituierte Pyrimidinylgruppe, eine substituierte oder unsubstituierte Triazinylgruppe, die durch die chemische Formel B dargestellte Gruppe oder eine Kombination davon sind.

9. Zusammensetzung nach einem der Ansprüche 1 bis 8, wobei:
die erste Verbindung durch die chemische Formel 1A-1-b dargestellt ist und die zweite Verbindung durch die chemische Formel 2A oder die chemische Formel 2B dargestellt ist, 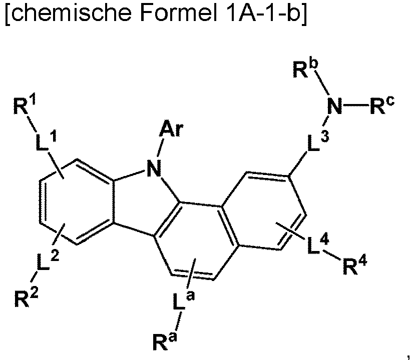
wobei in der chemischen Formel 1A-1-b Ar eine substituierte oder unsubstituierte Phenylgruppe, eine substituierte oder unsubstituierte Biphenylgruppe, eine substituierte oder unsubstituierte Terphenylgruppe, eine substituierte oder unsubstituierte Naphthylgruppe, eine substituierte oder unsubstituierte Anthracenylgruppe, eine substituierte oder unsubstituierte Phenanthrenylgruppe, eine substituierte oder unsubstituierte Triphenylenylgruppe, eine substituierte oder unsubstituierte Fluorenylgruppe, eine substituierte oder unsubstituierte Dibenzofuranylgruppe, eine substituierte oder unsubstituierte Dibenzothiophenylgruppe, eine substituierte oder unsubstituierte Carbazolylgruppe oder eine Kombination davon ist,
L^{a} und L¹ bis L⁴ jeweils unabhängig voneinander eine Einfachbindung, eine substituierte oder unsubstituierte Phenylengruppe, eine substituierte oder unsubstituierte Biphenylengruppe, eine substituierte oder unsubstituierte Terphenylengruppe oder eine substituierte oder unsubstituierte Naphthylengruppe sind,
R^{a}, R¹, R² und R⁴ jeweils unabhängig voneinander Wasserstoff, Deuterium, eine Cyanogruppe, eine substituierte oder unsubstituierte C1- bis C30-Alkylgruppe, eine substituierte oder unsubstituierte C6- bis C30-Arylgruppe, eine substituierte oder unsubstituierte heterozyklische C2- bis C30-Gruppe oder eine Kombination davon sind, und
R^{b} und R^{c} jeweils unabhängig voneinander eine substituierte oder unsubstituierte Phenylgruppe, eine substituierte oder unsubstituierte Biphenylgruppe eine substituierte oder unsubstituierte Anthracenylgruppe, eine substituierte oder unsubstituierte Naphthylgruppe, eine substituierte oder unsubstituierte Phenanthrenylgruppe, eine substituierte oder unsubstituierte Triphenylenylgruppe, eine substituierte oder unsubstituierte Fluorenylgruppe, eine substituierte oder unsubstituierte Carbazolylgruppe, eine substituierte oder unsubstituierte Dibenzofuranylgruppe oder eine substituierte oder unsubstituierte Dibenzothiophenylgruppe sind; 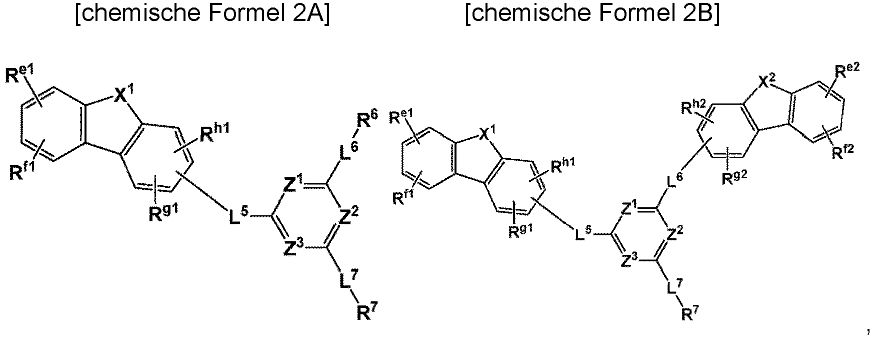 wobei in der chemischen Formel 2A und in der chemischen Formel 2B
Z¹ bis Z³ jeweils unabhängig voneinander N oder CR^{d} sind, mit der Maßgabe, dass mindestens zwei Komponenten von Z¹ bis Z³ N sind,
L⁵ bis L⁷ jeweils unabhängig voneinander eine Einfachbindung, eine substituierte oder unsubstituierte Phenylengruppe, eine substituierte oder unsubstituierte Biphenylengruppe, eine substituierte oder unsubstituierte Terphenylengruppe oder eine substituierte oder unsubstituierte Naphthylengruppe sind,
R⁶ und R⁷ jeweils unabhängig voneinander eine substituierte oder unsubstituierte Phenylgruppe, eine substituierte oder unsubstituierte Biphenylgruppe, eine substituierte oder unsubstituierte Terphenylgruppe, eine substituierte oder unsubstituierte Quaterphenylgruppe, eine substituierte oder unsubstituierte Naphthylgruppe, eine substituierte oder unsubstituierte Anthracenylgruppe, eine substituierte oder unsubstituierte Phenanthrenylgruppe, eine substituierte oder unsubstituierte Triphenylenylgruppe, eine substituierte oder unsubstituierte Fluorenylgruppe, eine substituierte oder unsubstituierte Pyridinylgruppe, eine substituierte oder unsubstituierte Pyrimidinylgruppe, eine substituierte oder unsubstituierte Triazinylgruppe, eine substituierte oder unsubstituierte Dibenzofuranylgruppe oder eine substituierte oder unsubstituierte Dibenzothiophenylgruppe sind,
X¹ und X² jeweils unabhängig voneinander O oder S sind, und
R^{d}, R^{e1}, R^{e2}, R^{f1}, R^{f2}, R^{g1}, R^{g2}, R^{h1} und R^{h2} jeweils unabhängig voneinander Wasserstoff, Deuterium, eine substituierte oder unsubstituierte C1- bis C30-Alkylgruppe, eine substituierte oder unsubstituierte C6- bis C30-Arylgruppe, eine substituierte oder unsubstituierte heterozyklische C2- bis C30-Gruppe, eine substituierte oder unsubstituierte Silylgruppe, eine substituierte oder unsubstituierte Amingruppe, ein Halogen, eine Cyanogruppe oder eine Kombination davon sind.

10. Zusammensetzung nach einem der Ansprüche 1 bis 9, wobei die zweite Verbindung durch die chemische Formel 2B-1 dargestellt ist: 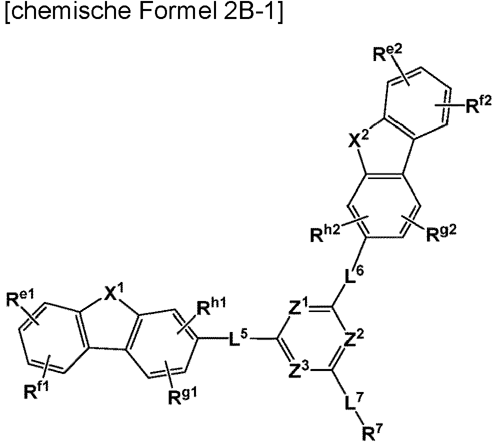 wobei in der chemischen Formel 2B-1
Z¹ bis Z³ jeweils unabhängig voneinander N oder CR^{d} sind, mit der Maßgabe, dass mindestens zwei Komponenten von Z¹ bis Z³ N sind,
L⁵ bis L⁷ jeweils unabhängig voneinander eine Einfachbindung, eine substituierte oder unsubstituierte Phenylengruppe, eine substituierte oder unsubstituierte Biphenylengruppe, eine substituierte oder unsubstituierte Terphenylengruppe oder eine substituierte oder unsubstituierte Naphthylengruppe sind,
R⁷ eine substituierte oder unsubstituierte Phenylgruppe, eine substituierte oder unsubstituierte Biphenylgruppe, eine substituierte oder unsubstituierte Terphenylgruppe, eine substituierte oder unsubstituierte Quaterphenylgruppe, eine substituierte oder unsubstituierte Naphthylgruppe, eine substituierte oder unsubstituierte Anthracenylgruppe, eine substituierte oder unsubstituierte Phenanthrenylgruppe, eine substituierte oder unsubstituierte Triphenylenylgruppe, eine substituierte oder unsubstituierte Fluorenylgruppe, eine substituierte oder unsubstituierte Pyridinylgruppe, eine substituierte oder unsubstituierte Pyrimidinylgruppe, eine substituierte oder unsubstituierte Triazinylgruppe, eine substituierte oder unsubstituierte Dibenzofuranylgruppe oder eine substituierte oder unsubstituierte Dibenzothiophenylgruppe ist,
X¹ und X² jeweils unabhängig voneinander O oder S sind, und
R^{d}, R^{e1}, R^{e2}, R^{f1}, R^{f2}, R^{g1}, R^{g2}, R^{h1} und R^{h2} jeweils unabhängig voneinander Wasserstoff, Deuterium, eine substituierte oder unsubstituierte C1- bis C30-Alkylgruppe, eine substituierte oder unsubstituierte C6- bis C30-Arylgruppe, eine substituierte oder unsubstituierte heterozyklische C2- bis C30-Gruppe, eine substituierte oder unsubstituierte Silylgruppe, eine substituierte oder unsubstituierte Amingruppe, ein Halogen, eine Cyanogruppe oder eine Kombination davon sind.

11. Organische optoelektronische Vorrichtung, mit:
einer Anode und einer Kathode, die einander zugewandt sind; und
mindestens einer zwischen der Anode und der Kathode angeordneten organischen Schicht, wobei die organische Schicht die Zusammensetzung nach einem der Ansprüche 1 bis 10 enthält.

12. Organische optoelektronische Vorrichtung nach Anspruch 11, wobei:
die organische Schicht eine lichtemittierende Schicht enthält, und
die lichtemittierende Schicht die Zusammensetzung enthält.

13. Organische optoelektronische Vorrichtung nach Anspruch 11 oder 12, wobei die erste Verbindung und die zweite Verbindung als phosphoreszierendes Wirtsmaterial in der lichtemittierenden Schicht enthalten sind.

14. Organische optoelektronische Vorrichtung nach Anspruch 11, wobei die Zusammensetzung eine rotes Licht emittierende Zusammensetzung ist.

15. Anzeigevorrichtung mit der organischen optoelektronischen Vorrichtung nach Anspruch 11.

## Revendications

1. Composition, comprenant :
un premier composé, le premier composé étant représenté par une combinaison des Formule Chimique 1 et Formule Chimique 2 reliées ensemble ; et
un deuxième composé, le deuxième composé étant représenté par la Formule Chimique 3, 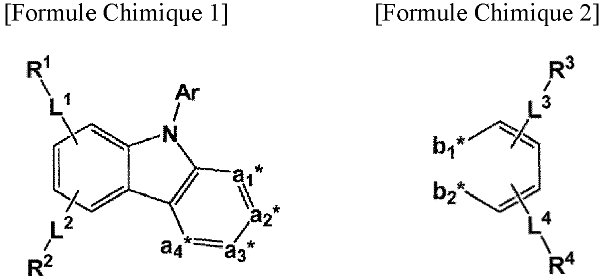
dans laquelle, dans les Formule Chimique 1 et Formule Chimique 2,
Ar est un groupement aryle en C6 à C30 substitué ou non substitué, un groupement hétérocyclique en C2 à C30 substitué ou non substitué, ou une combinaison de ceux-ci,
deux de a₁^{∗} à a₄^{∗} adjacents sont respectivement reliés avec b₁^{∗} et b₂^{∗}, et les deux de a₁^{∗} à a₄^{∗} restants non reliés avec b₁^{∗} et b₂^{∗} sont chacun indépendamment C-L^{a}-R^{a},
L^{a} et L¹ à L⁴ sont chacun indépendamment une simple liaison, un groupement arylène en C6 à C20 substitué ou non substitué, un groupement hétérocyclique en C2 à C20 divalent substitué ou non substitué, ou une combinaison de ceux-ci,
R^{a} et R¹ à R⁴ sont chacun indépendamment un hydrogène, un deutérium, un groupement cyano, un groupement amine substitué ou non substitué, un groupement alkyle en C1 à C30 substitué ou non substitué, un groupement aryle en C6 à C30 substitué ou non substitué, un groupement hétérocyclique en C2 à C30 substitué ou non substitué, ou une combinaison de ceux-ci, pourvu qu'au moins un de R^{a} et R¹ à R⁴ soit un groupement représenté par la Formule Chimique A : 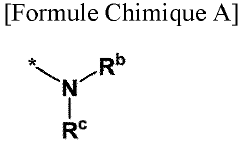
dans laquelle, dans la Formule Chimique A,
R^{b} et R^{c} sont chacun indépendamment un groupement aryle en C6 à C30 substitué ou non substitué, un groupement hétérocyclique en C2 à C30 substitué ou non substitué, ou une combinaison de ceux-ci, et
^{∗} est un point de liaison avec L^{a} et L¹ à L⁴ ; 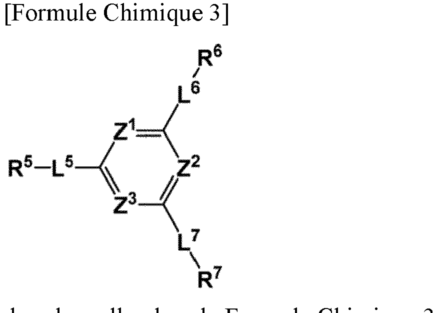
dans laquelle, dans la Formule Chimique 3,
Z¹ à Z³ sont chacun indépendamment N ou CR^{d}, dans laquelle R^{d} est un hydrogène, un deutérium, un groupement alkyle en C1 à C30 substitué ou non substitué, un groupement aryle en C6 à C30 substitué ou non substitué, un groupement hétérocyclique en C3 à C30 substitué ou non substitué, un groupement silyle substitué ou non substitué, un groupement amine substitué ou non substitué, un halogène, un groupement cyano, ou une combinaison de ceux-ci, pourvu qu'au moins deux de Z¹ à Z³ soient N,
L⁵ à L⁷ sont chacun indépendamment une simple liaison, un groupement arylène en C6 à C20 substitué ou non substitué, un groupement hétérocyclique en C2 à C20 divalent substitué ou non substitué ou une combinaison de ceux-ci,
R⁵ à R⁷ sont chacun indépendamment un groupement aryle en C6 à C30 substitué ou non substitué, un groupement hétérocyclique en C2 à C30 substitué ou non substitué, ou une combinaison de ceux-ci, pourvu qu'au moins un de R⁵ à R⁷ soit un groupement représenté par la Formule Chimique B : 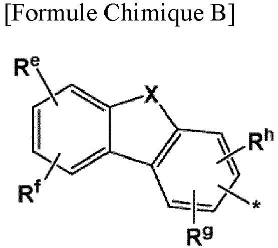
dans laquelle, dans la Formule Chimique B,
X est O ou S,
R^{e} à R^{h} sont chacun indépendamment un hydrogène, un deutérium, un groupement alkyle en C1 à C30 substitué ou non substitué, un groupement aryle en C6 à C30 substitué ou non substitué, un groupement hétérocyclique en C2 à C30 substitué ou non substitué, un groupement silyle substitué ou non substitué, un groupement amine substitué ou non substitué, un halogène, un groupement cyano, ou une combinaison de ceux-ci,
R^{e} et R^{f} sont chacun séparés ou sont reliés l'un avec l'autre pour former un cycle,
R^{g} et R^{h} sont chacun séparés ou sont reliés l'un avec l'autre pour former un cycle, et
^{∗} est un point de liaison avec l'un de L⁵ à L⁷.

2. Composition selon la revendication 1, dans laquelle le premier composé est représenté par l'une des Formule Chimique 1A à Formule Chimique 1C : 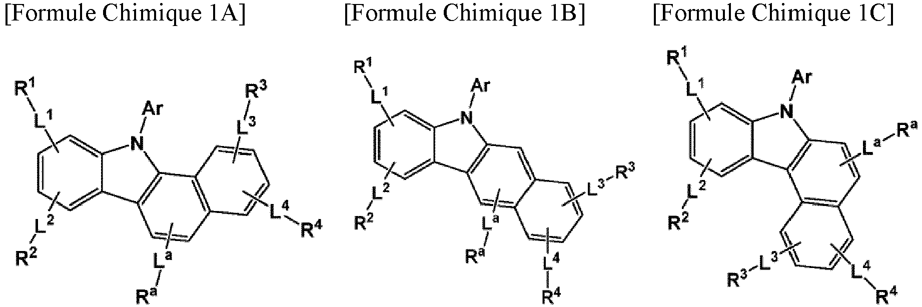
dans laquelle, dans les Formule Chimique 1A à Formule Chimique 1C,
Ar est un est un groupement aryle en C6 à C30 substitué ou non substitué, un groupement hétérocyclique en C2 à C30 substitué ou non substitué, ou une combinaison de ceux-ci,
L^{a} et L¹ à L⁴ sont chacun indépendamment une simple liaison, un groupement arylène en C6 à C20 substitué ou non substitué, un groupement hétérocyclique en C2 à C20 divalent substitué ou non substitué, ou une combinaison de ceux-ci,
R^{a} et R¹ à R⁴ sont chacun indépendamment un hydrogène, un deutérium, un groupement cyano, un groupement amine substitué ou non substitué, un groupement alkyle en C1 à C30 substitué ou non substitué, un groupement aryle en C6 à C30 substitué ou non substitué, un groupement hétérocyclique en C2 à C30 substitué ou non substitué, ou une combinaison de ceux-ci, pourvu qu'au moins un de R^{a} et R¹ à R⁴ soit un groupement représenté par la Formule Chimique A : 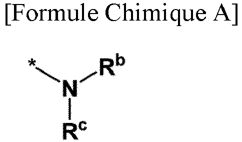
dans laquelle, dans la Formule Chimique A,
R^{b} et R^{c} sont chacun indépendamment un groupement aryle en C6 à C30 substitué ou non substitué, un groupement hétérocyclique en C2 à C30 substitué ou non substitué, ou une combinaison de ceux-ci, et
^{∗} est un point de liaison avec L^{a} et L¹ à L⁴.

3. Composition selon la revendication 1 ou la revendication 2, dans laquelle le premier composé est représenté par l'une des Formule Chimique 1A-1 à Formule Chimique 1A-3, et Formule Chimique 1B-1 à Formule Chimique 1B-3, et Formule Chimique 1C-1 à Formule Chimique 1C-3 : 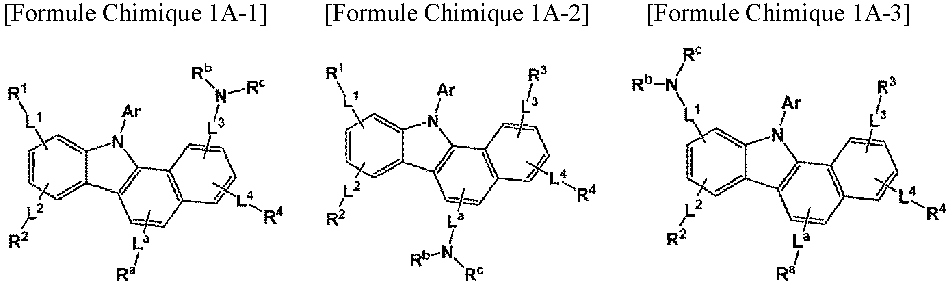 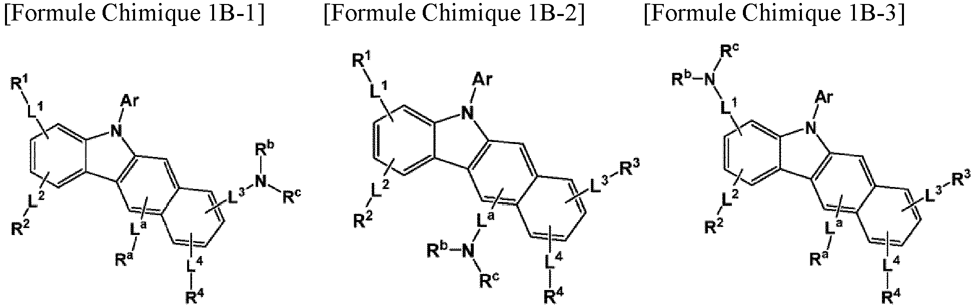 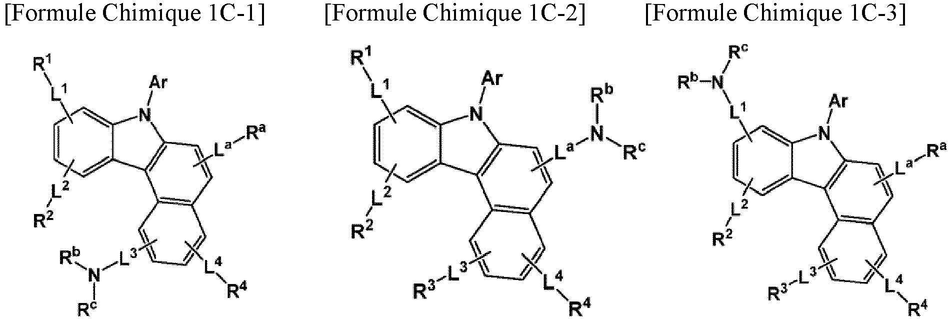
dans laquelle, dans les Formule Chimique 1A-1 à Formule Chimique 1A-3, Formule Chimique 1B-1 à Formule Chimique 1B-3, et Formule Chimique 1C-1 à Formule Chimique 1C-3,
Ar est un est un groupement aryle en C6 à C30 substitué ou non substitué, un groupement hétérocyclique en C2 à C30 substitué ou non substitué, ou une combinaison de ceux-ci,
L^{a} et L¹ à L⁴ sont chacun indépendamment une simple liaison, un groupement arylène en C6 à C20 substitué ou non substitué, un groupement hétérocyclique en C2 à C20 divalent substitué ou non substitué, ou une combinaison de ceux-ci,
R^{a} et R¹ à R⁴ sont chacun indépendamment un hydrogène, un deutérium, un groupement cyano, un groupement amine substitué ou non substitué, un groupement alkyle en C1 à C30 substitué ou non substitué, un groupement aryle en C6 à C30 substitué ou non substitué, un groupement hétérocyclique en C2 à C30 substitué ou non substitué, ou une combinaison de ceux-ci, et
R^{b} et R^{c} sont chacun indépendamment un groupement phényle substitué ou non substitué, un groupement biphényle substitué ou non substitué, un groupement anthracényle substitué ou non substitué, un groupement naphtyle substitué ou non substitué, un groupement phénanthrényle substitué ou non substitué, un groupement triphénylényle substitué ou non substitué, un groupement fluorényle substitué ou non substitué, un groupement carbazolyle substitué ou non substitué, un groupement dibenzofuranyle substitué ou non substitué, ou un groupement dibenzothiophényle substitué ou non substitué.

4. Composition selon l'une quelconque des revendications 1 à 3, dans laquelle le premier composé est représenté par la Formule Chimique 1A-1-b : 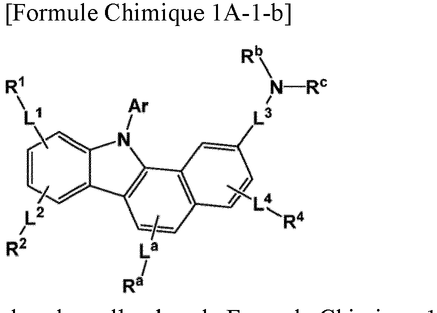
dans laquelle, dans la Formule Chimique 1A-1-b,
Ar est un est un groupement aryle en C6 à C30 substitué ou non substitué, un groupement hétérocyclique en C2 à C30 substitué ou non substitué, ou une combinaison de ceux-ci,
L^{a} et L¹ à L⁴ sont chacun indépendamment une simple liaison, un groupement arylène en C6 à C20 substitué ou non substitué, un groupement hétérocyclique en C2 à C20 divalent substitué ou non substitué, ou une combinaison de ceux-ci,
R^{a}, R¹, R² et R⁴ sont chacun indépendamment un hydrogène, un deutérium, un groupement cyano, un groupement alkyle en C1 à C30 substitué ou non substitué, un groupement aryle en C6 à C30 substitué ou non substitué, un groupement hétérocyclique en C2 à C30 substitué ou non substitué, ou une combinaison de ceux-ci, et
R^{b} et R^{c} sont chacun indépendamment un groupement phényle substitué ou non substitué, un groupement biphényle substitué ou non substitué, un groupement anthracényle substitué ou non substitué, un groupement naphtyle substitué ou non substitué, un groupement phénanthrényle substitué ou non substitué, un groupement triphénylényle substitué ou non substitué, un groupement fluorényle substitué ou non substitué, un groupement carbazolyle substitué ou non substitué, un groupement dibenzofuranyle substitué ou non substitué, ou un groupement dibenzothiophényle substitué ou non substitué.

5. Composition selon l'une quelconque des revendications 1 à 4, dans laquelle le deuxième composé est représenté par l'une des Formule Chimique 2A à Formule Chimique 2C : 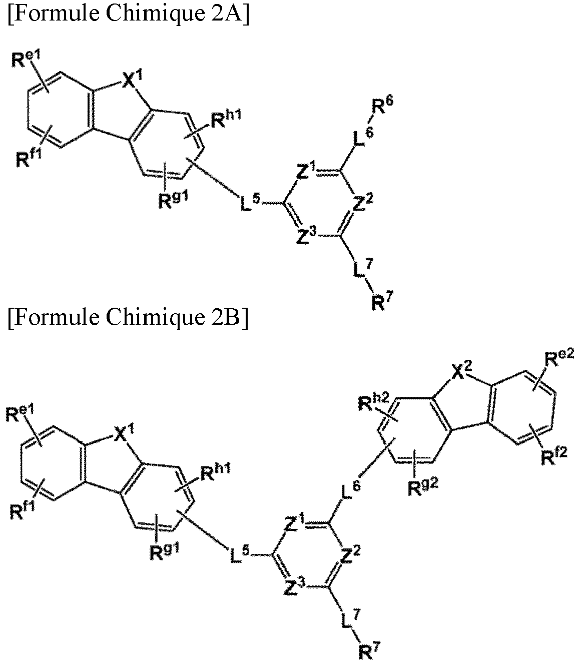 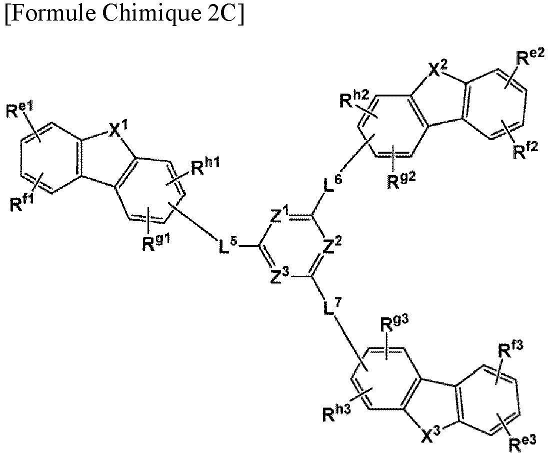
dans laquelle, dans les Formules Chimiques 2A à 2C,
Z¹ à Z³ sont chacun indépendamment N ou CR^{d}, dans laquelle R^{d} est un hydrogène, un deutérium, un groupement alkyle en C1 à C30 substitué ou non substitué, un groupement aryle en C6 à C30 substitué ou non substitué, un groupement hétérocyclique en C3 à C30 substitué ou non substitué, un groupement silyle substitué ou non substitué, un groupement amine substitué ou non substitué, un halogène, un groupement cyano, ou une combinaison de ceux-ci, pourvu qu'au moins deux de Z¹ à Z³ soient N,
L⁵ à L⁷ sont chacun indépendamment une simple liaison, un groupement arylène en C6 à C20 substitué ou non substitué, un groupement hétérocyclique en C2 à C20 divalent substitué ou non substitué ou une combinaison de ceux-ci,
R⁶ et R⁷ sont chacun indépendamment un groupement aryle en C6 à C30 substitué ou non substitué, un groupement hétérocyclique en C2 à C30 substitué ou non substitué, ou une combinaison de ceux-ci,
X¹ à X³ sont chacun indépendamment O ou S, et
R^{e1} à R^{e3}, R^{f1} à R^{f3}, R^{g1} à R^{g3} et R^{h1} à R^{h3} sont chacun indépendamment un hydrogène, un deutérium, un groupement alkyle en C1 à C30 substitué ou non substitué, un groupement aryle en C6 à C30 substitué ou non substitué, un groupement hétérocyclique en C2 à C30 substitué ou non substitué, un groupement silyle substitué ou non substitué, un groupement amine substitué ou non substitué, un halogène, un groupement cyano, ou une combinaison de ceux-ci.

6. Composition selon l'une quelconque des revendications 1 à 5, dans laquelle :
le deuxième composé est représenté par la Formule Chimique 2A ou la Formule Chimique 2B, et
R⁶ et R⁷ sont chacun indépendamment un groupement phényle substitué ou non substitué, un groupement biphényle substitué ou non substitué, un groupement terphényle substitué ou non substitué, un groupement naphtyle substitué ou non substitué, un groupement anthracényle substitué ou non substitué, un groupement phénanthrényle substitué ou non substitué, un groupement triphénylényle substitué ou non substitué, un groupement fluorényle substitué ou non substitué, un groupement pyridinyle substitué ou non substitué, un groupement pyrimidinyle substitué ou non substitué, un groupement triazinyle substitué ou non substitué, ou une combinaison de ceux-ci.

7. Composition selon l'une quelconque des revendications 1 à 6, dans laquelle la Formule Chimique B est représentée par l'une des Formule Chimique B-1 à Formule Chimique B-4 : 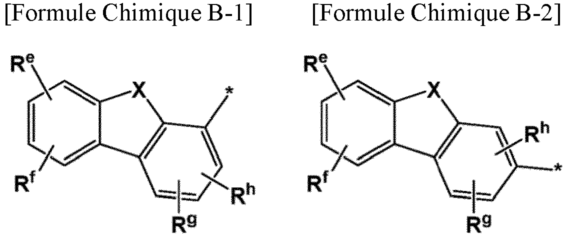 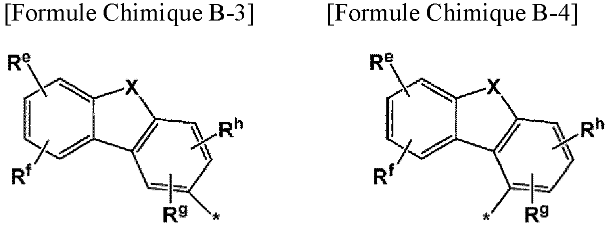
dans laquelle, dans les Formule Chimique B-1 à Formule Chimique B-4,
X est O ou S,
R^{e} à R^{h} sont chacun indépendamment un hydrogène, un deutérium, un groupement alkyle en C1 à C30 substitué ou non substitué, un groupement aryle en C6 à C30 substitué ou non substitué, un groupement hétérocyclique en C3 à C30 substitué ou non substitué, un groupement silyle substitué ou non substitué, un groupement amine substitué ou non substitué, un halogène, un groupement cyano, ou une combinaison de ceux-ci,
R^{e} et R^{f} sont chacun séparés ou sont reliés l'un avec l'autre pour former un cycle,
R^{g} et R^{h} sont chacun séparés ou sont reliés l'un avec l'autre pour former un cycle, et
^{∗} est un point de liaison avec l'un de L⁵ à L⁷.

8. Composition selon l'une quelconque des revendications 1 à 7, dans laquelle R⁵ à R⁷ sont chacun indépendamment un groupement phényle substitué ou non substitué, un groupement biphényle substitué ou non substitué, un groupement terphényle substitué ou non substitué, un groupement naphtyle substitué ou non substitué, un groupement anthracényle substitué ou non substitué, un groupement phénanthrényle substitué ou non substitué, un groupement triphénylényle substitué ou non substitué, un groupement fluorényle substitué ou non substitué, un groupement pyridinyle substitué ou non substitué, un groupement pyrimidinyle substitué ou non substitué, un groupement triazinyle substitué ou non substitué, le groupement représenté par la Formule Chimique B, ou une combinaison de ceux-ci.

9. Composition selon l'une quelconque des revendications 1 à 8, dans laquelle :
le premier composé est représenté par la Formule Chimique 1A-1-b, et
le deuxième composé est représenté par la Formule Chimique 2A ou la Formule Chimique 2B, 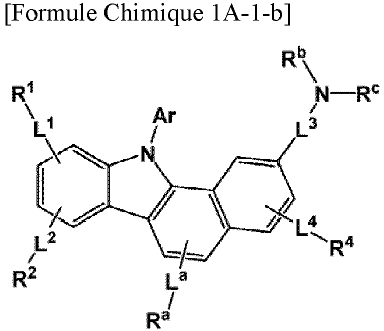
dans laquelle, dans la Formule Chimique 1A-1-b,
Ar est un groupement phényle substitué ou non substitué, un groupement biphényle substitué ou non substitué, un groupement terphényle substitué ou non substitué, un groupement naphtyle substitué ou non substitué, un groupement anthracényle substitué ou non substitué,
un groupement phénanthrényle substitué ou non substitué, un groupement triphénylényle substitué ou non substitué, un groupement fluorényle substitué ou non substitué, un groupement dibenzofuranyle substitué ou non substitué, un groupement dibenzothiophényle substitué ou non substitué, un groupement carbazolyle substitué ou non substitué, ou une combinaison de ceux-ci,
L^{a} et L¹ à L⁴ sont chacun indépendamment une simple liaison, un groupement phénylène substitué ou non substitué, un groupement biphénylène substitué ou non substitué, un groupement terphénylène substitué ou non substitué, ou un groupement naphtylène substitué ou non substitué,
R^{a}, R¹, R² et R⁴ sont chacun indépendamment un hydrogène, un deutérium, un groupement cyano, un groupement alkyle en C1 à C30 substitué ou non substitué, un groupement aryle en C6 à C30 substitué ou non substitué, un groupement hétérocyclique en C2 à C30 substitué ou non substitué, ou une combinaison de ceux-ci, et
R^{b} et R^{c} sont chacun indépendamment un groupement phényle substitué ou non substitué, un groupement biphényle substitué ou non substitué, un groupement anthracényle substitué ou non substitué, un groupement naphtyle substitué ou non substitué, un groupement phénanthrényle substitué ou non substitué, un groupement triphénylényle substitué ou non substitué, un groupement fluorényle substitué ou non substitué, un groupement carbazolyle substitué ou non substitué, un groupement dibenzofuranyle substitué ou non substitué, ou un
groupement dibenzothiophényle substitué ou non substitué ; 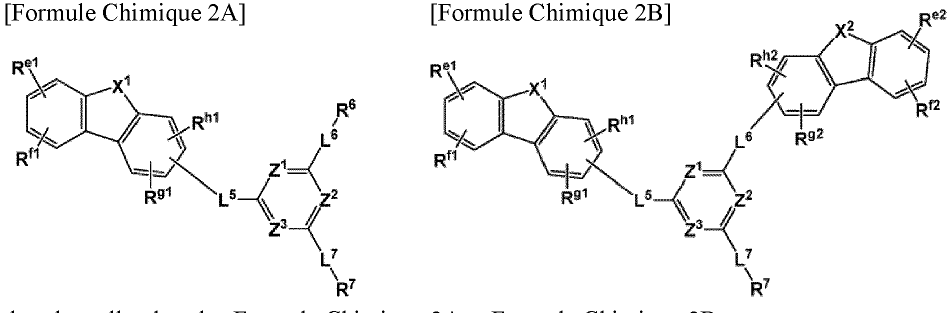
dans laquelle, dans les Formule Chimique 2A et Formule Chimique 2B,
Z¹ à Z³ sont chacun indépendamment N ou CR^{d}, pourvu qu'au moins deux de Z¹ à Z³ soient N,
L⁵ à L⁷ sont chacun indépendamment une simple liaison un groupement phénylène substitué ou non substitué, un groupement biphénylène substitué ou non substitué, un groupement terphénylène substitué ou non substitué, ou un groupement naphtylène substitué ou non substitué,
R⁶ et R⁷ sont chacun indépendamment un groupement phényle substitué ou non substitué, un groupement biphényle substitué ou non substitué, un groupement terphényle substitué ou non substitué, un groupement quaterphényle substitué ou non substitué, un groupement naphtyle substitué ou non substitué, un groupement anthracényle substitué ou non substitué, un groupement phénanthrényle substitué ou non substitué, un groupement triphénylényle substitué ou non substitué, un groupement fluorényle substitué ou non substitué, un groupement pyridinyle substitué ou non substitué, un groupement pyrimidinyle substitué ou non substitué, un groupement triazinyle substitué ou non substitué, un groupement dibenzofuranyle substitué ou non substitué, ou un groupement dibenzothiophényle substitué ou non substitué,
X¹ et X² sont chacun indépendamment O ou S, et
R^{f3}, R^{e1}, R^{e2}, R^{f1}, R^{f2}, R^{g1}, R^{g2}, R^{h1}, et R^{h2} sont chacun indépendamment un hydrogène, un deutérium, un groupement alkyle en C1 à C30 substitué ou non substitué, un groupement aryle en C6 à C30 substitué ou non substitué, un groupement hétérocyclique en C2 à C30 substitué ou non substitué, un groupement silyle substitué ou non substitué, un groupement amine substitué ou non substitué, un halogène, un groupement cyano, ou une combinaison de ceux-ci.

10. Composition selon l'une quelconque des revendications 1 à 9, dans laquelle le deuxième composé est représenté par la Formule Chimique 2B-1 : 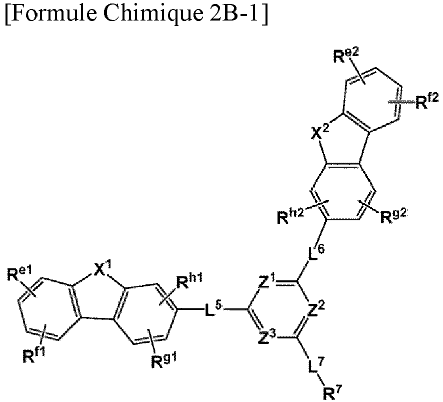
dans laquelle, dans la Formule Chimique 2B-1,
Z¹ à Z³ sont chacun indépendamment N ou CR^{d}, pourvu qu'au moins deux de Z¹ à Z³ soient N,
L⁵ à L⁷ sont chacun indépendamment une simple liaison un groupement phénylène substitué ou non substitué, un groupement biphénylène substitué ou non substitué, un groupement terphénylène substitué ou non substitué, ou un groupement naphtylène substitué ou non substitué,
R⁷ est un groupement phényle substitué ou non substitué, un groupement biphényle substitué ou non substitué, un groupement terphényle substitué ou non substitué, un groupement quaterphényle substitué ou non substitué, un groupement naphtyle substitué ou non substitué, un groupement anthracényle substitué ou non substitué, un groupement phénanthrényle substitué ou non substitué, un groupement triphénylényle substitué ou non substitué, un groupement fluorényle substitué ou non substitué, un groupement pyridinyle substitué ou non substitué, un groupement pyrimidinyle substitué ou non substitué, un groupement triazinyle substitué ou non substitué, un groupement dibenzofuranyle substitué ou non substitué, ou un groupement dibenzothiophényle substitué ou non substitué,
X¹ et X² sont chacun indépendamment O ou S, et
R^{f3}, R^{e1}, R^{e2}, R^{f1}, R^{f2}, R^{g1}, R^{g2}, R^{h1}, et R^{h2} sont chacun indépendamment un hydrogène, un deutérium, un groupement alkyle en C1 à C30 substitué ou non substitué, un groupement aryle en C6 à C30 substitué ou non substitué, un groupement hétérocyclique en C2 à C30 substitué ou non substitué, un groupement silyle substitué ou non substitué, un groupement amine substitué ou non substitué, un halogène, un groupement cyano, ou une combinaison de ceux-ci.

11. Dispositif optoélectronique organique, comprenant :
une anode et une cathode se faisant face ; et
au moins une couche organique disposée entre l'anode et la cathode, dans laquelle la couche organique inclut la composition selon l'une quelconque des revendications 1 à 10.

12. Dispositif optoélectronique organique selon la revendication 11, dans lequel :
la couche organique inclut une couche émettrice de lumière, et
la couche émettrice de lumière inclut la composition.

13. Dispositif optoélectronique organique selon la revendication 11 ou la revendication 12, dans lequel le premier composé et le deuxième composé sont inclus en tant qu'hôtes phosphorescents de la couche émettrice de lumière.

14. Dispositif optoélectronique organique selon la revendication 11, dans lequel la composition est une couche émettrice de lumière rouge.

15. Dispositif d'affichage comprenant le dispositif optoélectronique organique selon la revendication 11.
